# EUROPEAN PATENT SPECIFICATION

(11) **EP 2 948 188 B1**
(45) Date of publication and mention of the grant of the patent: **08.09.2021**
(21) Application number: 14743474.0
(22) Date of filing: 24.01.2014
(51) Int. Cl.: A61K 51/04, A61B 6/00, A61B 18/14, A61P 43/00, G01N 33/60, G01N 33/68, A61K 101/02

(54) **NEURONAL IMAGING AND TREATMENT**
NEURONALE BILDGEBUNG UND BEHANDLUNG
IMAGERIE NEURONALE ET TRAITEMENT

(30) Priority: 24.01.2013 US 201361756112 P; 11.03.2013 US 201361776599 P; 20.03.2013 US 201361803611 P; 06.06.2013 US 201361831664 P; 08.09.2013 US 201361875069 P; 08.09.2013 US 201361875074 P; 08.09.2013 US 201361875070 P; 10.01.2014 US 201461925670 P; 10.01.2014 US 201461925669 P
(43) Date of publication of application: 02.12.2015
(73) Proprietor: Tylerton International Holdings Inc., Road Town (VG)
(72) Inventor: BEN-HAIM, Shlomo, 1207 Geneva (CH)
(74) Representative: Korenberg, Alexander Tal
(86) International application number: PCT/IL2014/050090
(87) International publication number: WO 2014/115152

(56) References cited:
- EP-A1- 2 591 722
- US-A1- 2006 127 309
- US-A1- 2010 221 182
- R. ARORA: "Recent Insights Into the Role of the Autonomic Nervous System in the Creation of Substrate for Atrial Fibrillation: Implications for Therapies Targeting the Atrial Autonomic Nervous System", CIRCULATION. ARRHYTHMIA AND ELECTROPHYSIOLOGY, vol. 5, no. 4, 1 August 2012 (2012-08-01), pages 850-859, XP055236980, United States ISSN: 1941-3149, DOI: 10.1161/CIRCEP.112.972273
- LEMERY R ET AL: "Feasibility study of endocardial mapping of ganglionated plexuses during catheter ablation of atrial fibrillation", HEART RHYTHM, ELSEVIER, US, vol. 3, no. 4, 1 April 2006 (2006-04-01), pages 387-396, XP024972538, ISSN: 1547-5271, DOI: 10.1016/J.HRTHM.2006.01.009 [retrieved on 2006-04-01]
- ERNST, SABINE: "IMAGE GUIDED ABLATION OF GANGLIONATED PLEXI AS AN ADDITION TO PV ISOLATION - FOLLOW-UP RESULTS OF THE INITIAL CASE SERIES", HEART RHYTHM, vol. 12, no. 5, May 2015 (2015-05), XP029240122, ISSN: 1547-5271, DOI: 10.1016/J.HRTHM.2015.03.058
- RISPLER SHMUEL ET AL: "Quantitative 123I-MIBG SPECT/CT assessment of cardiac sympathetic innervation - A new diagnostic tool for heart failure", INTERNATIONAL JOURNAL OF CARDIOLOGY, vol. 168, no. 2, 17 January 2013 (2013-01-17), pages 1556-1558, XP028740607, ISSN: 0167-5273, DOI: 10.1016/J.IJCARD.2012.12.077
- T Wichter ET AL: "Regional myocardial sympathetic dysinnervation in arrhythmogenic right ventricular cardiomyopathy. An analysis using 123I-meta-iodobenzylguanidine scintigraphy", Circulation, 1 February 1994 (1994-02-01), pages 667-683, XP055555546, Baltimore DOI: 10.1161/01.CIR.89.2.667 Retrieved from the Internet: URL:https://www.ahajournals.org/doi/pdf/10 .1161/01.CIR.89.2.667

## Description

### RELATED APPLICATIONS:

This application claims the benefit of priority of U.S. Provisional Patent Application No. 61/756,112, filed January 24, 2013, U.S. Provisional Patent Application No. 61/776,599, filed March 11, 2013, U.S. Provisional Patent Application No. 61/803,611 filed March 20, 2013, U.S. Provisional Patent Application No. 61/831,664, filed June 6, 2013, U.S. Provisional Patent Application Nos. 61/875,069, 61/875,070 and 61/875,074, filed September 8, 2013, and U.S. Provisional Patent Application Nos. 61/925,669 and 61/925,670, filed January 10, 2014.

### FIELD AND BACKGROUND OF THE INVENTION

Disclosed herein are methods and systems of imaging and, more particularly, but not exclusively, methods and systems of medical imaging using a radioactive tracer with affinity to nervous tissue.

The autonomic nervous system (ANS) is a part of the peripheral nervous system that controls, usually at an unconscious level, various bodily functions such as heart rate, vasomotor activity, digestion, respiration, reflex actions and the like. The ANS is regulated by the medulla oblongata and hypothalamus in the brain.

ANS functions include afferent (sensory) activity - transmitting signals from the viscera to the central nervous system (CNS), as well as efferent (motor) activity -transmitting signals from the CNS to the periphery.

Efferent activity in the ANS is typically divided into two divisions, the sympathetic nervous system and the parasympathetic nervous system. To a considerable degree, these divisions act in parallel to one another, and often have opposing effects. The sympathetic division is usually associated with mobilization of a quick response, whereas the parasympathetic division is usually associated with lower activities.

The peripheral nervous system also includes the somatic nervous system (SoNS), which controls voluntary movements via efferent neurons and conducts, via afferent neurons, impulses of pain, touch and temperature from the body surface, and muscle, tendon and joint sense from within the body.

Afferent (sensory) neurons of the ANS and SoNS extend from peripheral organs to synapses in the spinal cord. Cell bodies of afferent neurons are aggregated in spinal ganglia, just outside the spinal cord.

Efferent SoNS neurons are located in the spinal cord and extend to synapses at neuromuscular junctions at the innervated muscle.

In contrast to afferent and SoNS efferent activity, ANS efferent innervation is characterized by the involvement of two sequential efferent neurons which communicate via a synapse in a ganglion. The preganglionic neuron carries a signal from the central nervous system to the ganglion, and the postganglionic neuron carries a signal from the ganglion to the target (e.g., an innervated organ).

Sympathetic ganglia include the paravertebral ganglia, which are located along the spine, prevertebral ganglia, which are located in the abdomen and innervate abdominal organs, and the adrenal medulla in the adrenal gland, which is a modified ganglion in which the postganglionic cells release hormones into the blood instead of acting as neurons.

In comparison to sympathetic ganglia, parasympathetic ganglia are usually small and located close to the organ they innervate, that is, the postganglionic neurons are relatively short.

Acetylcholine is the primary neurotransmitter secreted at synapses by sympathetic and parasympathetic preganglionic neurons in ganglia, by SoNS neurons and parasympathetic postganglionic neurons at innervated organs, and by sympathetic postganglionic neurons at sweat glands. Acetylcholine receptors in ANS ganglia and neuromuscular junctions are typically nicotinic receptors, and acetylcholine receptors at ANS synapses in innervated organs are typically muscarinic receptors.

In contrast, sympathetic postganglionic neurons generally secrete adrenergic neurotransmitters, primarily norepinephrine (noradrenaline).

The sympathetic and parasympathetic nervous systems are not entirely separate. For example, cardiac ganglia, typically considered as part of the parasympathetic nervous system, also include adrenergic synapses of sympathetic neurons, which provide input to parasympathetic postganglionic neurons, and a similar sympathetic input to parasympathetic postganglionic neurons occurs in pelvic prevertebral ganglia [Smith, Am J Physiol 1999, 276:R455-R467; Arora et al., Anat Rec A Discov Mol Cell Evol Biol 2003, 271:249-258].

Atrial fibrillation may be treated by ablation or surgery aimed at disrupting autonomic signaling to atria, as both sympathetic and parasympathetic stimuli appear to be involved in atrial fibrillation. Neurons around the pulmonary veins, and the nearby cardiac ganglionated plexuses, have been reported to be effective targets for ablation and/or surgery [Arora, Circ Arrhythm Electrophysiol 2012, 5:850-859; Tan et al., Heart Rhythm 2007, 4:S57-S60].

A variety of medical imaging techniques are available for obtaining images of internal organs. Techniques such as X-ray computerized tomography (CT), magnetic resonance imaging (MRI), and ultrasound scans utilize an external source of irradiation; whereas techniques such as positron emission tomography (PET) and single-photon emission computed tomography (SPECT) utilize nuclear radiation emitted by a radioactive tracer within the body.

In such techniques, data may be obtained in a manner which provides 3-dimensional information, such that the data can be processed so as to generate a volumetric image. Orbiting detectors from multiple directions may be used to generate a volumetric image by computed tomography. PET and SPECT use a collimator to allow only radiation from a certain direction to reach the detector. Typically, this collimator is constructed to provide a multiplicity of small holes in a dense, high-atomic number material such as lead or tungsten. Radiation will pass through a hole if it travels in a direction aligned with a hole but will tend to be absorbed by the collimator material if it travels in a different direction.

Radioactive tracers may be selected so as to allow for selective imaging of a particular tissue type or activity.

Langer & Haldin [Eur J Nucl Med 2002, 29:416-434] describe PET and SPECT radioactive tracers for mapping the cardiac nervous system, including catecholamines and catecholamine analogs such as mIBG (m-iodobenzylguanidine) and *m*-hydroxyephedrine for imaging adrenergic synapses, and vesamicol derivatives for imaging cholinergic synapses.

mIBG uptake and washout rate in the heart, as determined by SPECT, have been reported to predict atrial fibrillation, including recurrence of atrial fibrillation after ablation therapy [Arora, Circ Arrhythm Electrophysiol 2012, 5:850-859]. mIBG SPECT images have poor spatial resolution in comparison to [C-11]hydroxyephedrine PET images, but [C-11]hydroxyephedrine PET images are more difficult to obtain, and their clinical significance is less clear due to lack of clinical studies [Matsunari et al., Circ Cardiovasc Imaging 2010, 3:595-603].

Ross & Seibyl [J Nucl Med Tech 2004, 32:209-214] describe a variety of iodine-123 labeled ligands for SPECT imaging of neuroreceptors.

Additional art includes Vallabhajosula & Nikolopoulou [Semin Nucl Med 2011, 41:324-33], U.S. Patent Application Publication No. 2010/0221182 and U.S. Patent Nos. 6, 211,360, 6,358,492, 5,077,035 and 5,789,420.

Wichter, T et al; "Regional myocardial sympathetic dysinnervation in arrhythmogenic right ventricular cardiomyopathy. An analysis using 123I-meta-iodobenzylguanidine scintigraphy", Circulation, 1 February 1994, pages 667-683, describes the use of ¹²³I-NUBG in a SPECT imaging method for use in the diagnosis of cardiac arrhythmia.

### SUMMARY OF THE INVENTION

Aspects of the invention are set out in the independent claims. Particular embodiments of these aspects are set out in the dependent claims. Any subject matter contained herein that does not fall within the scope of the appended claims is considered as being useful for understanding the invention.

Disclosed herein is an imaging method for imaging at least one synaptic center of an autonomic nervous system in a subject, the method comprising: administering to the subject a radioactive tracer which selectively binds to synapses of the autonomic nervous system; and measuring radioactive emission of the radioactive tracer to obtain data describing a distribution of the radioactive tracer in the body, thereby imaging the radioactive tracer in the body, the method further comprising analyzing the data in order to identify at least one region exhibiting a high concentration of the radioactive tracer, thereby imaging at least one synaptic center.

The imaging may comprise 3-dimensional imaging.

The region may have a diameter of no more than 20 mm.

The synaptic center may be selected from the group consisting of an autonomic ganglion and an autonomic ganglionated plexus.

The method may further comprise evaluating a neuronal activity in at least one synaptic center.

The method may further comprise generating a map showing a distribution and/or neuronal activity of synapses of the autonomic nervous system, the map including the at least one synaptic center.

The method may further comprise overlaying the distribution and/or neuronal activity on an anatomical map.

The anatomical map may include an image of at least a portion of at least one organ.

The method may further comprise obtaining an image of at least a portion of a body of the subject, and using the image to generate the anatomical map.

The image of at least a portion of a body may be obtained using an imaging modality selected from the group consisting of a positron emission tomography (PET) modality, a computerized tomography (CT) modality, a magnetic resonance imaging (MRI) modality and an ultrasound modality.

The analyzing may comprise: reconstructing an anatomical image of a region of a body of the subject, the region comprising a portion of at least one internal body part; processing the anatomical image to generate at least one image mask corresponding to dimensions of the at least one internal body part; and correlating the at least one generated image mask with the data describing a distribution of the radioactive tracer in the body, for guiding a reconstruction of an image depicting the at least one synaptic center.

The at least one image mask may be generated based on templates that define the location of a synaptic center within and/or in proximity to the at least one internal body part.

The method may further comprise removing data describing a presence of the radioactive tracer from anatomical regions that do not contain synaptic centers based on the anatomical data of the anatomical regions.

The method may further comprise identifying the at least one synaptic center within the at least one generated image mask based on at least one predefined rule, the at least one predefined rule comprising radioactive emission of a radioactive tracer above a predefined threshold.

The correlating may comprise positioning the at least one image mask to correspond with regions exhibiting a high concentration of the radioactive tracer according to the data describing a distribution of the radioactive tracer in the body.

The method may further comprise comparing the data with a reference data set of synapse distribution and/or neuronal activity.

The reference data set may be indicative of normal synapse distribution and/or neuronal activity.

The reference data set may be indicative of a disease or disorder characterized by abnormal synapse distribution and/or neuronal activity.

The method may further comprise diagnosing a disease or disorder associated with an abnormal autonomic nervous system activity based on the imaging.

The method may further comprise stimulating a neuronal activity in conjunction with the imaging, and characterizing at least one synaptic center based on the stimulating.

The method may comprise performing the imaging at multiple time points and characterizing distribution and/or neuronal activity at different time points.

The multiple time points may comprise time points before and after a treatment, the method further comprising evaluating an effect of the treatment on autonomic nervous system activity.

The method may further comprise characterizing a rate of change in concentration of the radioactive tracer in at least one region of the body.

An activity in a synaptic center may be determined according to a correlation with a washout rate of the radioactive tracer.

The method may further comprise guiding a treatment based on the imaging of at least one synaptic center.

Tthe treatment may comprise modulating neuronal activity in at least one synaptic center imaged by the method.

The modulating may comprise ablation of the synaptic center.

The synaptic center may be selected from the group consisting of a cardiac ganglionated plexus, a pelvic plexus and a celiac ganglion.

The method may comprise administering and imaging a first radioactive tracer which selectively binds to synapses of the autonomic nervous system, and further administering and imaging at least one additional radioactive tracer, wherein a radioactive isotope the first radioactive tracer and of each the at least one additional radioactive tracer are different from one another.

The at least one additional radioactive tracer may selectively bind to synapses of the autonomic nervous system.

The first radioactive tracer may selectively bind to synapses which are different than the synapses selectively bound by the at least one additional radioactive tracer.

The at least one additional radioactive tracer may not selectively bind to synapses.

Also described herein is a method of treating a disease or disorder associated with an abnormal autonomic nervous system activity in a subject in need thereof, the method comprising imaging, according to any one of the methods described herein, at least one synaptic center of the autonomic nervous system associated with the disease or disorder in a subject in need thereof, and treating the disease or disorder based on the imaging.

The treating may comprise modulating neuronal activity in at least one synaptic center imaged by the imaging.

Tthe disease or disorder may be selected from the group consisting of cardiac arrhythmia, prostatic hyperplasia, an autoimmune disease or disorder, diabetes, stress, erectile dysfunction, irritable bowel syndrome, thyrotoxicosis, hypertension, hypertrophic cardiomyopathy, chronic obstructive pulmonary disease, syncope, hypothyroidism, idiopathic heart failure, asthma, a deposition disease, pathological weight gain, tortocolis, idiopathic dilated cardiomyopathy, right ventricular outflow tachycardia, Brugada syndrome, tetralogy of Fallot, hypertrophic obstructive cardiomyopathy, sleep apnea, metabolic derangement of liver, hyperhydrosis, excessive salivation and excessive lacrimation.

Also described herein is a method of determining a normal autonomic nervous system activity or abnormal autonomic nervous system activity in a subject, the method comprising: administering a first radioactive tracer which selectively binds to synapses of the autonomic nervous system and imaging, according to the method of claim 24, at least one synaptic center of the autonomic nervous system, to thereby obtain data describing a distribution of the first radioactive tracer in the body; and administering at least one additional radioactive tracer and imaging the at least one additional radioactive tracer in at least a portion of the body of the subject, to thereby obtain data describing a distribution of the at least one additional radioactive tracer in the body, wherein a combination of the data describing a distribution of the first radioactive tracer in the body and the data describing a distribution of the at least one additional radioactive tracer in the body is indicative of normal or abnormal synapse distribution and/or neuronal activity in the autonomous nervous system.

The at least one additional radioactive tracer may selectively bind to synapses of the autonomic nervous system which are different than synapses selectively bound by the first radioactive tracer.

The combination of distributions of radioactive tracers may be indicative of normal or abnormal balance between a sympathetic nervous system activity and a parasympathetic nervous system activity.

At least one of the at least one additional radioactive tracer may not selectively bind to synapses, and the combination of distributions of radioactive tracers may be indicative of normal or abnormal synapse distribution and/or neuronal activity in relation to a tissue distribution and/or activity indicated by the additional radioactive tracer which does not selectively bind to synapses.

The abnormal autonomic nervous system activity may be associated with a disease or disorder selected from the group consisting of cardiac arrhythmia, prostatic hyperplasia, an autoimmune disease or disorder, diabetes, stress, erectile dysfunction, irritable bowel syndrome, thyrotoxicosis, hypertension, hypertrophic cardiomyopathy, chronic obstructive pulmonary disease, syncope, hypothyroidism, idiopathic heart failure, asthma, deposition diseases, pathological weight gain, tortocolis, idiopathic dilated cardiomyopathy, right ventricular outflow tachycardia, Brugada syndrome, tetralogy of Fallot, hypertrophic obstructive cardiomyopathy, sleep apnea, asthma, metabolic derangement of liver, hyperhydrosis, excessive salivation and excessive lacrimation.

The synaptic center may be characterized by adrenergic activity.

The radioactive tracer may have the general formula I: 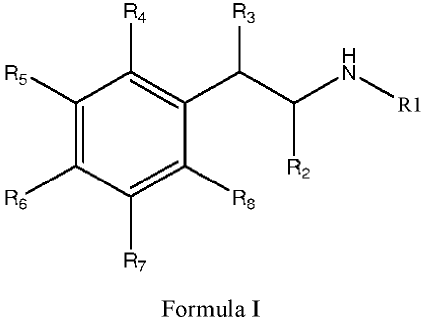 or a pharmaceutically acceptable salt thereof, wherein: R₁ is selected from the group consisting of hydrogen and alkyl; and R₂-R₈ are each independently selected from the group consisting of hydrogen, alkyl, hydroxy, alkoxy and halo.

The radioactive tracer may have the general formula II:

A-B-D-E Formula II

or a pharmaceutically acceptable salt thereof, wherein: A is selected from the group consisting of aryl and heteroaryl, each being substituted or non-substituted; B is selected from the group consisting of O, S, NR₁₀ or is absent; D is selected from the group consisting of alkyl, cycloalkyl, heteroalicyclic, aryl and heteroaryl; and E is - NR₁₁-C(=NH)NH₂, wherein R₁₁ is selected from the group consisting of hydrogen, alkyl, cycloalkyl, heteroalicyclic, aryl and heteroaryl, or alternatively, R₁₁ and R₁₀ are linked together to form a heteroalicyclic or heteroaryl ring comprising B, D and NR₁₁.

The radioactive tracer may be selected from the group consisting of a norepinephrine transporter (NET) ligand, a vesicular monoamine transporter (VMAT) ligand, a norepinephrine receptor agonist, a norepinephrine receptor antagonist and a norepinephrine reuptake inhibitor.

The synaptic center may be characterized by cholinergic activity.

The cholinergic activity may comprise nicotinic cholinergic activity.

The cholinergic activity may comprise muscarinic cholinergic activity.
The radioactive tracer may have the general formula III: 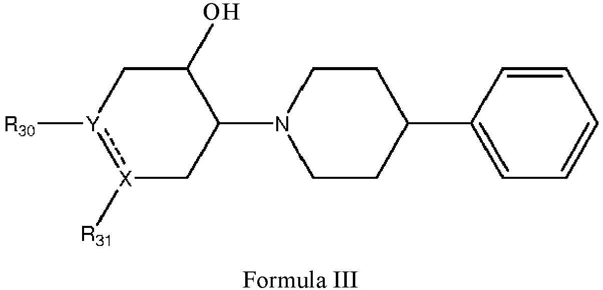 or a pharmaceutically acceptable salt thereof, wherein: X and Y are each independently N or CH and the dashed line represents a saturated bond, or alternatively, X and Y are each C and the dashed line represents an unsaturated bond; and R₃₀ and R₃₁ are each independently selected from the group consisting of hydrogen, and substituted or non-substituted phenyl, benzyl or benzoyl, or alternatively, R₃₀ and R₃₁ together form a substituted or non-substituted phenyl ring.

The radioactive tracer may have the general formula IV: 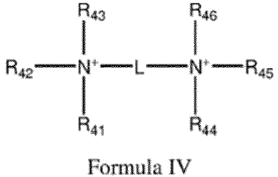 or a pharmaceutically acceptable salt thereof, wherein: R₄₁-R₄₆ are each independently substituted or non-substituted alkyl, or alternatively, any two of R₄₁-R₄₃ and/or any two of R₄₄-R₄₆ together form a 3-, 4-, 5-, or 6-membered substituted or non-substituted heteroaryl or heteroalicyclic ring; and L is a substituted or non-substituted hydrocarbon chain from 2-10 atoms in length.

The radioactive tracer may have the general formula V:

G-J-K Formula V

or a pharmaceutically acceptable salt thereof, wherein: G is a substituted or non-substituted phenyl or pyridinyl moiety; J is absent or is selected from the group consisting of O, S, -O-alkyl-, -S-alkyl, -alkyl-O- and alkyl-S-; and K is a substituted or non-substituted heteroalicyclic ring comprising at least one nitrogen atom.

The radioactive tracer may be selected from the group consisting of a nicotinic acetylcholine receptor agonist and a nicotinic acetylcholine receptor antagonist.

The radioactive tracer may be selected from the group consisting of a muscarinic acetylcholine receptor agonist and a muscarinic acetylcholine receptor antagonist.

Also described herein is a use of a radioactive tracer which selectively binds to synapses of the autonomic nervous system, in the manufacture of a diagnostic agent for use in the any one of the methods described herein, and in any one of the embodiments thereof.

Also described herein is a radioactive tracer which selectively binds to synapses of the autonomic nervous system, for use in any one of the methods as described herein, and in any one of the embodiments thereof.

Also described herein is a use of a radioactive tracer which selectively binds to synapses of the autonomic nervous system, in the manufacture of a diagnostic agent for use in a method of determining a normal autonomic nervous system activity or abnormal autonomic nervous system activity in a subject, wherein the radioactive tracer is for use in combination with at least one additional radioactive tracer, such that data obtained by the radioactive tracer which selectively binds to synapses of the autonomic nervous system describes a distribution and/or neuronal activity of at least one synapse of the autonomic nervous system and data obtained by the at least one additional radioactive tracer describes a distribution and/or activity different than the distribution and/or neuronal activity of the at least one synapse.

Also described herein is a radioactive tracer which selectively binds to synapses of the autonomic nervous system, for use in of determining a normal autonomic nervous system activity or abnormal autonomic nervous system activity in a subject, wherein the radioactive tracer is for use in combination with at least one additional radioactive tracer, such that a first data is obtained by the radioactive tracer which selectively binds to synapses of the autonomic nervous system and describes a distribution and/or neuronal activity of at least one synapse of the autonomic nervous system and at least a second data, obtained by the at least one additional radioactive tracer, describes a distribution and/or activity different than the first data.

The at least one additional radioactive tracer may selectively bind to synapses of the autonomic nervous system.

A radioisotope of the radioactive tracer and a radioisotope of the at least one additional radioactive tracer may be different from one another.

The at least second data may describe a type or an activity of synapses different than the type of an activity of the synapses of the first data.

The at least one additional radioactive tracer may not selectively bind to synapses.

A combination of the first data and the at least second data may be indicative of normal or abnormal synapse distribution and/or neuronal activity in relation to a tissue distribution and/or activity indicated by the additional radioactive tracer which does not selectively bind to synapses.

Also described herein is a kit for determining a normal autonomic nervous system activity or abnormal autonomic nervous system activity in a subject, the kit comprising a first radioactive tracer which selectively binds to synapses of the autonomic nervous system and at least one additional radioactive tracer, wherein the radioactive tracer which binds selectively to synapses of the autonomic nervous system is useful for obtaining a first data describing a distribution and/or neuronal activity of at least one synapse of the autonomic nervous system, and wherein the at least one additional radioactive tracer is useful for obtaining data describing a distribution and/or activity different than the first data.

The at least one additional radioactive tracer may selectively bind to synapses of the autonomic nervous system.

A radioisotope of the radioactive tracer and a radioisotope of the at least one additional radioactive tracer may be different from one another.

The at least second data may describe a type or an activity of synapses different than the type of an activity of the synapses of the first data.

The kit may be for determining a normal or abnormal correlation between sympathetic nervous system activity and parasympathetic nervous system activity.

The at least one additional radioactive tracer may not selectively bind to synapses.

A combination of the first data and the at least second data may be indicative of normal or abnormal synapse distribution and/or neuronal activity in relation to a tissue distribution and/or activity indicated by the additional radioactive tracer which does not selectively bind to synapses.

The kit may be for determining a normal or abnormal synapse activity in relation to an activity of a tissue innervated by the synapse activity.

The radioactive tracers may be packaged individually within the kit.

The radioactive tracer which binds selectively to synapses in the autonomic nervous system may be mIBG.

Also described herein is a radiolabeled mIBG (metaiodobenzylguanidine), for use in an imaging method of identifying autonomic nervous system ganglia in size of between 1 and 20 mm in maximal diameter.

The imaging method may be used for mapping a distribution and/or activity of autonomic nervous system synapses and/or ganglia.

The imaging method may be used for displaying a map of a distribution and/or activity of autonomic nervous system synapses and/or ganglions.

The method may further comprise overlaying the map on an image of an organ.

The imaging method may further comprise determining abnormal synapse distribution and/or activity by comparing the distribution with a set of distributions which is indicative of a normal synapse distribution and/or with a set of distributions which is indicative of a medical condition or disease.

The imaging method may further comprise stimulating an autonomic nervous system in conjunction with the imaging.

The imaging method may be used for diagnosing and/or monitoring a medical condition or disease associated with an autonomic nervous system.

Also described herein is a radiolabeled mIBG (metaiodobenzylguanidine), for use in a method of diagnosing and/or monitoring a medical condition or disease associated with an autonomic nervous system.

The method may comprise identifying a change in a nervous tissue between two imaging sessions.

The treatment of the medical condition or disease may be effected between the two imaging sessions.

The imaging method may be for use in guiding a therapy of the medical condition or disease.

Also described herein is a radiolabeled mIBG (metaiodobenzylguanidine), for use in a method of guiding a therapy of a medical condition or disease associated with an autonomic nervous system.

The medical condition or disease may be caused, exacerbated or sustained by an input or involvement of an autonomic nervous system.

The medical condition or disease may be selected from the group consisting of hypertension, cardiac arrhythmias, diabetes, stress and irritable bowel syndrome.

Also described herein is a radiolabeled mIBG (metaiodobenzylguanidine), for use in an imaging method of locating a nervous tissue which comprises at least one ganglionic plexus (GP).

The imaging method may be used for identifying an innervated tissue, the method comprising: collecting radiation emission data from tissue, assigning the data to spatial locations according to a model of a structure of an organ and identifying nervous tissue synapses and/or innervations based on data associated with the organ.

Locating the nervous tissue may be performed according to a combination of an imaging data and anatomical data.

The anatomical data may be generated by an imaging modality selected from a group consisting of a positron emission tomography (PET) modality, a computerized tomography (CT) modality, a magnetic resonance imaging (MRI) modality, and an ultrasound modality.

Locating the nervous tissue may comprise identifying at least one region of interest (ROI) in an intrabody area or volume that comprises the nervous tissue according to a match with a reference value representing a reference uptake rate of the radiolabeled mIBG by an organ.

Locating the nervous tissue may be used for targeting a sub-region in an intrabody area that comprises the nervous tissue as a target for a medical treatment.

The medical treatment may be an ablation of at least one ganglionic plexus.

Also described herein is a radiolabeled mIBG (metaiodobenzylguanidine) for use in an imaging method of guiding a treatment procedure in a target nervous tissue which comprises at least one ganglion plexus of a patient.

The imaging method may comprise obtaining radioimaging data of an intrabody volume which comprises the target nervous tissue in the patient, acquiring a location of an intra-body treatment probe in the intrabody volume, and presenting the radioimaging data and the intra-body treatment probe location to an operator during the treatment procedure.

Also described herein is a nuclear imaging apparatus, configured to control any one of the imaging methods as described, including any one of the embodiments thereof.

Unless otherwise defined, all technical and/or scientific terms used herein have the same meaning as commonly understood by one of ordinary skill in the art to which the invention pertains. Although methods and materials similar or equivalent to those described herein can be used in the practice or testing of embodiments of the invention, exemplary methods and/or materials are described below. In case of conflict, the patent specification, including definitions, will control. In addition, the materials, methods, and examples are illustrative only and are not intended to be necessarily limiting.

Implementation of the methods and/or systems disclosed herein can involve performing or completing selected tasks manually, automatically, or a combination thereof. Moreover, according to actual instrumentation and equipment of embodiments of the methods and/or systems, several selected tasks could be implemented by hardware, by software or by firmware or by a combination thereof using an operating system.

For example, hardware for performing selected taskscould be implemented as a chip or a circuit. As software, selected tasks could be implemented as a plurality of software instructions being executed by a computer using any suitable operating system. One or more tasks of methods and/or systems as described herein may be performed by a data processor, such as a computing platform for executing a plurality of instructions. Optionally, the data processor includes a volatile memory for storing instructions and/or data and/or a non-volatile storage, for example, a magnetic hard-disk and/or removable media, for storing instructions and/or data. Optionally, a network connection is provided as well. A display and/or a user input device such as a keyboard or mouse are optionally provided as well.

### BRIEF DESCRIPTION OF THE DRAWINGS

Some embodiments of the invention and disclosure are herein described, by way of example only, with reference to the accompanying drawings. With specific reference now to the drawings in detail, it is stressed that the particulars shown are by way of example and for purposes of illustrative discussion of embodiments of the invention. In this regard, the description taken with the drawings makes apparent to those skilled in the art how embodiments of the invention may be practiced.

In the drawings:
FIG. 1 is a flowchart of a method of localizing nervous tissue based on a combination of anatomical data and SPECT data of an intrabody volume, according to some examples of the disclosure ;
FIG. 2 is a flowchart of a method of localizing a nervous tissue based on an association of different regions in a SPECT image to different organs and/or tissues based on a mapping function, according to some examples of the disclosure;
FIG. 3 is a flowchart of a method of obtaining autonomic nervous system (ANS) information, in accordance with some examples of the disclosure;
FIG. 4 is a flow chart of a computer-implemented method for combining functional and anatomical images and/or locating synaptic centers, in accordance with some examples of the disclosure;
FIG. 5 is a flowchart of a method for performing an ablation treatment by mapping complex fractionated atrial electrogram (CFAE) sites, contractile force (CF) sites, and/or dominant frequency (DF) sites in the atria as target areas for a treatment, such as ablation, according to some examples of the disclosure;
FIGs. 6A-6D present images of a left atrium and left ventricle viewed from different angles, in which the left atrium is colored in accordance with mIBG activity according to an exemplary disclosure (green = low activity, red = high activity), showing a maximal activity level in the left inferior pulmonary vein;
FIGs. 7A and 7B present images of a left atrium viewed from different angles, in which the left atrium is colored in accordance with mIBG activity according to an exemplary disclosure (green = low activity, red = high activity);
FIGs. 8A and 8B present images of a right ventricle and left ventricle viewed from different angles, in which the right ventricle is colored in accordance with mIBG activity according to an exemplary disclosure, showing a maximal activity level in the interventricular septum;
FIGs. 9A-9C present images of a heart showing locations of three ganglionated plexuses (GP) identified according to an exemplary disclosure, where each one of FIGs. 9A-9C includes (from left to right) a transverse cut image, a coronal cut image and a sagittal cut image showing the location of one of the three GPs;
FIGs. 10A-10C show the three GP locations of FIGs. 9A-9C (as circles) on an anatomical map of the heart, according to an exemplary disclosure(IVC = inferior vena cava; LA = left atrium; LV = left ventricle; PA = pulmonary artery; RA = right atrium; SVC = superior vena cava);
FIG. 11 depicts an application site of high frequency stimulation (HFS) (circle having dashed pattern) on a 3D simulation of the heart of a patient, according to some examples of the disclosure;
FIG. 12 is an electrocardiogram showing a negative response to the application of HFS at the application site depicted in FIG. 11;
FIGs. 13A and 13B depict an application site of high frequency stimulation (HFS) (circle having dashed pattern) on a 3D simulation of the heart of a patient viewed from different angles, in which the application cite is associated with a RIPV (right inferior pulmonary vein) ganglionated plexus, according to some examples of the disclosure
FIG. 14 is an electrocardiogram showing a positive response to the application of HFS at the application site depicted in FIGs. 13A and 13B;
FIGs. 15A and 15B depict a site of ablation (circle having dashed pattern) associated with a LIPV ganglionated plexus on a 3D simulation of the heart of a patient viewed from different angles, according to some examples of the disclosure;
FIG. 16 is an electrocardiogram showing a negative response to application of HFS at the ablation site depicted in FIGs. 15A and 15B;
FIG. 17 presents SPECT images of a left atrium, showing sestamibi distribution (left image), mIBG distribution (middle image) and a degree of correlation between sestamibi and mIBG distribution (right image) in accordance with an exemplarydisclosure;
FIG. 18 presents SPECT images of a left ventricle, showing sestamibi distribution (left image), mIBG distribution (middle image) and a degree of correlation between sestamibi and mIBG distribution (right image) in accordance with an exemplarydisclosure;
FIG. 19 is a flowchart of a method of diagnosing and treating benign prostatic hyperplasia based on a combination of pelvic anatomical data and radioligand data, according to some examples of the disclosure;
FIG. 20 is a flowchart of a method of diagnosing and treating erectile disorder based on a combination of pelvic anatomical data and radioligand data, according to some examples of the disclosure;
FIG. 21 is a flowchart of a method of diagnosing and treating diabetes based on a combination of abdominal anatomical data and radioligand data, according to some examples of the disclosure;
FIG. 22 is a flowchart of a method of diagnosing and treating arthritis based on a combination of abdominal anatomical data and radioligand data, according to some examples of the disclosure; and
FIG. 23 is a flowchart of a method of diagnosing and treating irritable bowel syndrome based on a combination of abdominal anatomical data and radioligand data, according to some examples of the disclosure.

### DESCRIPTION OF THE DISCLOSURE AND EMBODIMENTS OF THE INVENTION

Disclosed herein are methods and systems of imaging and, more particularly, but not exclusively, methods and systems of medical imaging using a radioactive tracer with affinity to nervous tissue.

The present inventor has envisioned that radioactive tracers with affinity to nervous tissue can be utilized for providing information regarding the distribution, precise location and/or activity of autonomic nervous system (ANS) synaptic centers (e.g., ANS ganglia) *in vivo.* In particular, emission of radioactive tracers in the body may be measured by nuclear imaging techniques (e.g., PET, SPECT), and the obtained data can be analyzed in a manner designed to identify the synaptic centers in an image, thereby revealing the distribution, location and/or activity the synaptic centers. The inventor has further envisioned that such information can be of great importance in the diagnosis and/or treatment of a wide variety of conditions associated with abnormal ANS activity.

While reducing the invention to practice, the inventor has obtained radioactive tracer data, which, when obtained using known methodologies, lacks sufficient resolution to identify individual synaptic centers, from the heart region of a subject, and analyzed the data so as to identify and image cardiac ganglionated plexuses. The inventor has further demonstrated the utility of such images in ablation of cardiac ganglionated plexuses.

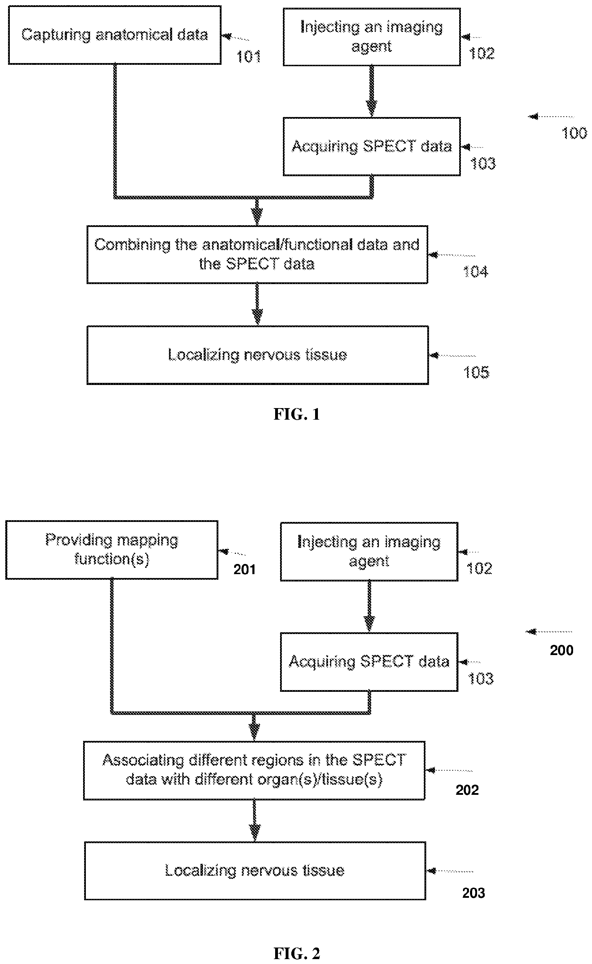

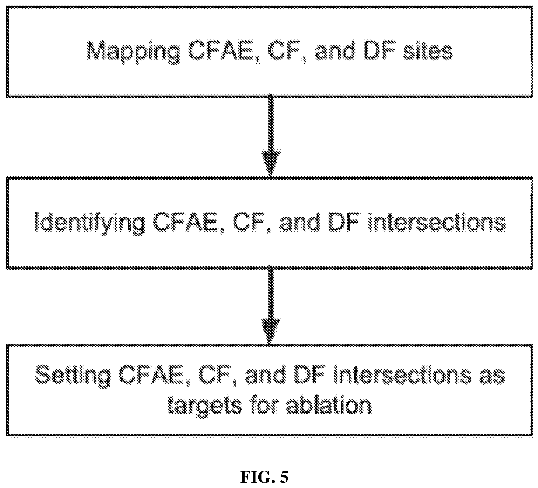

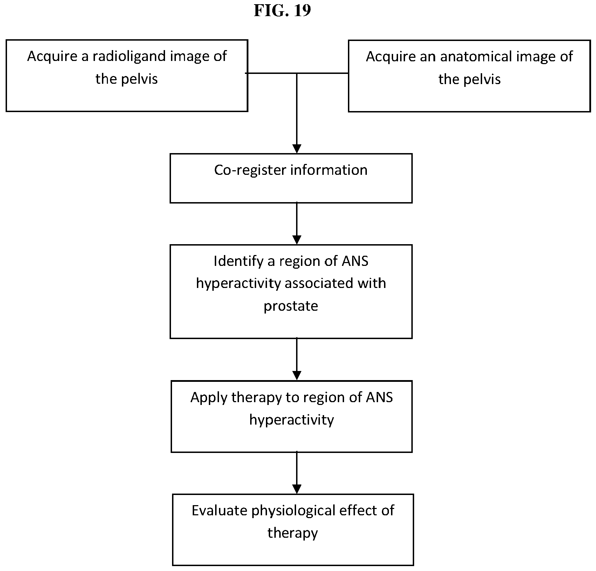

FIGs. 1-4 show flowcharts of methods of obtaining autonomic nervous system (ANS) information, in accordance with some examples of the disclosure. FIG. 5 shows a flowchart of a method for performing an ablation treatment, according to some examples of the disclosure. FIGs. 19-23 show flowcharts of methods of diagnosing and treating various conditions associated with ANS activity, in accordance with some examples of the disclosure.

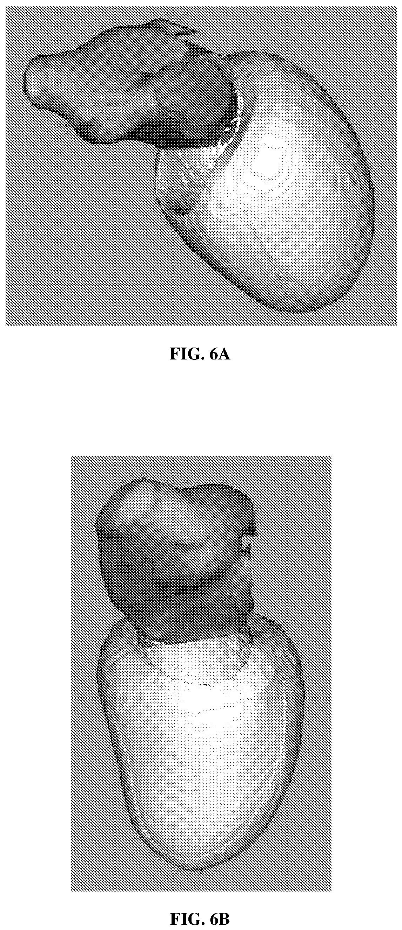

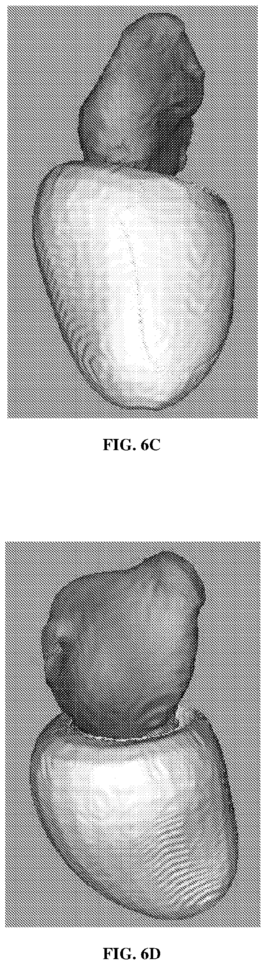

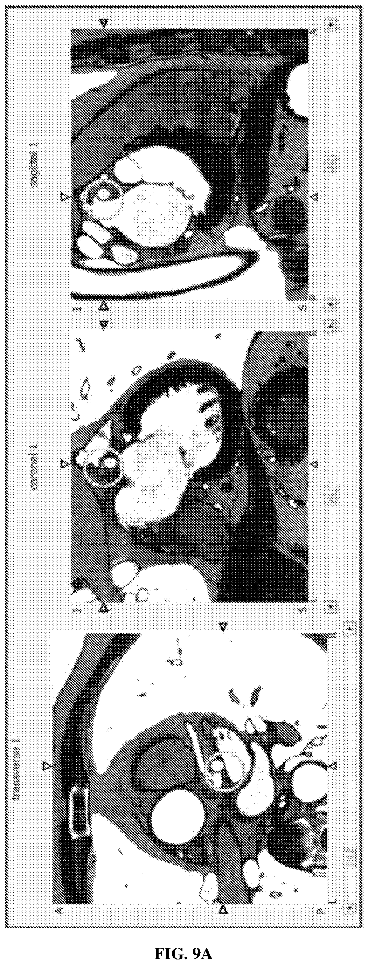

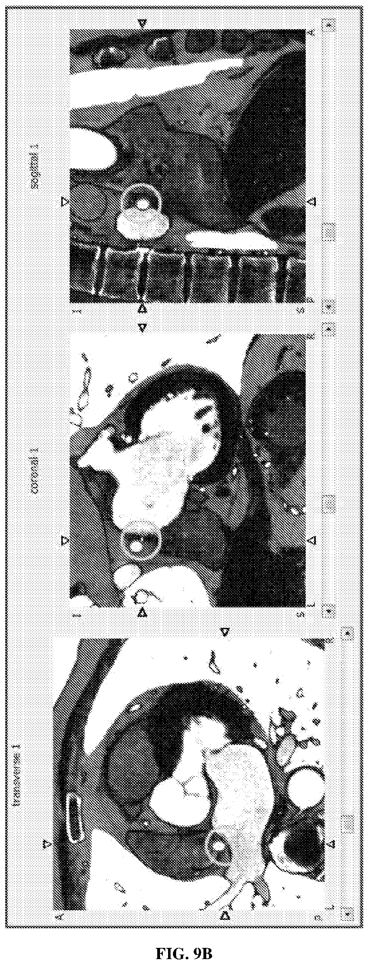

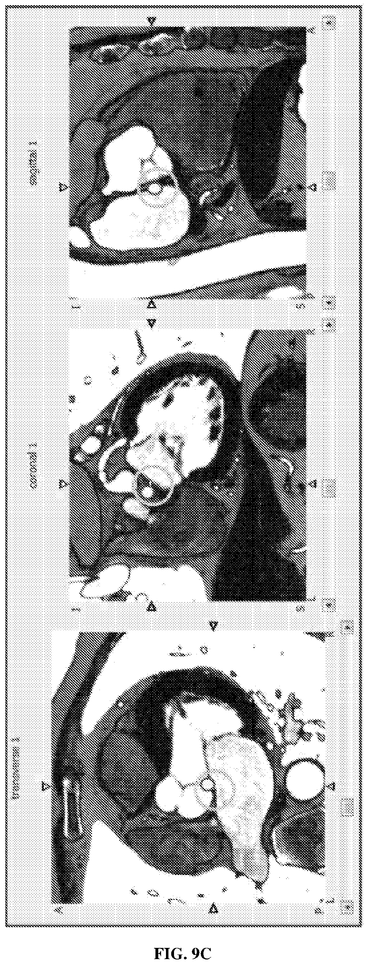

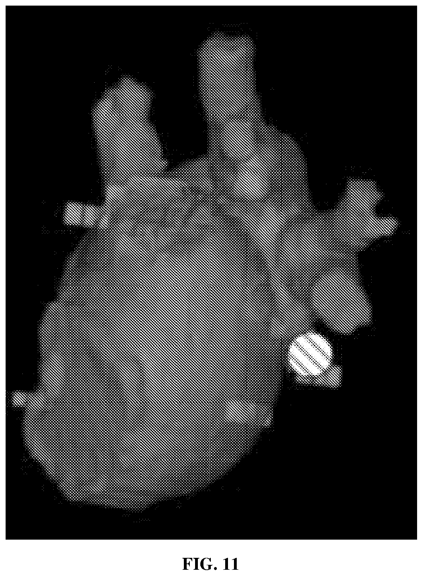

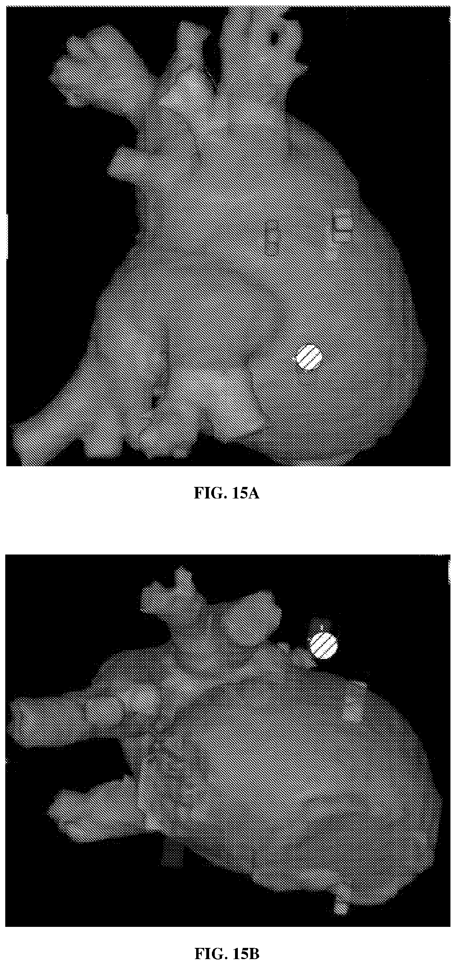

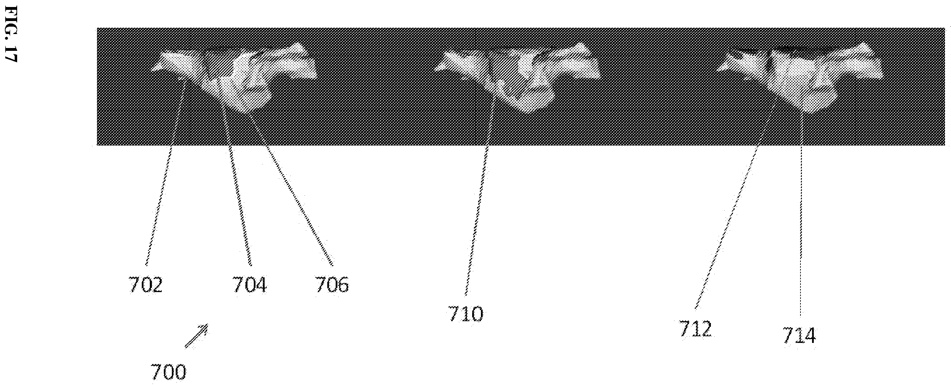

FIGs. 6A-8B present images of heart tissue which depict regions exhibiting a high concentration of a radioactive tracer (mIBG) which exhibits affinity to adrenergic synapses, according to some examples of the disclosure. FIGs. 9A-10C present images of heart tissue which depict the location of cardiac ganglionated plexuses, as determined according to some examples of the disclosure. The accuracy of this technique was confirmed by high frequency stimulation of individual sites, as shown in FIGs. 11-14. FIGs. 15A-16 depict a successful ablation of a cardiac ganglionated plexus, according to some embodiments of the present invention. FIGs. 17-18 present images of heart tissue which depict levels of a radioactive tracer (mIBG) which exhibits affinity to adrenergic synapses, levels of a radioactive imaging agent (sestamibi) which indicates perfusion, and correlations between the distributions of the mIBG and sestamibi.

These results indicate that embodiments of the present invention, and examples of the disclosure, are useful in accurately imaging features of nervous tissue, including ganglia, ganglionated plexuses and other regions rich in synapses.

It is to be appreciated that nervous tissue, such as individual ganglia, is difficult to image or otherwise identify using conventional methods, particularly in view of the relatively small dimensions of nervous tissue, poor uptake of conventional imaging agents relative to surrounding tissue, and lack of physical features which distinguish the nervous tissue from surrounding tissue. For example, cardiac ganglia are commonly surrounded by fatty connective tissue adjacent to epicardial muscle and/or imbedded in a fat pad overlying a posterior surface of the heart, and the close proximity of these ganglia to a fat layer is a significant obstacle to identifying the ganglia. This obstacle can be overcome by some embodiments of the present invention.

According to an aspect of some examples of the disclosure, there is provided a method of imaging nervous tissue comprising at least one synaptic center, the method comprising the use a radioactive tracer (e.g., as described herein) which selectively binds to synapses of the autonomic nervous system. The method may comprise imaging at least one synaptic center. At least two radioactive tracers (e.g., as described herein) which selectively bind to synapses may be used. For example, the radioactive tracers may optionally bind to different synapse types (e.g., adrenergic vs. cholinergic, parasympathetic vs. sympathetic), and/or provide different information regarding synapses (e.g., location vs. activity). One or more radioactive tracers (e.g., as described herein) which selectively bind to synapses may be used in combination with at least one radioactive tracer which does not selectively bind to synapses, for example, facilitating acquisition of information regarding anatomical features other than synapses. Anatomical features other than synapses which may optionally be imaged include nervous tissue features other than synapses (e.g., myelin) and features not associated with nervous tissue (e.g., as described herein). The imaging may be 3-dimensional imaging.

Radiolabeled metaiodobenzylguanidine (mIB G) is an exemplary radioactive tracer.

As used, herein, the phrase "nervous tissue" refers to tissue characterized by the presence of neurons. Examples of nervous tissue include, without limitation, synaptic centers (as defined herein), ganglia, neural fibers, neural synapses, neural sub-systems (e.g., an autonomic sub-system (such as the sympathetic and the parasympathetic autonomic sub-systems), a peripheral sub system), and organ-specific nervous tissues, such as a carotid body, aortic arch, pulmonary, renal, splenic, hepatic, inferior mesenteric, superior mesenteric, muscular and/or penile nervous tissue.

The nervous tissue and/or synaptic center imaged by the method may be associated with (e.g., part of) the autonomic nervous system. The nervous tissue and/or synaptic center may be associated with (e.g., part of) the sympathetic nervous system. Alternatively or additionally, the nervous tissue and/or synaptic center is associated with (e.g., part of) the parasympathetic nervous system.

Herein, the term "synaptic center" refers to a region in the body, outside the central nervous system, characterized by a high concentration (e.g., relative to the surrounding tissue) of synapses (e.g., ANS synapses). Examples of a synaptic center include, without limitation, a ganglion, a ganglionated plexus, a neuromuscular junction and any other aggregate of synapses innervating an organ.

As used herein, the phrases "ganglionated plexus" and "ganglionic plexus", which are used interchangeably, refer to a plurality of interconnected ganglia.

The region may have a diameter of no more than 20 mm. A maximal diameter (e.g., in at least one cross-sectional dimension) of an identified synaptic center (e.g., ganglia) may be between 1 and 20 mm.

The imaging may comprise identifying the nervous tissue comprising at least one synaptic center. The nervous tissue may comprise at least one ganglion. The nervous tissue may comprise at least one ganglionated plexus (GP).

The imaging may comprise identifying at least one synaptic center.

The method may comprise identifying ganglia.the method may comprise identifying autonomic nervous system ganglia.

Herein, the phrase "identifying[...] ganglia" encompasses identifying one or more synaptic centers, wherein each synaptic center may be an individual ganglion and/or comprise a plurality of ganglia (e.g., a ganglionated plexus). In some cases, the difference between an individual ganglion and a synaptic center comprising a plurality of ganglia (e.g., a ganglionated plexus) is merely semantic (e.g., wherein different people in the art use different terminology) and/or of no significant medical importance.

The synaptic center may be selected from the group consisting of an autonomic ganglion and an autonomic ganglionated plexus.

Herein, the terms "autonomic ganglion" and "autonomic ganglionated plexus" refer to a ganglion or ganglionated plexus, respectively, which is a part of the autonomic nervous system. The adrenal medulla is considered herein as an autonomic ganglion.

The method is optionally effected by administering to the subject a radioactive tracer, and measuring radioactive emission (e.g., via single-photon emission computed tomography (SPECT) and/or positron emission tomography (PET)) of the radioactive tracer to obtain data describing a distribution of the radioactive tracer in the body, thereby imaging the radioactive tracer in the body (e.g., imaging a distribution of radioactive tracer in the body).

The method optionally further comprises analyzing the data (e.g., as described herein) in order to identify at least one region exhibiting a high concentration of a radioactive tracer, thereby imaging at least one synaptic center.

The method may further comprise evaluating a neuronal activity in at least one synaptic center.

The method may be a method of locating the nervous tissue. The nervous tissue may be a nervous tissue comprising at least one ganglionic plexus. The radioactive tracer may be mIBG.

The method may comprise locating at least one synaptic center (e.g., a ganglionic plexus).

A sub-region in an intrabody area that comprises the located nervous tissue may be targeted for a medical treatment. An exemplary medical treatment is ablation of at least one synaptic center (e.g., a ganglionic plexus).

As used herein, "locating" or "localizing" (these two terms being used interchangeably) a nervous tissue, synaptic center, ganglion and/or GP refers to characterizing the location of a nervous tissue, synaptic center, ganglion and/or GP identified according to any one of the embodiments described herein. The location may be characterized relative to one or more reference locations, such as, for example, one or more anatomical locations (e.g., organ(s) or other synaptic centers) in the body of the subject, a location of a medical device placed in the body or near the body of the subject, and/or a location of a person other than a subject (e.g., a surgeon and/or a practitioner of the method), for example, relative to the field of vision of the person. An imaged distribution and/or neuronal activity may be overlaid on an anatomical map.

The method may be for guiding a treatment procedure in a target nervous tissue (e.g., as described in more detail herein). The nervous tissue may comprise at least one ganglionic plexus. Guiding may be effected, for example, by obtaining data from the radioactive tracer (e.g., mIBG) in an intrabody volume which comprises the target nervous tissue (e.g., as described herein), acquiring a location of an intra-body treatment probe in the intrabody volume, and presenting the radioactive tracer data and the intrabody treatment probe location to an operator during the treatment procedure. The procedure may be a catheterization procedure.

The method may further comprise generating, and optionally displaying, a map showing a distribution and/or neuronal activity of synapses of the autonomic nervous system, the map including the at least one synaptic center. A resolution of the map may be in a range of from 1 mm to 20 mm.

The method may be used for imaging a synaptic center characterized by adrenergic activity. The method may optionally image only synaptic centers characterized by adrenergic activity, or alternatively, further image synaptic centers not characterized by adrenergic activity, such as cholinergic synaptic centers (e.g., based on selection of suitable radioactive tracers, for example, as described herein).

The method may bes used for imaging a synaptic center characterized by cholinergic activity (e.g., nicotinic and/or muscarinic cholinergic activity). The method may optionally image only synaptic centers characterized by cholinergic activity e.g., nicotinic and/or muscarinic cholinergic activity), or alternatively, further image synaptic centers not characterized by cholinergic, such as adrenergic synaptic centers (e.g., based on selection of suitable radioactive tracers, for example, as described herein).

In some examples, wherein analyzing the data comprises reconstructing an anatomical image of a region of a body of the subject, the region comprising a portion of at least one internal body part; processing the anatomical image to generate at least one image mask corresponding to dimensions of at least one internal body part; and correlating the generated image mask(s) with the data describing a distribution of the radioactive tracer in the body, for guiding a reconstruction of an image depicting at least one synaptic center, for example, according to an example described herein which utilizes such a mask.

In some examples according to any one of the methods or systems described herein, imaging is performed using more than one radioactive tracer. Two radioactive tracers may be used (e.g., as described herein).

For example, a method according to any one of the examples described herein may comprise administering and imaging a first radioactive tracer which selectively binds to synapses of the autonomic nervous system (e.g., as described herein), and further comprises administering and imaging at least one additional radioactive tracer (e.g., a radioactive tracer and/or imaging agent described herein). In some embodiments, a radioactive isotope of said first radioactive tracer and a radioactive isotope of each of said at least one additional radioactive tracer are different from one another. An additional radioactive tracer may optionally be a radioactive tracer exhibiting an affinity to synapses (e.g., a radioactive tracer described herein), for example, synapses of the ANS; or a radioactive tracer which does not exhibit an affinity to synapses (e.g., a radioactive imaging agent described herein).

The first radioactive tracer may selectively bind to synapses which are different than the synapses selectively bound by said at least one additional radioactive tracer. Examples of different types of synapses (e.g., adrenergic vs. cholinergic, sympathetic vs. parasympathetic, muscarinic vs. nicotinic) and radioactive tracers which bind each type are described herein in detail.

Two or more radioactive tracers which bind to a synapse may be used to provide information regarding different synapse activities. For example, one radioactive tracer may be used for imaging activity of a synaptic center (e.g., a tracer providing a signal relatively sensitive to synapse activity), whereas another radioactive tracer may be used for imaging synapse distribution, e.g., location and/or synapse density (e.g., a tracer providing a signal relatively insensitive to synapse activity). Additionally or alternatively, different tracers may be suitable for imaging different types of activity, for example, responses to different types of stimulus (e.g., as described herein).

A radioactive tracer which does not exhibit an affinity to synapses may be used, for example, to provide information about anatomical features other than synapses. In some examples, such information may be used to characterize a location of an anatomical feature (e.g., for locating a synaptic center as described herein). In some examples, such information may be used to characterize an activity of an anatomical region, for example, vitality, functionality, and/or metabolic activity of anatomical region.

Different radioactive tracers may be used in combination have different radioactive isotopes (e.g., 1-123 and Tc-99m, as exemplified herein), which allows for concurrent measurement of the different tracers while distinguishing between the tracers based on an energy of radioactive emission.

Imaging of different radioactive tracers used in combination may not be effected by concurrent measurement. For example, one tracer may be administered and imaged, and another tracer may then be imaged after the signal of the first radioactive tracer has weakened sufficiently so as not to interfere considerably with the measurement of the other tracer.

A combination of information acquired from two or more radioactive tracers (as described herein) may be useful for providing information which is not provided by a single radioactive tracer. For example, a relationship between two types of information provided by different tracers may be important for characterizing ANS activity. In some embodiments, normal ANS activity is characterized by a certain relationship between two parameters reflecting results acquired with different tracers, whereas an abnormal ANS activity is characterized by a different-than-normal relationship (e.g., a "mismatch") between such parameters. For example, an abnormal relationship between the parameters may reflect an imbalance between different types of ANS activity (e.g., adrenergic vs. cholinergic activity and/or sympathetic vs. parasympathetic activity) and/or an imbalance between an ANS activity and an activity of an innervated tissue (e.g., synaptic stimulation of dysfunctional cardiac tissue may have deleterious effects, such as arrhythmia associated with an inability of the dysfunctional tissue to transmit electrical pulses in an appropriate manner).

Also described herein is a method of determining a normal autonomic nervous system activity or abnormal autonomic nervous system activity in a subject. The method comprises administering a first radioactive tracer which selectively binds to synapses of the autonomic nervous system (e.g., as described herein) and imaging at least one synaptic center of the autonomic nervous system (e.g., using an imaging method according to any one of the embodiments described herein), to thereby obtain data describing a distribution of the first radioactive tracer in the body; and administering at least one additional radioactive tracer (e.g., any one or more additional radioactive tracers described herein) and imaging the additional radioactive tracer(s) in at least a portion of the body of the subject, to thereby obtain data describing a distribution of the additional radioactive tracer(s) in the body. A combination of the data describing a distribution of the first radioactive tracer in the body and the data describing a distribution of the additional radioactive tracer(s) in the body is indicative of normal synapse distribution and/or neuronal activity or abnormal synapse distribution and/or neuronal activity in the autonomous nervous system (e.g., as described herein). For example, an abnormal synapse distribution and/or neuronal activity may be determined according to an imbalance between different types of ANS activity (e.g., as described herein) and/or an imbalance between an ANS activity and an activity of an innervated tissue (e.g., as described herein).

In some examples (e.g., those using a plurality of radioactive tracers with affinity to synapses), the combination of distributions of radioactive tracers is indicative of normal or abnormal balance between a sympathetic nervous system activity and a parasympathetic nervous system activity (e.g., as described herein).

At least one additional radioactive tracer may not selectively bind to synapses. In some such examples, the combination of distributions of radioactive tracers is indicative of normal or abnormal synapse distribution and/or neuronal activity in relation to a tissue distribution and/or activity indicated by the additional radioactive tracer which does not selectively bind to synapses (e.g., as described herein).

In some examples according to any of the methods and systems described herein, an abnormal ANS activity is associated with a disease or disorder, for example, any disease or disorders described herein.

Also described herein is a use of a radioactive tracer which selectively binds to synapses of the autonomic nervous system (e.g., as described herein) in the manufacture of a diagnostic agent. The diagnostic agent may be for use in a method described herein. In some examples, the radioactive tracer is for use in combination with at least one additional radioactive tracer (e.g., any one or more additional radioactive tracers as described herein), such that data obtained by the radioactive tracer which selectively binds to synapses of the autonomic nervous system describes a distribution and/or neuronal activity of at least one synapse of the autonomic nervous system and data obtained by the additional radioactive tracer(s) describes a different distribution and/or activity, that is, a distribution and/or activity different than the aforementioned distribution and/or neuronal activity of at least one synapse.

Also described herein is a radioactive tracer which selectively binds to synapses of the autonomic nervous system (e.g., as described herein), for use in a method described herein. In some examples, the radioactive tracer is for use in combination with at least one additional radioactive tracer (e.g., any one or more additional radioactive tracers as described herein), such that a first data is obtained by the radioactive tracer which selectively binds to synapses of the autonomic nervous system (which describes a distribution and/or neuronal activity of at least one synapse of the autonomic nervous system) and at least a second data (optionally a second data and a third data, optionally a second data, third data and fourth data, and so forth), obtained by the additional radioactive tracer(s), which describes a distribution and/or activity different than the first data (e.g., as described herein).

Also described herein is a kit for determining a normal autonomic nervous system activity or abnormal autonomic nervous system activity in a subject (e.g., according to any one of the embodiments described herein). The kit comprising a first radioactive tracer which selectively binds to synapses of the autonomic nervous system (e.g., as described herein) and at least one additional radioactive tracer (e.g., any one or more additional radioactive tracers as described herein). The radioactive tracer which binds selectively to synapses of the autonomic nervous system is useful for obtaining a first data describing a distribution and/or neuronal activity of at least one synapse of the autonomic nervous system, and the additional radioactive tracer(s) is useful for obtaining data describing a distribution and/or activity different than said first data (e.g., as described herein). In some examples, the radioactive tracers are packaged individually within the kit. In some examples, the radioactive tracers are packaged together, for example, formulated as a composition for co-administering the radioactive tracers.

A radioisotope of the first radioactive tracer (according to any of the examples described herein which utilize a plurality of radioactive tracers) and a radioisotope of the additional radioactive tracer(s) may be different from one another, e.g., as described herein.

The (at least) second data may describe a type or an activity of synapses different than the type of an activity of synapses described by the first data (e.g., different activities as described herein).

A combination of radioactive tracers (e.g., according to a use, method, kit or radioactive tracer described herein) may be utilized for determining a normal or abnormal correlation (e.g., balance or imbalance) between sympathetic nervous system activity and parasympathetic nervous system activity, for example, when the radioactive tracers each bind to synapses (e.g., as described herein).

In some examples, a combination of radioactive tracers (e.g., according to a use, method, kit or radioactive tracer described herein) is utilized for determining a normal or abnormal synapse activity (e.g., as determined by a radioactive tracer with affinity to synapses) in relation to an activity of a tissue innervated by said synapse activity (e.g., as determined by a radioactive tracer which binds to the tissue and not to synapses), for example, a balance or imbalance between innervation and activity of an innervated tissue (e.g., as described herein).

A radioactive tracer which binds selectively to synapses in the autonomic nervous (e.g., according to a use, method, kit or radioactive tracer described herein) may be mIBG. The use of mIBG in combination with a Tc-99m containing imaging agent is exemplified herein.

Also described herein is a nuclear imaging apparatus, configured to control an imaging method described herein. The apparatus comprises a nuclear imaging modality (e.g., a SPECT and/or PET modality) configured for data acquisition as described herein. In some examples, the apparatus further comprises a modality for acquiring anatomical data as described herein. In some embodiments, the nuclear imaging modality and modality for acquiring anatomical data are configured for co-registering data from the different modalities.

Any one of the radiolabeled compound described herein may be used in combination with any other radiolabeled compound, with any one of the examples or embodiments described herein regarding data acquisition, with any one of the examples or embodiments described herein regarding data analysis and identification of synaptic centers, with any one of the examples or embodiments described herein regarding location of synaptic centers, with any one of the examples or embodiments described herein regarding diagnosis and monitoring, and/or with any one of the examples or embodiments described herein regarding modulation of synaptic center activity.

### Radioactive tracers:

As used herein, the term "radioactive tracer" refers to an atom or molecule comprising a radioactive isotope. Molecules comprising a radioactive isotope are also referred to herein as being "radiolabeled". Unless indicated otherwise, a radioactive tracer described herein exhibits an affinity to nervous tissue (e.g., of the autonomic nervous system), as described herein.

Herein, a "radioactive isotope" refers to a naturally occurring and/or artificially produced atom with an unstable nucleus that undergoes spontaneous decay.

Detection of radiation emitted by a radioactive tracer may be used in the context of a radioimaging technique described herein, for example, PET and/or SPECT.

Alternatively or additionally, the radiation emitted by a radioactive tracer may be used for radiotherapy, for example, killing cells via radiation.

Radioactive isotopes which decay, at least in part, by positron emission are suitable for use in PET (positron emission tomography) techniques (e.g., as described herein). Such isotopes are known in the art, and include, for example, carbon-11 (C-11), nitrogen-13 (N-13), oxygen-15 (O-15), fluorine-18 (F-18), gallium-68 (Ga-68) bromine-76 (Br-76), rubidium-82 (Rb-82), iodine-124 (I-124) and iodine-131 (I-131). C-11, N13, O-15, F-18, Rb-82 and I-124 are examples of isotopes commonly used for PET.

Without being bound by any particular theory, it is believed that radioactive isotopes which release a photon upon decay (e.g., gamma radiation, X-ray radiation) without releasing an energetic massive particle (e.g., an alpha radiation particle, an electron, a positron, a proton and/or a neutron) are particularly suitable for use in SPECT (single photon emission computed tomography) techniques (e.g., as described herein), as energetic massive particles may cause considerable amounts of cellular damage, with little contribution to acquired SPECT data. However, any radioactive isotope which emits radiation which can exit the body can optionally be used for SPECT, including for example, all radioactive isotopes which can be used for PET (e.g., as described herein).

The radioactive isotope may be characterized by decay via an electron capture mechanism and/or is a metastable isotope. Decay of such isotopes are commonly characterized by photon release without release of an energetic massive particle. Examples of isotopes characterized by electron capture decay include, without limitation, nickel-56 (Ni-56), gallium-67 (Ga-67), selenium-75 (Se-75), indium-111 (In-111), iodine-123 (I-123), iodine-125 (I-125), and thallium-201 (T1-201). Metastable technetium-99 (Tc-99m) is an example of a metastable isotope which decays by photon emission. Ga-67, In-111, I-123, T1-201 and Tc-99m are examples of isotopes commonly used for SPECT.

Radioactive isotopes characterized by emission of radiation which does not penetrate far (e.g., about 2 cm or less) in a physiological environment are suitable for radiotherapy (e.g., by selectively irradiating a specific region in a body). Examples of isotopes suitable for radiotherapy include, without limitation, bromine-77 (Br-77), strontium-89 (Sr-89), yttrium-90 (Y-90), iodine-131 (1-131), samarium-153 (Sm-153).

The isotope may be characterized by a half-life of at least 5 minutes. In some embodiments, the isotope is characterized by a half-life of at least 1 hour. Shorter half-lives may present a considerable difficulty in performing measurements in a subject before decay of a large proportion of the isotope.

The isotope may be characterized by a half-life of no more than 7 days. The isotope may be characterized by a half-life of no more than 48 hours. Longer half-lives may result in only a very small proportion of the isotope decaying during measurements in a subject, which may lead to a weak signal.

In some examples, the isotope is characterized by a half-life in a range of from 4 to 48 hours.

In some examples the radioactive isotope is of an element commonly present in organic molecules, such as radioactive carbon, radioactive hydrogen, radioactive oxygen and/or radioactive nitrogen (e.g., C-11, N-13, 0-15) and/or a radioactive halogen atom (e.g., F-18, Br-76, Br-77, 1-123, 1-124, 1-125, 1-131). Halogen atoms are present in many organic molecules and may readily be attached to others, for example, by halogen substitution forming a carbon-halogen covalent bond.

As used herein, the phrase "radioactive carbon" encompasses any radioactive carbon isotopes. C-11 is a non-limiting example of a radioactive carbon suitable for use in nuclear imaging techniques (e.g., PET).

As used herein, the phrase "radioactive hydrogen" encompasses any radioactive hydrogen isotopes.

As used herein, the phrase "radioactive nitrogen" encompasses any radioactive nitrogen isotopes. N-13 is a non-limiting example of a radioactive nitrogen suitable for use in nuclear imaging techniques (e.g., PET).

As used herein, the phrase "radioactive oxygen" encompasses any radioactive oxygen isotopes. 0-15 is a non-limiting example of a radioactive oxygen suitable for use in nuclear imaging techniques (e.g., PET).

As used herein, the phrase "radioactive halogen" encompasses any radioactive halogen (e.g., fluorine, chlorine, bromine, iodine) isotopes. F-18, Br-76, Br-77, 1-123, 1-124, 1-125, 1-131 are non-limiting examples of a radioactive halogen suitable for use in nuclear imaging techniques (e.g., PET in the case of F-18, Br-76, 1-124 and 1-131, and SPECT in the case of I-123 and 1-125).

The radioactive isotope 1-123 is used for SPECT. 1-123 may be characterized by a relatively low gamma radiation energy (159 keV) and a half-life of 13.2 hours which is particularly suitable for SPECT.

The radioactive tracer may be a radio-ligand.

Herein, the term "radio-ligand" refers to a radiolabeled compound which selectively binds a target (e.g., a protein), that is, the radiolabeled compound serves as a ligand of the target. Selective binding to a target may comprise long-term binding (e.g., a ligand which is an agonist or antagonist of a receptor) or short term binding (e.g., a ligand which is transported by a molecular transporter, a substrate of an enzyme). A radio-ligand may optionally be prepared by structural modification of a non-radioactive ligand known in the art, for example, by substituting one group in a known ligand with a group comprising a radioactive isotope (e.g., replacement of a hydrogen, hydroxy, amine or alkyl (e.g., methyl) with a radioactive halogen; replacement of a hydroxy or amine with a radioactive alkyl (e.g., methyl) or radioactive halogen), such that a structure of a radioligand can be different from the non-radioactive ligand known in the art.

In some examples, the radio-ligand selectively binds a target (e.g., a receptor, a molecular transporter) characteristic of certain cell types, such that the radio-ligand facilitates selective imaging of such cell types. In some examples, the radio-ligand selectively binds neurons.

Selective binding of a radioactive tracer to a cell may comprise selectively binding an outer surface of the cell, selectively binding a target within a cell, and/or selective uptake by a cell (e.g., wherein the radioactive tracer remains unbound to any particular target within the cell).

The radioactive tracer (e.g., a radio-ligand as described herein) may selectively bind to ganglia (e.g., ganglionic cells). In some embodiments, the radioactive tracer (e.g., a radio-ligand as described herein) selectively binds to sympathetic ganglia and/or parasympathetic ganglia.

The radioactive tracer (e.g., a radio-ligand as described herein) may selectively bind to neurons. The radioactive tracer (e.g., a radio-ligand as described herein) may selectively bind to neurons of one or more specific types of neuron, for example, a neuron characterized by secretion and/or emission of a specific neurotransmitter.

Metabolism of a radioactive tracer may interfere with selective imaging by releasing radioactive metabolites to various regions of the body in a non-selective manner.

Hence, in some examples, the radioactive tracer is selected such that metabolism of the radioactive tracer and/or release of radioactive metabolites from a cell which binds to the radioactive tracer occur with a relatively long half-life, for example, a least four hours, at least 8 hours, at least 24 hours, at least 48 hours, and even at least 72 hours. Such a half-life may be readily determined by the skilled person by observing changes in location and/or intensity of a radioactive tracer in the presence of a cell which binds to the radioactive tracer.

In some examples, a radioactive tracer comprises a radioactive halogen isotope (e.g., F-18, Br-76, Br-77, 1-123, 1-124, 1-125, 1-131) covalently bound to a carbon atom which is bound via an unsaturated covalent bond to another carbon atom, for example, within an aryl or heteroaryl group, or within an alkenyl group (e.g., a vinyl halide group).

Without being bound by any particular theory, it is believed that halogen-carbon bonds (especially bromine-carbon bonds and iodine-carbon bonds) are particularly resistant to cleavage when the carbon atom is bound via an unsaturated covalent bond to another carbon atom, and that it is advantageous to minimize cleavage which may result in the radioactive halide travelling to regions other than a targeted area, thereby potentially interfering with selective imaging.

The radio-ligand may be an agent known in the art as binding to neurons (e.g., to a neurotransmitter receptor and/or transporter) or a halogenated derivative of such an agent. In some embodiments, the agent or halogenated derivative comprises a halogen atom (e.g., a radioactive halogen isotope described herein) attached to an aromatic ring thereof (e.g., phenyl).

Herein, the term "halogenated derivative" refers to a compound which differs from a parent compound only in that one or more substituents (e.g., hydrogen substituents) attached to a carbon atom have been replaced by a halogen atom and/or in that one or more halogen atoms attached to a carbon atom have been replaced by a different species of halogen atom.

Examples of suitable agents which may serve as suitable radio-ligands when comprising a radioactive isotope (e.g., C-11, N-13, 0-15, F-18, Br-76, Br-77, 1-123, 1-124, 1-125, 1-131) include, for example, neurotransmitters and analogs thereof, vesamicol and derivatives thereof, agonists and antagonists of neurotransmitter receptors, and norepinephrine reuptake inhibitors.

In some examples, the radio-ligand is a neurotransmitter or an analog of a neurotransmitter comprising a radioactive isotope (e.g., an isotope described herein). In some embodiments, the radio-ligand is a neurotransmitter radiolabeled by C-11, 0-13 and/or N-15.

Examples of neurotransmitters include, without limitation, catecholamine neurotransmitters (e.g., dopamine, norepinephrine, epinephrine) and acetylcholine. Such neurotransmitters, especially norepinephrine and acetylcholine, are widely used by the autonomic nervous system.

Many analogs of neurotransmitters (e.g., analogs capable of selectively binding to a neurotransmitter receptor and/or transporter) are known in the art, and may be utilized as radio-ligands in embodiments of the invention.

The analog may be a halogenated derivative of a neurotransmitter (e.g., comprising a radioactive halogen isotope described herein). In some embodiments, the neurotransmitter is substituted on an aromatic ring thereof (e.g., phenyl) by the halogen atom, which may optionally be a radioactive halogen atom.

The analog may be a halogenated derivative of a neurotransmitter analog known in the art (e.g., comprising a radioactive halogen isotope described herein). In some embodiments, the neurotransmitter analog is substituted on an aromatic ring thereof (e.g., phenyl) by the halogen atom, which may optionally be a radioactive halogen atom.

The radio-ligand may be selective for regions of adrenergic activity (e.g., adrenergic synapses). Examples of regions of adrenergic activity include, for example, sympathetic synapses wherein a sympathetic postganglionic neuron communicates with an organ or another neuron (e.g., in a sympathetic or parasympathetic ganglion).

Herein, the term "adrenergic" refers to activity characterized by the use of an epinephrine (adrenaline) derivative, typically norepinephrine, for signaling.

In some examples, the radio-ligand has the general formula I: 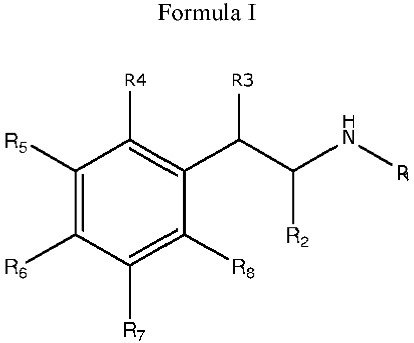 or a pharmaceutically acceptable salt thereof, wherein R₁ is selected from the group consisting of hydrogen and alkyl, and R₂-R₈ are each independently selected from the group consisting of hydrogen, alkyl, hydroxy, alkoxy and halo (e.g., iodo, bromo, fluoro), and at least one atom is radioactive (e.g., radioactive carbon, radioactive nitrogen, radioactive oxygen, and/or radioactive halogen atom, as described herein).

Each atom in general formula I may be radioactive or non-radioactive. For example, a halogen atom (if present) in general formula I may be radioactive (e.g., F-18, Br-76, Br-77, 1-123, 1-124, 1-125, 1-131) or non-radioactive; a carbon atom (e.g., in an alkyl or aryl) in general formula I may be radioactive (e.g., C-11) or non-radioactive; a nitrogen atom (e.g., in the depicted amine group) in general formula I may be radioactive (e.g., N-13) or non-radioactive; and an oxygen atom (if present, e.g., in a hydroxy or alkoxy) in general formula I may be radioactive (e.g., 0-15) or nonradioactive.

The alkyl may be C1-C₄ alkyl (e.g., methyl, ethyl, n-propyl, isopropyl).

The alkyl may be non-substituted. In some examples, the alkyl is methyl.

An alkyl as described herein may comprise a radioactive carbon (e.g., C-11).

In some examples, when an alkyl as described herein is a substituted alkyl, one or more atoms of the substituent is a radioactive atom. For example, if an alkyl is substituted by an alkoxy group, the oxygen in the alkoxy group can be a radioactive oxygen. In an alkyl substituted by a halide, the halide can be a radioactive halide (e.g., radioactive fluorine, radioactive bromine, radioactive iodine).

In some examples, none of R₂-R₈ is halo, and at least one atom (e.g., one atom) is C-11, N-13, and/or 0-15. In some embodiments, at least one atom (e.g., one atom) is C-11.

R₁ may be an alkyl (e.g., methyl) comprising C-11.

In some examples (e.g., wherein R₁ is hydrogen), the carbon atom attached to R₂ and/or the carbon atom attached to R₃ may be C-11.

In some examples, R₄-R₈ are each independently selected from the group consisting of hydrogen, hydroxy and halo (e.g., a radioactive halogen isotope). In some examples, R₄, R₇ and R₈ are hydrogen or halo. In some examples, R₄ and R₇ are each hydrogen.

At least one of R₄-R₈ may be halo (e.g., a radioactive halogen isotope). In some examples, only one of R₄-R₈ is halo. In some examples, R₆ or R₈ is halo. In some embodiments, neither R₂ nor R₃ is halo.

At least one of R₅ and R₆ may be hydroxy.

In some examples, R₅ is hydroxy and R₆ is hydrogen or halo (e.g., a radioactive halogen isotope). In some examples, R₂ is alkyl (e.g., methyl). In some examples, R₃ is hydroxy.

Examples of compounds in which R₃ and R₅ are hydroxy and R₄ and R₆-R₈ are hydrogen or halo include, without limitation, the norepinephrine analogs metaraminol (wherein R₁, R₄ and R₆-R₈ are hydrogen and R₂ is methyl), phenylephrine (wherein R₂, R₄ and R₆-R₈ are hydrogen and R₁ is methyl) and meta-hydroxyephedrine (wherein R₄ and R₆-R₅ are hydrogen and R₁ and R₂ are methyl), which may be radiolabeled, for example, with C-11, as well as halogenated derivatives thereof such as 4-fluorometaraminol (4-FMR), 6-fluorometaraminol (6-FMR), 4-bromometaraminol (4-BMR), 6-bromometaraminol (6-BMR), 4-iodometaraminol (4-IMR) and 6-iodometaraminol (6-IMR), which may be radiolabeled, for example, with a radioactive halogen isotope described herein.

In some examples R₅ and R₆ are each hydroxy. In some embodiments, R₅ and R₆ are each hydroxy, R₁ is hydrogen or methyl, R₂ is hydrogen, R₃ is hydrogen or hydroxy, and R₄, R₇ and R₈ are each hydrogen or halo. Examples of such compounds include, without limitation, the neurotransmitters dopamine (wherein R₁, R₃, R₄, R₇ and R₈ are each hydrogen), norepinephrine (wherein R₁, R₄, R₇ and R₈ are each hydrogen and R₃ is hydroxy) and epinephrine (wherein R₄, R₇ and R₈ are each hydrogen, R₁ is methyl and R₃ is hydroxy), and the analog epinine (wherein R₁ is methyl and R₃, R₄, R₇ and R₈ are each hydrogen), which may be radiolabeled, for example, with C-11, as well as halogenated derivatives thereof such as 6-fluoronorepinephrine, 6-fluorodopamine and 6-iododopamine, which may be radiolabeled, for example, with a radioactive halogen isotope (e.g., as described herein).

In some examples, R₃ is hydroxy and the carbon atom bound to R₃ has an (R) chirality, as in norepinephrine and epinephrine.

In some examples, R₂ is alkyl (e.g., methyl) and the carbon atom bound to R₂ has an *(S)* chirality, as in catecholamine analogs such as ephedrine and dextromethamphetamine.

In some examples, the carbon atom bound to R₂ and/or the carbon atom bound to R₃ is in a form of a mixture of (*R*) and *(S)* chirality (e.g., a racemate).

In general, compounds wherein R₃ is hydroxy are relatively similar to norepinephrine and epinephrine, and typically exhibit affinity towards norepinephrine and/or epinephrine secreting and/or binding cells, whereas compounds wherein R3 is hydrogen are more similar to dopamine and typically exhibit affinity towards dopamine secreting and/or binding cells. However, dopamine and analogs and halogenated derivatives thereof may also act as a norepinephrine analog, exhibiting affinity to norepinephrine transporter (NET; e.g., neuronal norepinephrine transporter) which is located in norepinephrine secreting cells.

Radio-ligands which comprise radiolabeled norepinephrine or an analog thereof, or a halogenated derivative thereof (e.g., dopamine, epinephrine, epinine, 6-fluoronorepinephrine, 6-fluorodopamine, 6-iododopamine, metaraminol, phenylephrine. meta-hydroxyephedrine, 4-FMR, 6-FMR, 4-BMR, 6-BMR, 4-IMR and 6-IMR) may be used to selectively image norepinephrine transporter (NET; e.g., neuronal norepinephrine transporter) and/or vesicular monoamine transporter (VMAT). VMAT and NET are present, for example, in presynaptic neurons of adrenergic synapses (e.g., in the sympathetic nervous system).

Radio-ligands which comprise radiolabeled dopamine or an analog thereof, or a halogenated derivative thereof (e.g., dopamine, epinine, 6-fluorodopamine, 6-iododopamine) may also be used to image dopamine binding cells, for example, in spleen, bone marrow, kidneys and pancreas.

Norepinephrine reuptake inhibitors and halogenated derivatives thereof may also be used to selectively image norepinephrine transporter (NET).

Examples of norepinephrine reuptake inhibitors (NRIs) include, without limitation, selective NRIs such as amedalin, atomoxetine, CP-39,332, daledalin, edivoxetine, esreboxetine, lortalamine, maprotiline, mazindol, nisoxetine, nomifensine, reboxetine, talopram, talsupram, tandamine and viloxazine, as well as non-selective NRIs such as bupropion, ciclazindol, desipramine, manifaxine, radafaxine, tapentadol and teniloxazine. All of the aforementioned NRIs may be radiolabeled by including a radioactive isotope (e.g., C-11, N-13 and/or O-15).

Manifaxine and edivoxetine are examples of NRIs which comprise fluorine and therefore may be radiolabeled by including F-18.

In some examples, a halogenated derivative of an NRI is an NRI (e.g., an NRI described herein) derivatized by replacing a hydrogen atom (e.g., a hydrogen atom attached to an aromatic ring) with a radioactive halogen atom (e.g., F-18, Br-76, Br-77, 1-123, 1-134, 1-125, 1-131).

In some examples, a halogenated derivative of an NRI is an NRI (e.g., as described herein) derivatized by replacing a halogen atom (e.g., chlorine, fluorine) with a radioactive halogen atom (e.g., F-18, Br-76, Br-77, 1-123, 1-134, 1-125, 1-131). Bupropion, ciclazindol, lortalamine, mazindol and radafaxine are examples of NRIs which may be derivatized by replacing a chlorine atom (e.g., a chlorine atom attached to an aromatic ring) with a radioactive fluorine, bromine or iodine atom. Manifaxine and edivoxetine are examples of NRIs which may be derivatized by replacing a fluorine atom (e.g., a fluorine atom attached to an aromatic ring) with a radioactive bromine or iodine atom.

In some examples, the radio-ligand has the general formula II:

A-B-D-E Formula II

or a pharmaceutically acceptable salt thereof, wherein:
A is selected from the group consisting of aryl and heteroaryl (each being substituted or non-substituted);
B is selected from the group consisting of O, S, NR₁₀ or is absent;
D is selected from the group consisting of alkyl, cycloalkyl, heteroalicyclic, aryl and heteroaryl; and
E is -NR₁₁-C(=NH)NH₂ (a guanidine group), wherein R₁₁ is selected from the group consisting of hydrogen, alkyl, cycloalkyl, heteroalicyclic, aryl and heteroaryl, or alternatively, R₁₁ and R₁₀ are linked together to form a heteroalicyclic or heteroaryl ring comprising B, D and NR₁₁,
wherein at least one atom is radioactive (e.g., radioactive carbon, radioactive nitrogen, radioactive oxygen, and/or radioactive halogen atom, as described herein).

Each atom in general formula II may be radioactive or non-radioactive. For example, a halogen atom (if present) in general formula II may be radioactive (e.g., F18, Br-76, Br-77, 1-123, 1-124, 1-125, 1-131) or non-radioactive; a carbon atom in general formula II may be radioactive (e.g., C-11) or non-radioactive; a nitrogen atom in general formula II may be radioactive (e.g., N-13) or non-radioactive; and an oxygen atom (if present) in general formula II may be radioactive (e.g., 0-15) or nonradioactive..

Such compounds may act as a catecholamine neurotransmitter analog, due to the aryl and heteroaryl at one end of the molecule (which may be analogous to the catechol moiety of a catecholamine) and a positively charged guanidine group at the other end of the molecule (which may be analogous to the amine group of a catecholamine).

In some examples, A is an aryl or heteroaryl substituted by a substituent selected from the group consisting of halo, hydroxy and amine (e.g., -NH₂). In some embodiments, the hydroxy or amine is at the 4-position (para-position) of the aryl or heteroaryl (e.g., phenyl, pyridinyl) with respect to the variables B and D.

In some examples, A is an aryl or heteroaryl substituted only by halo (e.g., by a single halogen atom), that is, all other atoms attached to the aryl or heteroaryl ring are hydrogen. In some embodiments, the halogen is iodine (e.g., 1-123, 1-124, 1-125, 1-131).

In some examples, A is substituted or non-substituted aryl. In some embodiments, A is substituted phenyl (e.g., iodophenyl) or non-substituted phenyl.

In some examples, the halo-substituted phenyl is substituted by halo at the 3-position (meta-position) or 4-position (para-position) with respect to the variables B and D. In some embodiments, the halo-substituted phenyl is substituted by halo (e.g., iodo) at the 3-position (meta-position), for example, 3-iodophenyl, 3-bromophenyl, 3-iodo-4-hydroxyphenyl, 4-fluoro-3-iodophenyl and/or 3-iodo-4-aminophenyl. 3-iodophenyl is an exemplary halo-substituted aryl. In some embodiments, the halo-substituted phenyl is substituted by halo (e.g., fluoro) at the 4-position (para-position), for example, 4-fluorophenyl and/or 4-fluoro-3-iodophenyl.

In some examples, B is NR₁₀ and R₁₁ and R₁₀ are linked together to form a heteroalicyclic or heteroaryl ring. In some embodiments, B, D and NR₁₁ together form a piperazine ring (e.g., a non-substituted piperazine ring).

In some examples, D is methylene (CH₂). In exemplary embodiments, B is absent and D is methylene.

In some examples, such compounds act as a catecholamine neurotransmitter (e.g., norepinephrine) analog, due to the aryl and heteroaryl at one end of the molecule (which may be analogous to the catechol moiety of a catecholamine) and a positively charged guanidine group at the other end of the molecule (which may be analogous to the amine group of a catecholamine).

Radio-ligands having a guanidine group such as the variable E in formula II may be used to selectively image norepinephrine transporter (NET; e.g., neuronal norepinephrine transporter) and/or vesicular monoamine transporter (VMAT), for example, in adrenergic synapses.

Examples of radio-ligands having a guanidine group include, without limitation, m-iodobenzylguanidine (mIB G), m-bromobenzylguanidine (mBBG), *p-*fluorobenzylguanidine (pFBG), 4-fluoro-3-iodobenzylguanidine (FIB G), 4-amino-3-iodobenzylguanidine, 1-amidino-4-phenylpiperazine and 1-amidino-4-(4-iodophenyl)piperazine.

The radio-ligand may be selective for regions of cholinergic activity (e.g., cholinergic synapses). Examples of regions of cholinergic activity include, for example, a sympathetic ganglion, a parasympathetic ganglion, a parasympathetic synapse (where a parasympathetic postganglionic neuron communicates with an organ), a neuromuscular junction (where a somatic neuron communicates with muscle), and a sympathetic synapse of sweat glands.

Herein, the term "cholinergic" refers to activity characterized by the use of a choline derivative, typically acetylcholine, for signaling.

Herein and in the art, the phrase "sympathetic ganglion" refers to a location where neurons of the sympathetic nervous system meet. In particular, neurons connecting the central nervous system and sympathetic ganglion, referred to as "preganglionic neurons" communicate via a (cholinergic) synapse in the ganglion with neurons connecting the ganglion to an organ, referred to as "postganglionic neurons". The phrase "sympathetic ganglion" herein encompasses the adrenal medulla, with the chromaffin cells of the adrenal medulla being considered herein as postganglionic sympathetic cells, which release catecholamines such as epinephrine and norepinephrine into the blood rather than in a synapse.

The radio-ligand may be selective for regions of cholinergic activity characterized by nicotinic acetylcholine receptors. Examples of regions characterized by nicotinic acetylcholine receptors include, for example, a sympathetic ganglion, a parasympathetic ganglion, and a neuromuscular junction.

The radio-ligand may comprise a nicotinic acetylcholine receptor agonist, wherein at least one atom is radioactive (e.g., radioactive carbon, radioactive nitrogen, radioactive oxygen, and/or radioactive halogen atom, as described herein). Examples of nicotinic acetylcholine receptor agonists which may be used as radio-ligands according to some embodiments of the invention include, without limitation, acetylcholine, choline, carbachol, nicotine, epibatidine, tebanicline, 5-IA-85380, SIB-1553A and dimethylphenylpiperazinium (DMPP). DMPP is an example of an agonist selective for ganglion-type nicotinic receptors.

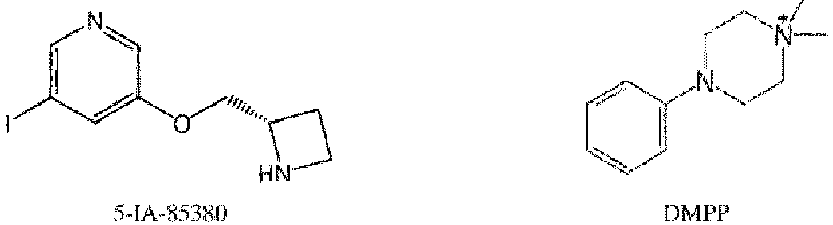

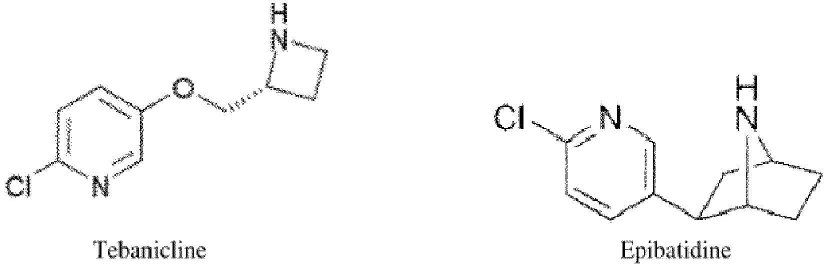

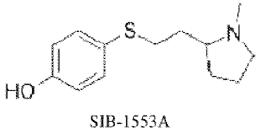

Herein throughout, wherever radioactive tracers (e.g., radio-ligands) are described (e.g., by name and/or formula), it is to be understood that at least one atom in at least a portion of the molecules of the tracer is a radioactive isotope, although the radioactive isotope may not be explicitly indicated by the description (e.g., name and/or formula) of the tracer. Each atom may be radioactive or non-radioactive. For example, a halogen atom (if present) may be radioactive (e.g., F-18, Br-76, Br-77, 1-123, 1-124, 1125, 1-131) or nonradioactive; a carbon atom may be radioactive (e.g., C-11) or nonradioactive; a nitrogen atom (if present) may be radioactive (e.g., N-13) or nonradioactive; and an oxygen atom (if present) may be radioactive (e.g., 0-15) or nonradioactive. It will be apparent to the skilled person which radioactive isotopes (e.g., as described herein) may be included in each tracer described herein. Based in this knowledge, and the guidance provided herein, it will be apparent to the skilled person which radioactive tracers described herein may be used for a given imaging technique such as PET or SPECT. For example, radioactive tracers comprising at least one iodine atom are particularly suitable for SPECT, as such a tracer may comprise 1-123, which is particularly suitable for SPECT.

The radio-ligand may comprise a nicotinic acetylcholine receptor antagonist. Examples of nicotinic acetylcholine receptor antagonists which may be used as radio-ligands according to some embodiments of the invention include, without limitation, GTS-21, AR-R17779, mecamylamine, trimetaphan (e.g., trimetaphan camsilate), hexamethonium, pentolinium, chlorisondamine, pempidine, pentamine, 18-methoxycoronaridine (18-MC), 18-methylaminocoronaridine (18-MAC), 2-methoxyethyl-18 -methoxycoronaridinate (ME-18-MC). Examples of antagonists selective for ganglion-type nicotinic receptors include, without limitation, trimetaphan, hexamethonium, 18-MC, 18-MAC and ME- 18-MC.

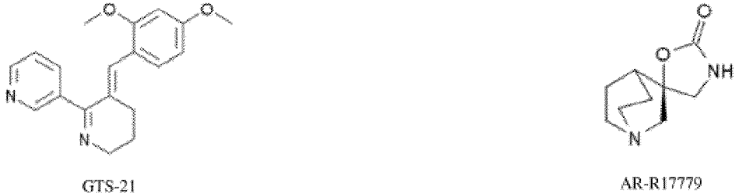

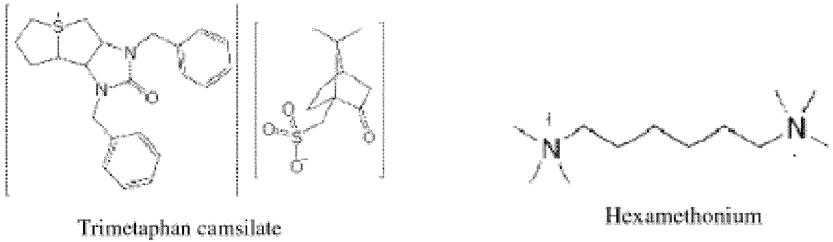

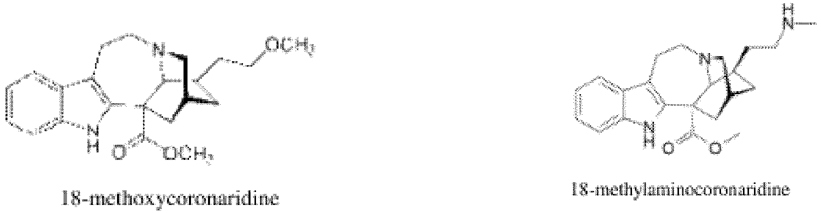

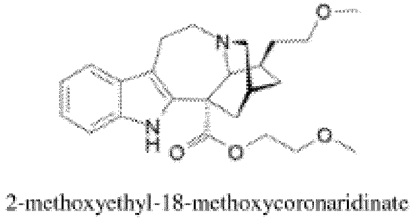

The radio-ligand may be capable of binding to regions of cholinergic activity characterized by ganglion-type nicotinic acetylcholine receptors, which are specific to the ANS ganglia, thereby facilitating imaging of ganglia. In some examples, the radio-ligand selectively binds to regions characterized by ganglion-type nicotinic acetylcholine receptors, thereby facilitating selective imaging of ganglia.

In some examples, the radio-ligand is selective for regions of cholinergic activity characterized by muscarinic acetylcholine receptors. Examples of regions characterized by muscarinic acetylcholine receptors include, for example, a parasympathetic synapse, a neuromuscular junction (where a somatic neuron communicates with muscle), and a sympathetic synapse of sweat glands.

The radio-ligand may comprise a muscarinic acetylcholine receptor agonist.

The radio-ligand may comprise a muscarinic acetylcholine receptor antagonist. Clidinium (and salts thereof), also known in the art as "MQNB", is an example of a muscarinic acetylcholine receptor antagonist suitable for being radiolabeled (e.g., by C-11 at the N-methyl group).

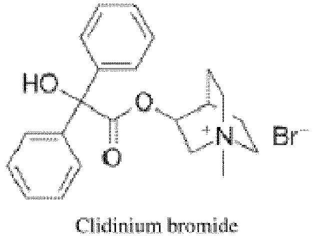

In some examples, the radio-ligand is capable of binding to regions of cholinergic activity characterized by M1 muscarinic acetylcholine receptors, which are associated with exocrine glands and ganglia; M2 muscarinic acetylcholine receptors, which are specific to the heart; and/or M3 muscarinic acetylcholine receptors, which are associated with smooth muscle (e.g., endothelial cells) and lung, thereby facilitating imaging of a particular tissue. In some examples, the radio-ligand selectively binds to regions characterized by M1 muscarinic receptors. In some embodiments, the radio-ligand selectively binds to regions characterized by M2 muscarinic receptors. In some embodiments, the radio-ligand selectively binds to regions characterized by M3 muscarinic receptors.

In some examples, the radio-ligand comprises a mixture of an agonist and antagonist, so as to provide binding to a target while minimizing a net amount of potentially deleterious agonism or antagonism of target activity.

In some examples, the radio-ligand is vesamicol or a vesamicol derivative, such as a benzovesamicol or an azavesamicol.

In some examples, the radio-ligand has the general formula III: 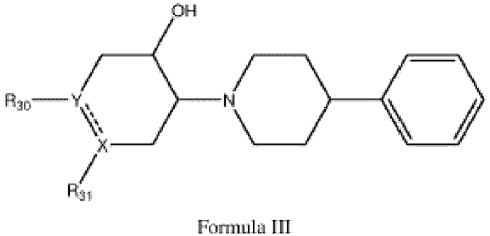
or a pharmaceutically acceptable salt thereof, wherein X and Y are each independently N or CH, the dashed line representing a saturated bond, or alternatively, X and Y are each C, the dashed line representing a saturated bond; and
R₃₀ and R₃₁ are each independently selected from the group consisting of hydrogen, and substituted or non-substituted phenyl, benzyl or benzoyl, or alternatively, R₃₀ and R₃₁ together form a substituted or non-substituted phenyl ring (wherein X and Y are each C and the dashed line represents an unsaturated bond),
wherein at least one atom is radioactive (e.g., radioactive carbon, radioactive nitrogen, radioactive oxygen, and/or radioactive halogen atom, as described herein).

Each atom in general formula III may be radioactive or non-radioactive. For example, a halogen atom (if present) in general formula III may be radioactive (e.g., F18, Br-76, Br-77, 1-123, 1-124, 1-125, 1-131) or non-radioactive; a carbon atom (e.g., in an alkyl, cycloalkyl, aryl or heteroalicyclic) in general formula III may be radioactive (e.g., C-11) or non-radioactive; a nitrogen atom (e.g., in the depicted heteroalicyclic group) in general formula III may be radioactive (e.g., N-13) or non-radioactive; and an oxygen atom (e.g., in the depicted hydroxy) in general formula III may be radioactive (e.g., O-15) or non-radioactive.

R₃₀ and/or R₃₁ may be a substituted phenyl, benzyl or benzoyl, each being substituted on the phenyl ring by halo (e.g., a radioactive halogen isotope described herein).

The carbon atom bound to the hydroxy group in Formula III may have an *(R)* chirality.

The cycloalkyl or heteroalicyclic carbon atom bound to the piperidine ring Formula III may have an *(R)* chirality. In some examples, the cycloalkyl or heteroalicyclic carbon atom bound to the hydroxy group in Formula III and the carbon atom bound to the piperidine ring in Formula III each have an *(R)* chirality.

The radio-ligand may be vesamicol, wherein X and Y are each CH and R₃₀ and R₃₁ are each hydrogen (e.g., vesamicol radiolabeled with C-11), or a derivative thereof, wherein R₃₀ and/or R₃₁ is not hydrogen.

The radio-ligand may be an azavesamicol, wherein at least one of X and Y is N. In some embodiments, one of X and Y is N. When Y is N and X is CH, the compound is referred to as a trozamicol. When X is N and Y is CH, the compound is referred to as a prezamicol. 4-fluorobenzyltrozamicol (FBT) is an example of a trozamicol radio-ligand, which may be radiolabeled, for example, with F-18.

In some embodiments, a nitrogen atom represented by X and/or Y is substituted, whereas a CH represented by X and/or Y is not substituted, that is, when Y is N and X is CH, R30 is not hydrogen and R31 is hydrogen, when Y is CH and X is N, R₃₀ is hydrogen and R₃₁ is not hydrogen.

In some examples, the radio-ligand is a benzovesamicol, wherein R₃₀ and R₃₁ together form a substituted or non-substituted phenyl ring. In some embodiments, R₃₀ and R₃₁ together form a phenyl ring substituted with halo (e.g., iodo), haloalkyl (e.g., fluoroalkyl) or haloalkoxy (e.g., fluoroalkoxy). In some embodiments, the haloalkoxy is 2-fluoroethoxy. Examples of benzovesamicols which may optionally be radiolabeled with a radioactive halogen isotope (e.g., as described herein) include, without limitation, 5-iodobenzovesamicol (5-IB VM) and 5-fluoroethoxybenzovesamicol (FEOBV).

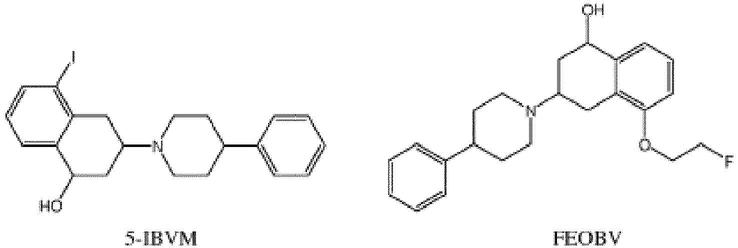

Vesamicol and vesamicol derivatives (e.g., as described herein) may be used to selectively image presynaptic cholinergic neurons, for example, in a region of cholinergic activity (e.g., as described herein), for example, in ganglia.

In some examples, the radio-ligand is an ibogamine analog as described in U.S. Patent No. 6,211,360, or a halogenated derivative thereof. Such analogs (e.g., 18-MC, 18-MAC and ME-18-MC) exhibit antagonism of nicotinic receptors, particularly ganglionic-type nicotinic receptors.

In some examples, the radio-ligand has the general formula IV: 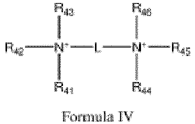 or a pharmaceutically acceptable salt thereof, wherein:
R₄₁-R₄₆ are each independently substituted or non-substituted alkyl, or alternatively, any two of R₄₁-R₄₃ and/or any two of R₄₄-R₄₆ together form a 3-, 4-, 5-, or 6-membered substituted or non-substituted heteroaryl or heteroalicyclic ring; and
L is a substituted or non-substituted hydrocarbon chain from 2-10 atoms in length,
wherein at least one atom is radioactive (e.g., radioactive carbon, radioactive nitrogen, radioactive oxygen, and/or radioactive halogen atom, as described herein).

Each atom in general formula IV may be radioactive or non-radioactive. For example, a halogen atom (if present) in general formula IV may be radioactive (e.g., F18, Br-76, Br-77, 1-123, 1-124, 1-125, 1-131) or non-radioactive; a carbon atom (e.g., in an alkyl) in general formula IV may be radioactive (e.g., C-11) or non-radioactive; a nitrogen atom (e.g., in a depicted ammonium group) in general formula IV may be radioactive (e.g., N-13) or non-radioactive; and an oxygen atom (if present) in general formula IV may be radioactive (e.g., 0-15) or non-radioactive.

As used herein, a "hydrocarbon chain" refers to a linear chain comprising a substituted or non-substituted carbon backbone. The hydrocarbon chain may contain one or two heteroatoms such as N, 0 and/or S within the chain backbone. For example, the hydrocarbon chain may be alkyl-NR'-alkyl (wherein R' is hydrogen, alkyl, cycloalkyl, aryl, heteroaryl, heteroalicyclic), alkyl-O-alkyl or alkyl-S-alkyl.

In some embodiments, the hydrocarbon chain is an alkyl group, such as ethylene, trimethylene, tetramethylene, pentamethylene, hexamethylene, heptamethylene, octamethylene, nonamethylene or decamethylene.

The hydrocarbon chain may comprise an amine group within the chain. In some embodiments, the hydrocarbon chain is alkyl-NR'-alkyl (wherein R' is as defined herein). In some embodiments, R' is alkyl (e.g., methyl). In some embodiments, the hydrocarbon chain is -CH₂CH₂N(CH₃)CH₂CH₂-.

The hydrocarbon chain may be 5 or 6 atoms in length (e.g., pentamethylene, hexamethylene, CH₂CH₂N(R')CH₂CH₂-).

In some examples, R₄₁-R₄₆ are each independently substituted or non-substituted alkyl 1-4 carbon atoms in length (e.g., methyl, ethyl, n-propyl, isopropyl, n-butyl, isobutyl, t-butyl), or alternatively, any two of R₄₁-R₄₃ and/or any two of R₄₄-R₄₆ together form a 3-, 4-, 5-, or 6-membered substituted or non-substituted heteroalicyclic ring, for example, a 3-membered aziridine ring, a 4-membered azetidine ring, a 5-membered pyrrolidine ring or a 6-membered piperidine ring. In some embodiments, the alkyl is methyl or ethyl. In some embodiments, the alkyl is methyl. In some embodiments, the heteroalicyclic ring is a substituted or non-substituted pyrrolidine ring.

Compounds having general formula IV are doubly charged and therefore unlikely to pass the blood-brain barrier to a considerably degree. This property may be useful in reducing adverse side effects associated with inhibition of cholinergic activity in the brain.

Compounds having general formula IV which may be used as radio-ligands include antagonists of nicotinic receptors, particularly ganglionic-type nicotinic receptors, such as hexamethonium, pentolinium, pentamine and chlorisondamine.

In some examples, the radio-ligand has the general formula V:

G-J-K Formula V

or a pharmaceutically acceptable salt thereof, wherein:
G is a substituted or non-substituted phenyl or pyridinyl moiety;
J is absent or is selected from the group consisting of 0, S, -O-alkyl-, -S-alkyl, alkyl-O- and alkyl-S-; and
K is a substituted or non-substituted heteroalicyclic ring comprising at least one nitrogen atom,
wherein at least one atom is radioactive (e.g., radioactive carbon, radioactive nitrogen, radioactive oxygen, and/or radioactive halogen atom, as described herein).

Each atom in general formula V may be radioactive or non-radioactive. For example, a halogen atom (if present) in general formula V may be radioactive (e.g., F18, Br-76, Br-77, 1-123, 1-124, 1-125, 1-131) or non-radioactive; a carbon atom (e.g., in an alkyl, aryl or heteroaryl) in general formula V may be radioactive (e.g., C-11) or nonradioactive; a nitrogen atom (e.g., in a pyridinyl or heteroalicyclic) in general formula V may be radioactive (e.g., N-13) or non-radioactive; and an oxygen atom (e.g., in J) in general formula V may be radioactive (e.g., 0-15) or non-radioactive..

Formula V encompasses nicotine (wherein G is pyridin-3-yl, J is absent and K is N-methyl-pyrrolidin-2-yl) and analogs thereof, which typically exhibit agonist activity towards nicotinic receptors.

The phenyl or pyridinyl moiety may be substituted (e.g., at a *meta* position with respect to J) by halo (e.g., chloro, iodo). The halo may be a radioactive isotope (e.g., a radioactive iodine isotope described herein).

In some examples, Gis a substituted or non-substituted phenyl or pyridin-3-yl. In some embodiments, the pyridin-3-yl is a 6-halo-pyridin-3-yl, that is, substituted by halo at the *meta* position with respect to J (e.g., as described herein).

In some examples, J comprises an alkyl having one or two carbon atoms. In some embodiments, J comprises an alkyl having one or two carbon atoms (e.g., CH₂, CH₂CH₂). In some embodiments, J is -O-CH₂. In some embodiments, J is -S-CH₂CH₂.

In some examples, K is a substituted or non-substituted 4-membered ring, 5-membered ring or 6-membered ring.

In some examples, K has one nitrogen atom (e.g., substituted or non-substituted azetidine, pyrrolidine or piperidine) or two nitrogen atoms (e.g., substituted or non-substituted imidazolidine or piperazine).

When K is a substituted ring, the substituent may be attached to one or more nitrogen atom and/or to one or more carbon atom of the heteroalicyclic ring. Examples include, without limitation, an N-methyl substituent, N,N-dimethyl substituents, C-methyl substituents, and a methylene or ethylene bridge. For example, in some embodiments, K is a 7-aza-bicyclo[2.2.1]heptane, which is a pyrrolidine ring substituted by an ethylene bridge.

In some examples, K is selected form the group consisting of substituted or non-substituted pyrrolidin-2-yl (e.g., N-methyl-pyyrolidin-2-yl), pyrrolidin-3-yl (e.g., 7-aza-bicyclo[2.2.1]hept-2-y1), azetidin-2-yl (e.g., non-substituted azetidin-2-yl) and piperazinyl (e.g., 4,4-dimethylpiperazin-1-yl).

Examples of compounds having general formula V which may be used as radio-ligands include, without limitation, nicotine, epibatidine, DMPP, tebanicline and 5-I-A-85380.

Incorporation of a radioactive isotope in a radioactive tracer as described herein may be performed by various techniques known in the art.

A radioactive halogen (e.g., fluorine, bromine, iodine) isotope may be attached to an aromatic ring by electrophilic and/or nucleophilic aromatic substitution, for example, as described by Vallabhajosula & Nikolopoulou [Semin Nucl Med 2011, 41:324-33].

Compounds comprising an N-alkyl group (e.g., N-methyl, N-ethyl), for example, DMPP, SIB-1553A, 18-methylaminocoronaridine and compounds according to Formula IV (e.g., hexamethonium, pentolinium, pentamine and chlorisondamine), radiolabeled by C-11 in the N-alkyl group by nucleophilic reaction of an amine group with a C-11 labeled small molecule (e.g., methyl iodide, ethyl iodide).

It is to be appreciated that many neurotransmitters and analogs thereof will not effectively cross the blood-brain barrier, and are therefore particularly useful for imaging of the peripheral nervous system, as potentially adverse effects on the brain are reduced or avoided.

Examples of radio-ligands suitable for use in brain imaging, e.g., capable of passing the blood-brain barrier, include, without limitation, 5-IBVM, iodobenzamide, epidepride, iomazenil, 5-I-A-85380, ADAM, altropane, PE2I, ioflupane, f3-CIT (RTI-55), RTI-121, RTI-229, RTI-352, TRODAT-1 and TROTEC-1.

Additional ligands which may be radiolabeled and their uses in imaging, as well as techniques for preparing radio-ligands, are described in Langer & Haldin [Eur J Nucl Med 2002, 29:416-434], Vallabhajosula & Nikolopoulou [Semin Nucl Med 2011, 41:324-33], Ross & Seibyl [J Nucl Med Tech 2004, 32:209-214], U.S. Patent Application Publication No. 2010/0221182 and U.S. Patent Nos. 6,358,492, 5,077,035 and 5,789,420.

It is expected that during the life of a patent maturing from this application many relevant radioactive isotopes and/or radioactive tracers such as radio-ligands will be developed and the scope of the terms "radioactive isotope", "radioactive tracer" and "radio-ligand" are intended to include all such new technologies *a priori.*

In particular, it is expected that during the life of a patent maturing from this application many relevant families of radio-ligands that bind to ANS cells will be developed and the scope of the term "radio-ligand" is intended to include all such new technologies α *priori.*

Herein, the term "alkyl" describes a saturated or unsaturated aliphatic hydrocarbon including straight chain and branched chain groups. Preferably, the alkyl group has 1 to 20 carbon atoms. Whenever a numerical range; e.g., "1 to 20", is stated herein, it implies that the group, in this case the alkyl group, may contain 1 carbon atom, 2 carbon atoms, 3 carbon atoms, etc., up to and including 20 carbon atoms. More preferably, the alkyl is a medium size alkyl having 1 to 10 carbon atoms. Most preferably, unless otherwise indicated, the alkyl is a lower alkyl having 1 to 4 carbon atoms. The alkyl group may be substituted or non-substituted. Substituted alkyl may have one or more substituents, whereby each substituent group can independently be, for example, cycloalkyl, alkenyl, alkynyl, aryl, heteroaryl, heteroalicyclic, amine, halide, sulfonate, sulfoxide, phosphonate, hydroxy, alkoxy, aryloxy, thiohydroxy, thioalkoxy, thioaryloxy, cyano, nitro, azo, sulfonamide, carboxy, thiocarbamate, urea, thiourea, carbamate, amide, and hydrazine.

When unsaturated, an alkyl may comprise at least one carbon-carbon double bond, in which case it may also be referred to as an "alkenyl", and/or at least one carbon-carbon triple bond, in which case it may also be referred to as an "alkynyl".

The alkyl group can be an end group, as this phrase is defined herein, wherein it is attached to a single adjacent atom, or a linking group, as this phrase is defined herein, which connects two or more moieties.

Herein, the phrase "end group" describes a group (e.g., a substituent) that is attached to a single moiety in the compound via one atom thereof.

The phrase "linking group" describes a group (e.g., a substituent) that is attached to two or more moieties in the compound.

The term "cycloalkyl" describes an all-carbon monocyclic or fused ring (i.e., rings which share an adjacent pair of carbon atoms) group where one or more of the rings does not have a completely conjugated pi-electron system. The cycloalkyl group may be substituted or non-substituted. Substituted cycloalkyl may have one or more substituents, whereby each substituent group can independently be, for example, alkyl, cycloalkyl, aryl, heteroaryl, heteroalicyclic, amine, halide, sulfonate, sulfoxide, phosphonate, hydroxy, alkoxy, aryloxy, thiohydroxy, thioalkoxy, thioaryloxy, cyano, nitro, azo, sulfonamide, carboxy, thiocarbamate, urea, thiourea, carbamate, amide, and hydrazine. The cycloalkyl group can be an end group, as this phrase is defined herein, wherein it is attached to a single adjacent atom, or a linking group, as this phrase is defined herein, connecting two or more moieties.

The term "aryl" describes an all-carbon monocyclic or fused-ring polycyclic (i.e., rings which share adjacent pairs of carbon atoms) groups having a completely conjugated pi-electron system. The aryl group may be substituted or non-substituted. Substituted aryl may have one or more substituents, whereby each substituent group can independently be, for example, alkyl, cycloalkyl, aryl, heteroaryl, heteroalicyclic, amine, halide, sulfonate, sulfoxide, phosphonate, hydroxy, alkoxy, aryloxy, thiohydroxy, thioalkoxy, thioaryloxy, cyano, nitro, azo, sulfonamide, carboxy, thiocarbamate, urea, thiourea, carbamate, amide, and hydrazine. The aryl group can be an end group, as this term is defined herein, wherein it is attached to a single adjacent atom, or a linking group, as this term is defined herein, connecting two or more moieties.

The term "heteroaryl" describes a monocyclic or fused ring (i.e., rings which share an adjacent pair of atoms) group having in the ring(s) one or more atoms, such as, for example, nitrogen, oxygen and sulfur and, in addition, having a completely conjugated pi-electron system. Examples, without limitation, of heteroaryl groups include pyrrole, furan, thiophene, imidazole, oxazole, thiazole, pyrazole, pyridine, pyrimidine, quinoline, isoquinoline and purine. The heteroaryl group may be substituted or non-substituted. Substituted heteroaryl may have one or more substituents, whereby each substituent group can independently be, for example, alkyl, cycloalkyl, aryl, heteroaryl, heteroalicyclic, amine, halide, sulfonate, sulfoxide, phosphonate, hydroxy, alkoxy, aryloxy, thiohydroxy, thioalkoxy, thioaryloxy, cyano, nitro, azo, sulfonamide, carboxy, thiocarbamate, urea, thiourea, carbamate, amide, and hydrazine. The heteroaryl group can be an end group, as this phrase is defined herein, where it is attached to a single adjacent atom, or a linking group, as this phrase is defined herein, connecting two or more moieties. Representative examples are pyridine, pyrrole, oxazole, indole, purine and the like.

The term "heteroalicyclic" describes a monocyclic or fused ring group having in the ring(s) one or more atoms such as nitrogen, oxygen and sulfur. The rings may also have one or more double bonds. However, the rings do not have a completely conjugated pi-electron system. The heteroalicyclic may be substituted or non-substituted. Substituted heteroalicyclic may have one or more substituents, whereby each substituent group can independently be, for example, alkyl, cycloalkyl, aryl, heteroaryl, heteroalicyclic, amine, halide, sulfonate, sulfoxide, phosphonate, hydroxy, alkoxy, aryloxy, thiohydroxy, thioalkoxy, thioaryloxy, cyano, nitro, azo, sulfonamide, carboxy, thiocarbamate, urea, thiourea, carbamate, amide, and hydrazine. The heteroalicyclic group can be an end group, as this phrase is defined herein, where it is attached to a single adjacent atom, or a linking group, as this phrase is defined herein, connecting two or more moieties. Representative examples are piperidine, piperazine, tetrahydrofuran, tetrahydropyran, morpholine and the like.

As used herein, the terms "amine" and "amino" describe both a -NRxRy group - NRx- group, wherein Rx and Ry are each independently hydrogen, alkyl, cycloalkyl, aryl, heteroaryl or heteroalicyclic, as these terms are defined herein. When Rx or Ry is heteroaryl or heteroalicyclic, the amine nitrogen atom is bound to a carbon atom of the heteroaryl or heteroalicyclic ring.

The amine group can therefore be a primary amine, where both Rx and Ry are hydrogen, a secondary amine, where Rx is hydrogen and Ry is alkyl, cycloalkyl, aryl, heteroaryl or heteroalicyclic, or a tertiary amine, where each of Rx and Ry is independently alkyl, cycloalkyl, aryl, heteroaryl or heteroalicyclic.

The terms "halide" and "halo" refer to fluorine, chlorine, bromine or iodine.

The term "haloalkyl" describes an alkyl group as defined herein, further substituted by one or more halide(s).

The term "phosphonate" refers to an -O-P(=O)(ORx)- group, with Rx as defined herein.

The term "sulfoxide" or "sulfinyl" describes a -S(=O) Rx group, where Rx is as defined herein.

The terms "sulfonate" and "sulfonyl" describe a -S(=O)₂-Rx group, where Rx is as defined herein.

The term "sulfonamide", as used herein, encompasses both S-sulfonamides and N-sulfonamides.

The term "S-sulfonamide" describes a -S(=O)₂-NRxR_{Y} group, with Rx and Ry as defined herein.

The term "N-sulfonamide" describes an RxS(=O)₂-NR_{Y}- group, where Rx and Ry are as defined herein.

The terms "carbonyl" and "acyl" as used herein, describe a -C(=O)-Rx group, with Rx as defined herein.

The terms "hydroxy" and "hydroxyl" describe a -OH group.

The term "alkoxy" describes both an -O-alkyl and an -O-cycloalkyl group, as defined herein.

The term "aryloxy" describes both an -O-aryl and an -O-heteroaryl group, as defined herein.

The term "thiohydroxy" describes a -SH group.

The term "thioalkoxy" describes both a -S-alkyl group, and a -S-cycloalkyl group, as defined herein.

The term "thioaryloxy" describes both a -S-aryl and a -S-heteroaryl group, as defined herein.

The terms "cyano" and "nitrile" describe a -C=N group.

The term "nitro" describes an -NO₂ group.

The term "azo" describes an -N=N-Rx group, with Rx as defined herein.

The terms "carboxy" and "carboxyl", as used herein, encompasses both C-carboxy and O-carboxy groups.

The term "C-carboxy" describes a -C(=O)-ORx group, where Rx is as defined herein.

The term "O-carboxy" describes a -OC(=O)-Rx group, where Rx is as defined herein.

The term "urea" describes a -NRxC(=O)-NRyRw group, where Rx and Ry are as defined herein and Rw is as defined herein for Rx and Ry.

The term "thiourea" describes a -NRx-C(=S)-NRyRw group, with Rx, Ry and Rw as defined herein.

The term "amide", as used herein, encompasses both C-amides and N-amides.

The term "C-amide" describes a -C(=O)-NRxRy group, where Rx and Ry are as defined herein.

The term "N-amide" describes a RxC(=O)-NRy- group, where Rx and Ry are as defined herein.

The term "carbamyl" or "carbamate", as used herein, encompasses both N-carbamates and O-carbamates.

The term "N-carbamate" describes an RyOC(=O)-NRx- group, with Rx and Ry as defined herein.

The term "O-carbamate" describes an -OC(=O)-NRxRy group, with Rx and Ry as defined herein.

The term "thiocarbamyl" or "thiocarbamate", as used herein, encompasses both O-thiocarbamates and N-thiocarbamates.

The term "O-thiocarbamate" describes a -OC(=S)-NRxRy group, with Rx and Ry as defined herein.

The term "N-thiocarbamate" describes an RyOC(=S)NRx- group, with Rx and Ry as defined herein.

The term "guanidine" describes a -RxNC(=N)-NRyRw group, where Rx, Ry and Rw are as defined herein.

The term "hydrazine", as used herein, describes a -NRx-NRyRw group, with Rx, Ry, and Rw as defined herein.

### Data acquisition:

Any of the radioactive tracers described herein may be utilized, via any suitable nuclear imaging technique, to acquire data which contains information regarding a distribution of the radioactive tracer in the body (also referred to herein as "radioactive tracer data"), for use in imaging as described herein. In any one of the aspects and embodiments described herein, suitable nuclear imaging techniques include, for example, SPECT and PET imaging techniques. In exemplary embodiments, the nuclear imaging technique is SPECT and the radioactive tracer is radioactive mIBG (e.g., I-123-mIBG).

It is expected that during the life of a patent maturing from this application many relevant nuclear imaging techniques and systems will be developed and the scopes of the terms "nuclear imaging", "radioactive tracer data", "SPECT", "PET" and "modality" are intended to include all such new technologies *α priori.*

A SPECT technique may involve any SPECT modality known in the art, including, without limitation, a conventional standard SPECT imaging modality (e.g., a dual detector Anger camera modality, also referred to as "A-SPECT"), a electrocardiogram-gated SPECT (GSPECT) modality, a respiratory-gated SPECT modality, a SPECT-CT modality (co-registration of SPECT and X-ray CT images), a SPECT-MRI modality (co-registration of SPECT and magnetic nuclear resonance images) and/or a high-speed SPECT modality such as a multiple scanning detector SPECT modality (e.g., a D-SPECT™ system, such as available from Spectrum Dynamics), a multiple-pinhole detector SPECT modality (e.g., a Discovery™ NM 530c system), a multiple curved crystal SPECT modality (e.g., a CardiArc® system) and/or a multiple confocal collimator SPECT modality (e.g., an IQ-SPECT system).

According to some embodiments of the invention, the SPECT modality is selected from the group consisting of an A-SPECT modality, an electrocardiogram-gated SPECT (GSPECT) modality, a SPECT-CT modality and a multiple scanning detector SPECT modality (which for brevity is also referred to herein as a "D-SPECT" modality).

"GSPECT" and "electrocardiogram-gated SPECT" refer to a well-known SPECT modality, as described for example, by Paul et al. [J Nucl Med Technol 2004, 32:1798-187].

High speed SPECT modalities are described, for example, by Garcia et al. [J Nucl Med 2011, 52:210-217].

In some embodiments, the SPECT modality includes a set of collimators, for example scale division (SD) collimators. Optionally, between 10 and 20 collimators are used, for example 14. Optionally, the scan pattern includes about 360 positions around 360 degrees.

In some embodiments, the used reconstruction algorithm is ordered-subsets expectation maximization (OSEM) and/or depth-dependent resolution recovery (RR).

In some embodiments, the pixel size is between about 2.5 millimeter³ and about 4.9 mm³.

In some embodiments in which SPECT is used, the subject is injected with an I-123 radiolabeled tracer, for example, I-123 radiolabeled mIBG, optionally in a dose of from about 2 mCi to 12 mCi, and optionally from 3 mCi to 8 mCi, for example, about 5mCi.

A PET technique may involve any PET modality known in the art, including, without limitation, a conventional PET imaging modality, an electrocardiogram-gated PET modality, a respiratory-gated PET modality, a PET-CT modality (co-registration of PET and x-ray CT images) and/or a PET-MRI modality (co-registration of PET and magnetic nuclear resonance images).

Gated PET modalities are described, for example, by Buther et al. [J Nucl Med 2009, 50:674-681].

It is to be appreciated that co-registration of PET or SPECT data with CT and/or magnetic nuclear resonance imaging (MRI) data may be useful for obtaining anatomical data (e.g., CT and/or MRI anatomical data) used for analysis according to some embodiments, as described herein.

In some embodiments, data (e.g., SPECT or PET data) is produced per segment, for example, segments of a few centimeters in dimension.

In some embodiments, data (e.g., SPECT or PET data) is reconstructed at a quality spatial resolution of about 1 cm x 1 cm x 1 cm or better (i.e., higher resolution). In some embodiments, the resolution is about 7 mm x 7 mm x 7 mm or better. In some embodiments, the resolution is about 5 mm x 5 mm x 5 mm or better. In some embodiments, the resolution is about 3 mm x 3 mm x 3 mm or better.

In some embodiments, data (e.g., SPECT or PET data) is reconstructed at a spatial resolution of at least 1 voxel per cubic centimeter. In some embodiments, the spatial resolution is at least 3 voxels per cubic centimeter. In some embodiments, the spatial resolution is at least 10 voxels per cubic centimeter. In some embodiments, the spatial resolution is at least 30 voxels per cubic centimeter.

Optionally, the data is reconstructed in a non-cubical voxel structures. Optionally, the SPECT data is reconstructed in voxels which are aligned with a model of the imaged object, such as an organ wall (e.g., hearts muscle wall) geometry.

In some embodiments, radioactive tracer data acquisition includes photon acquisition over a period of time of up to about 20 minutes (e.g., a period of time described herein for acquiring radioactive tracer data). In some embodiments, photon acquisition is for up to about 15 minutes. In some embodiments, photon acquisition is for up to about 10 minutes. In some embodiments, photon acquisition is for up to about 5 minutes. In some embodiments, photon acquisition is for up to about 3 minutes. In some embodiments, photon acquisition is for up to about 2 minutes. In some embodiments, photon acquisition is for about 2 to 8 minutes. In some embodiments, photon acquisition is for about 8 minutes. In some embodiments, photon acquisition is for about 10 minutes.

In some embodiments, data is acquired for multiple time points, for example, wherein a set of data is acquired for each time point. Data sets from multiple time points may optionally be used to characterize synaptic center radioactive tracer distribution and/or neuronal activity at different time points, for example, before and after a treatment. Additionally or alternatively, data sets from multiple time points may optionally be used to a rate of change (e.g., during a time period encompassing a plurality of time points) in concentration of the radioactive tracer in a body region. In some embodiments, the detected emission for one or more time points after an administration of radioactive tracer is adjusted in order to account for the exponential decrease in radioactive emission due to radioactive isotope decay (which does not reflect a change in the spatial distribution of the radioactive tracer).

As used herein, the phrase "time point" encompasses time periods having a non-infinitesimal duration, for example, depending on the temporal resolution of the technique for determining a spatial distribution of the radioactive tracer.

In some embodiments, a first data acquisition step (e.g., of about 10 minutes) is followed by a wait period which is further followed by a second data acquisition step (e.g., of about 10 minutes). In some embodiments, the wait period between data acquisition steps is of about 5, about 10, about 20, about 30, about 45, about 60, about 90, and about 120 minutes or any intermediate or longer periods. In some embodiments, the wait period is from about 5 to about 30 minutes. In some embodiments, the wait period is from about 20 to about 60 minutes. In some embodiments, the wait period is from about 30 to about 120 minutes. In some embodiments, the wait period is from about 1 to about 5 hours. In some embodiments, the wait period is from about 2 to about 48 hours.

In some embodiments, data is used in a format wherein a signal for a region in the body is correlated to a concentration of radioactive tracer in the region (e.g., the signal simply represents the amount of detected radioactivity emitted from the region in the body).

In some embodiments, the data includes a time-dependency of the radioactive tracer distribution (optionally adjusted so as not to show changes due to radioactive isotope decay).

In some embodiments, the time-dependency of the radioactive tracer distribution comprises a rate of change in the concentration of radioactive tracer in a region in the body.

In some embodiments, the time-dependency of the radioactive tracer distribution comprises a response to a stimulus, for example, comprising data representing radioactive tracer distribution prior to the stimulus as well as data representing radioactive tracer distribution during and/or subsequent to the stimulus.

As used herein, the term "stimulus" encompasses any act intended to affect the body of a subject in a manner which may alter a radioactive tracer distribution (e.g., in a manner which facilitates characterizing a synaptic center). In some embodiments, the stimulus comprises stimulating a neuronal activity. Examples of stimuli include, without limitation, an electrical stimulus (e.g., application of electrical voltage and/or current), a chemical stimulus (e.g., administration of a biologically active agent), a mechanical stimulus (e.g., physical contact), a thermal stimulus (e.g., change in temperature), and exercise.

In some embodiments, more than one stimulus is performed. In some embodiments, different types of stimuli are performed.

In some embodiments, data is used in a format wherein a signal for a region in the body is correlated to a change in concentration of radioactive tracer in the region, the change being between two or more time points for which data is acquired (optionally adjusted so as not to show changes due to radioactive isotope decay). In some embodiments, the signal represents a rate of change in concentration of radioactive tracer in the region in the body.

In some embodiments, the signal represents the difference in detected radioactivity emitted from the region in the body at two different time points. In some embodiments, the difference represents a rate of change (e.g., by dividing the difference by the time interval between the two time points). In some embodiments, the difference represents a response to a stimulus.

In some embodiments, the signal represents a change in detected radioactivity emitted from the region in the body over the course of three or more time points, for example, a signal which represents a rate of change.

In some embodiments, a signal representing a rate of change is time dependent, that is, different rates of change are determined for different time points, e.g., by fitting a curve to data points generated for three or more time points, and/or by determining a rate of change between pairs of adjacent time points for a plurality of different pairs. In some embodiments, a rate of change is determined prior to a stimulus as well as during and/or subsequent to the stimulus.

Examples of common changes in a concentration of radioactive tracer include an uptake rate and a washout rate. "Uptake" refers to accumulation of a tracer at a location, and is typically prominent shortly after injection of a tracer. "Washout" refers to a gradual clearance of a tracer from a location, and is typically prominent at a later stage after a rate of uptake has decreased considerably (e.g., when little tracer remains in circulation). Typically, specific binding of a tracer to a target (e.g., a synaptic center) is characterized by a higher uptake rate and lower washout rate than non-specific binding.

In some embodiments, an uptake rate and/or washout rate is determined in order to facilitate identification of a synaptic center specifically bound by the radioactive tracer.

In some embodiments, an uptake rate and/or washout rate is characterized as a sum of two or more rates. For example, the uptake and/or washout may optionally fit a biexponential kinetic model.

In some embodiments, a change in concentration of radioactive tracer (e.g., as observed by acquisition of data at multiple time points) is optionally processed (e.g., by searching for a best fit to a kinetic model) so as to calculate one or more uptake rates and/or one or more washout rates.

In some embodiments, data acquisition is performed after a delay between injection of the radioactive tracer, wherein the length of the delay is selected to allow for considerable washout of radioactive tracer which is bound non-specifically, but without excessive washout of radioactive tracer specifically bound at a synaptic center. The skilled person will be able to determine a suitable delay for any given radioactive tracer based on the washout rate for specifically and non-specifically bound tracer, for example, by experimentally determining washout rates based on a previous imaging session. Additionally or alternatively, data acquisition is performed without such a delay, such that data showing an uptake process may optionally be acquired.

In some embodiments, a stimulus is selected so as to specifically affect a distribution of radioactive tracer in a synaptic center. Detection of a response to such a stimulus may optionally be used to distinguish a synaptic center from a region which is not a synaptic center and/or to distinguish between synaptic centers (e.g., as described herein). Alternatively or additionally, the response itself may optionally comprise clinically useful information (e.g., as described herein).

In some embodiments, a stimulus comprises enhancement or inhibition of an activity in at least one synaptic center. In some embodiments, one or more neurons (e.g., a nerve fiber) leading to a synaptic center is stimulated, for example, by electrical, chemical and/or mechanical stimulation.

In some embodiments, enhancement of activity is effected by chemical stimulation, using a biologically active agent which stimulates neurotransmitter release (e.g., norepinephrine release). The modulation may be effected, for example, by local and/or systemic administration of the biologically active agent to the subject. Tyramine is an example of a biologically active agent which stimulates adrenergic neurotransmitter (e.g., norepinephrine) release.

In some embodiments, activity in a synaptic center affects rate of change in the concentration of a radioactive tracer. Such a phenomenon may optionally be used to interpret an effect of a stimulus on activity (e.g., as described herein) and/or to determine a level of activity in the absence of a stimulus (e.g., as described herein).

In some embodiments, enhanced activity in a synaptic center results in a relative increase (e.g., optionally a reduction in a rate of decrease) in radioactive tracer concentration, for example, by an increase in uptake rate.

In some embodiments, inhibited activity in a synaptic center results in a relative decrease (e.g., optionally a reduction in a rate of increase) in radioactive tracer concentration, for example, by a decrease in uptake rate.

In some embodiments, activity of a synaptic center is positively correlated with an uptake rate for a radioactive tracer.

In some embodiments, enhanced activity in a synaptic center results in a relative decrease (e.g., optionally a reduction in a rate of increase) in radioactive tracer concentration, for example, by an increase in washout rate.

In some embodiments, inhibited activity in a synaptic center results in a relative increase (e.g., optionally a reduction in a rate of decrease) in radioactive tracer concentration, for example, by a decrease in washout rate.

In some embodiments, activity of a synaptic center is positively correlated with a washout rate for a radioactive tracer. In some embodiments, activity of a synaptic center is also positively correlated with an uptake rate for the radioactive tracer.

In some embodiment, a correlation of a synaptic center activity (e.g., a positive or negative correlation according to any one of the embodiments described herein) with an amount of radioactive tracer and/or a rate of change thereof is used to determine a level activity of a synaptic center, based on data describing an amount of radioactive tracer and/or a rate of change thereof.

In some embodiments, a radioactive tracer which undergoes uptake into a presynaptic neuron (e.g., a neurotransmitter or analog thereof, as described herein) escapes from the neuron at a rate which depends on a rate of neurotransmitter release, for example, wherein the tracer is released with the neurotransmitter; and the correlation is optionally used to determine a level of synaptic activity (e.g., as indicated by a rate of neurotransmitter release). In some such embodiments, activation of the synaptic center increases the washout rate by facilitating escape of the radioactive tracer from neurons. In some embodiments, the radioactive tracer has the general formula II described herein. In some embodiments, the radioactive tracer is radioactive mIBG (e.g., I-123-mIBG).

In some embodiments, a radioactive tracer which is a ligand of a neurotransmitter receptor (e.g., a receptor agonist or antagonist, as described herein) is released from the receptor at a rate which depends on an amount of neurotransmitter available for binding the receptor, for example, wherein the tracer and neurotransmitter compete for the same receptor binding site; and the correlation is optionally used to determine a level of synaptic activity (e.g., as indicated by an amount of neurotransmitter available for binding the receptor). In some such embodiments, activation of the synaptic center increases the washout rate by increasing an amount of neurotransmitter in the synapse and thereby reducing an amount of available binding sites for the tracer.

In some embodiments, a stimulus comprises chemical modulation of radioactive tracer uptake. The modulation may be effected, for example, by local and/or systemic administration of a biologically active agent to the subject.

In some embodiments, the biologically active agent is an inhibitor of a protein (e.g., a transporter) which facilitates uptake of the tracer. In some embodiments, the biologically active agent is an inhibitor of a neurotransmitter transporter which transports the radioactive tracer (e.g., a neurotransmitter or analog thereof, as described herein). In some embodiments, the biologically active agent is an inhibitor of a norepinephrine transporter (NET), also known in the art as a norepinephrine reuptake inhibitor (NRI). Examples of NRIs include, without limitation, selective NRIs such as amedalin, atomoxetine, CP-39,332, daledalin, edivoxetine, esreboxetine, lortalamine, maprotiline, mazindol, nisoxetine, nomifensine, reboxetine, talopram, talsupram, tandamine and viloxazine, as well as non-selective NRIs such as bupropion, ciclazindol, desipramine, manifaxine, radafaxine, tapentadol and teniloxazine.

Inhibitors of neurotransmitter transporters are a common target for pharmaceutical development. Thus, it is expected that during the life of a patent maturing from this application many relevant neurotransmitter transporter inhibitors will be developed and the scope of the term "inhibitor of a neurotransmitter transporter" is intended to include all such new technologies *α priori.*

In some embodiments, a change in activity induced by a stimulus is not necessarily identified as an increase or decrease in activity, but merely as a change in behavior. For example, in some embodiments, it may not be known (e.g., for a given synaptic center and/or radioactive tracer) whether synaptic center activity is positively or negatively correlated with a concentration of the radioactive tracer in the synaptic center.

In some embodiments, the stimulus is characterized as affecting a particular subsystem of the nervous system.

In some embodiments, a stimulus is selected to primarily affect the sympathetic nervous system (e.g., as opposed to the parasympathetic nervous system).

In some embodiments, a stimulus is selected to primarily affect the parasympathetic nervous system (e.g., as opposed to the sympathetic nervous system).

In some embodiments, a stimulus is selected to primarily affect the autonomic nervous system (e.g., as opposed to the somatic nervous system).

Many stimuli of the autonomic nervous system, including stimuli specific for the sympathetic or parasympathetic branches thereof, are known in the art.

In some embodiments, the stimulus is selected to primarily affect the somatic nervous system (e.g., as opposed to the autonomic nervous system). Conscious actions, for example, typically affect an identifiable region of the somatic nervous system, as opposed, for example, to the autonomic nervous system.

In some embodiments, a concentration of radioactive tracer (e.g., as indicated by detected emission per volume within a given time period) is positively correlated to a level of synaptic activity (e.g., a number of nerve pulses transmitted by synapses). In some embodiments such a correlation is used to determine a level of synaptic activity. In some embodiments, such a correlation is used to normalize a signal when determining an amount of synapses (e.g., a synaptic density in a given region).

In some embodiments, a concentration of radioactive tracer is correlated to a concentration of radioactive tracer in the blood. In some embodiments such a correlation is used to normalize a signal when determining an amount of synapses (e.g., a number of nerve pulses transmitted by synapses) and/or activity of synapses (e.g., a synaptic density in a given region). In some embodiments, the method further comprises determining a level of radioactive tracer in the blood (e.g., via a blood test).

In some embodiments, a concentration of radioactive tracer is inversely correlated to a concentration of neurotransmitter in the blood (e.g., due to competition between tracer and neurotransmitter at uptake and/or receptor sites), for example, norepinephrine (e.g., in the case of a radioactive tracer selective for adrenergic synapses) or acetylcholine (e.g., in the case of a radioactive tracer selective for cholinergic synapses). In some embodiments such a correlation is used to normalize a signal when determining an amount of synapses (e.g., a number of nerve pulses transmitted by synapses) and/or activity of synapses (e.g., a synaptic density in a given region). In some embodiments, the method further comprises determining a level of at least one neurotransmitter in the blood (e.g., via a blood test).

### Data analysis and identification of synaptic centers:

In some embodiments, data acquired and optionally processed according to any one of the embodiments described herein is analyzed in order to identify a synaptic center. Such data includes, but is not necessarily limited to radioactive tracer data described herein (e.g., obtained using a nuclear imaging technique described herein). For example, the data may optionally further include anatomical data acquired according to any one of the embodiments described herein.

A presentation of information generated by identifying at least one synaptic center (as described herein) is referred to herein as a "model" comprising at least one synaptic center. The information may be used for generating and/or updating and/or modifying the model.

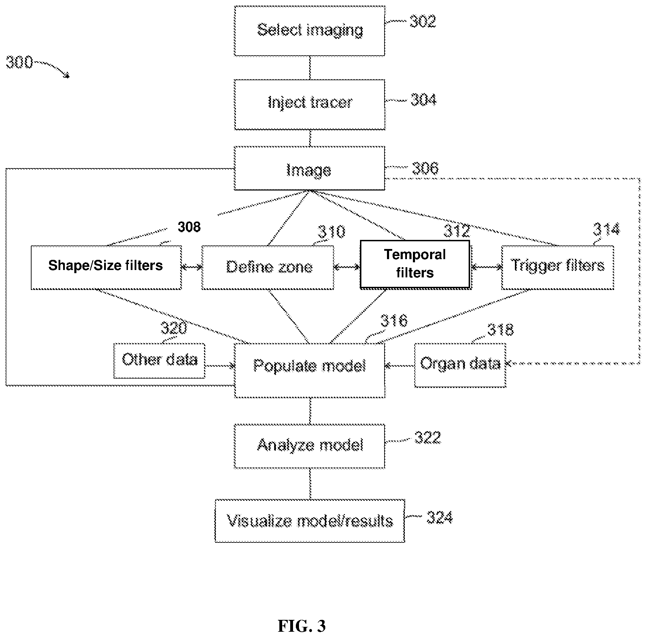

FIG. 3 is a flowchart of a method 300 of obtaining information by identifying at least one synaptic center, in accordance with some embodiments of the invention.

Steps 302-306 describe acts of data acquisition according to some embodiments described herein.

At 302, an operator (e.g., a physician), may select an imaging and/or modeling type (e.g., a SPECT or PET modality described herein). Optionally, this is based on a desired diagnosis. At 304, any one of the radioactive tracers described herein, or any combination thereof, is injected into the patient. At 306, emitted radiation may be captured so as to acquire data suitable for imaging (e.g., as described herein) and optionally reconstructed into an image. In some embodiments, no actual image is reconstructed. Optionally, the emission radiation is used to determine parameters for a model or to generate the model.

Optionally, GPs are identified based on one or more predefined thresholds and/or rules. Steps 308-314 describe optional techniques for identifying a presence of at least one synaptic center, which may be used alone or in combination, according to some embodiments described herein.

At 308, a size and/or shape filter is optionally used to identify blobs (i.e., regions characterized by a relatively high emission of radiation) having a size and/or shape of synaptic centers which are being imaged, for example, ganglia. In some embodiments of the invention, the shape of a ganglion is assumed to be a shape selected from the group consisting of spherical, ovoid and amygdaloid (almond-shaped) and/or having a diameter of between 2 and 10 mm. It should be noted that the expected size and/or shape and/or activity level may depend on the location in which a synaptic center is being searched for. In some embodiments, a synaptic center may be identified according to emission levels, e.g., a blob exhibiting an emission level in a certain range may be identified as a synaptic center.

In some embodiments, a synaptic center (e.g., GP) is identified as an object with a size of at least about 4X4X4 mm (e.g., for an epicardial ganglionated plexus).

In some embodiments, a synaptic center (e.g., GP) is identified as an object with a size of at least about 2X2X2 mm (e.g., for a myocardial ganglionated plexus).

Alternatively or additionally, the synaptic center may optionally be identified by comparing a signal (e.g., emission intensity) for a certain region to a signal of surrounding regions. For example, a synaptic center may optionally be identified in a region satisfying the rule that the total signal (e.g., emission intensity) of a region is a predefined factor times the standard deviation above average signal and/or the signal of adjacent regions is lower than a predefined fraction (e.g., half) of the signal in a region associated with a synaptic center (e.g., correlated for volume). Optionally, the user may select and/or modify the predefined factor and/or predefined fraction. For example, a different type of synaptic center may be identified using another predefined factor (times the standard deviation) and/or another predefined fraction (of an adjacent signal).

At 310, a zone in which to look for synaptic centers is optionally defined. Optionally, multiple zones are defined, for example, with different components being searched for in different zones. Optionally, within the zone, other methods for finding synaptic centers (e.g., as described herein) are applied. In some embodiments, a zone is defined according to anatomical data (e.g., an anatomical image) acquired according to any one of the embodiments described herein with regard to acquisition of anatomical data.

In some embodiments, the zone is defined based on an organ associated with a nervous tissue being imaged. In some embodiments, a synaptic center is assumed to lie a certain distance from the outer wall of an organ, for example, between 0.1 and 20 mm, and/or assumed to be partly or completely embedded in an organ wall and/or a specific part thereof. In some embodiments, a synaptic center (e.g., an aggregate of nerve endings at a surface of an innervated organ) is selectively imaged by defining a region of, for example, 3 mm inwards and outwards of the expected organ surface, for identifying a synaptic center. In some embodiments, anatomical landmarks on the organ or other tissue are used to define the zone. Optionally, each organ has an imaging protocol indicating zones. In some cases, the probability of a blob being identified as a synaptic center depends on the location thereof, which may be specific to each organ and/or based on a previous image of the patient or other patients with similar characteristics.

In some embodiments, densities of synaptic centers are characterized, rather than identification of individual synaptic centers.

In some embodiments, geometric regions are targeted by providing a geometrical model of the organ of interest and then correlating it with the imaging volume of a nuclear imaging camera used for detecting radiation emission. Thereafter, radiation counts that fit in a region of interest relative to the organ may be analyzed for potential identification as a synaptic center. Optionally, the correlation uses a known radiation-emitting behavior of the organ, which may be used to provide a reconstruction thereof, at least with sufficient detail to be used for scaling and/or rotating a model. In some cases, the model of the organ is provided using CT imaging. In some cases, the organ model is a mathematical model which is modified according to a specific imaging. In some embodiments, the model defines relative locations of interest for, for example, ganglia of certain sizes.

In some embodiments, a zone is defined based on surrounding tissue type. For example, in some embodiments a region which emits as fat is optionally assumed to also include synaptic centers of interest. In another example, the zone is defined to be a vascular structure of a certain diameter.

In some embodiments, one or more temporal filters (312) may be used to identify synaptic centers. In some embodiments, a known sympathetic cycle such as caused by breathing is used to detect tissues whose emission includes a significant component that varies as a function of time, matching, for example, breathing or heart beat.

In some embodiments, one or more trigger filters (314) may be used. In some embodiments, a stimulus which is expected to have an effect on a synaptic center activity (e.g., autonomous nervous system activity), e.g., according to any one of the embodiments described herein, is used as a trigger. Radioactive tracer data acquired according to any one of the embodiments described herein is optionally analyzed so as to selectively identify a synaptic center, e.g., a tissue which is characterized by a relatively high concentration of radioactive tracer and which exhibits a response to the trigger.

In some exemplary embodiments of the invention, one or more of the methods of synaptic center identification described herein may be used iteratively. After a method is applied to identify potential synaptic centers, the method may be reapplied, e.g., to fine-tune or correct the identification and/or to support better diagnosis.

In some embodiments, the method comprises act 308.

In some embodiments, the method comprises act 310.

In some embodiments, the method comprises act 312.

In some embodiments, the method comprises act 314.

In some embodiments, the method comprises acts 308 and 310.

In some embodiments, the method comprises acts 308 and 312.

In some embodiments, the method comprises acts 308 and 314.

In some embodiments, the method comprises acts 310 and 312.

In some embodiments, the method comprises acts 310 and 314.

In some embodiments, the method comprises acts 310 and 314.

In some embodiments, the method comprises acts 308, 310 and 312.

In some embodiments, the method comprises acts 308, 310 and 314.

In some embodiments, the method comprises acts 308, 312 and 314.

In some embodiments, the method comprises act 310, 312 and 314.

In some embodiments, the method comprises acts 308, 310, 312 and 314.

In some embodiments, different types of synaptic centers may be distinguished based on their different behavior. In some embodiments, sympathetic and parasympathetic synaptic centers may uptake different tracers, and can generate different images, for example, if a multi-energy imager is used to acquire radiation data. In some embodiments, different types of synaptic centers have different temporal cycles and/or different responses to one or more stimuli. In some embodiments, the data analysis system used to analyze the acquired radioactive tracer data (e.g., a computer) has a memory storing thereon different properties of various tissue types of interest. Optionally or alternatively, synaptic centers are distinguished from non-nervous tissue based on such differences.

At 316 a nervous system (e.g., ANS) model is optionally generated, populated (e.g., by addition of identified synaptic centers to the model), and/or refined with data based on the image acquisition, and optionally used for display (324). For example, the image data may provide the number and/or relative intensity of activity of different identified synaptic centers.

In some embodiments, a model is not generated *per se;* rather, the collected information is optionally matched to a set of existing models to determine a most useful model to use (e.g., and populate using the collected data).

In some embodiments, adapting and/or refining the model may optionally also use anatomical data about an associated anatomical region and/or organ (318) (also referred as organ data). The anatomical data may be acquired according to any one of the embodiments described herein with regard to acquisition of anatomical data.

Additionally or alternatively to collecting organ data, other data (320), such as blood hormone levels is optionally collected and provided to the modeling activity (e.g., of collecting data and generating and/or modifying a model to match the data),
Data for the model may be re-acquired and/or updated, for example, within a few seconds, minutes, hours or days, depending on the application. For example, in a case of mapping the response of a synaptic center (e.g., ganglion) to a stimulus, radioactive tracer data is optionally acquired before, during and/or after the stimulus. There are stimuli that can be delivered within seconds (e.g., light flashes) or minutes (e.g., chemical stimuli via injection) or hours (e.g., rapid atrial pacing for causing atrial fibrillation) or prolonged (e.g. physical training in an athlete or the effect or remodeling of the myocardium after revascularization).

At 322 the model is optionally analyzed and at 324 the model (e.g., static or dynamic) and/or analysis results are optionally displayed to a user and/or sent to a further analysis system and/or storage.

It is to be appreciated that model information (e.g., information collected or otherwise received for generating, modifying and/or otherwise updating a model) may be generated also from non-imaging studies. For example, if a model of the ANS predicts certain behavior under certain circumstances, measuring the circumstances and the results may be used to calibrate an existing model. Optionally, such an existing model may use anatomically generated networks of synaptic centers (e.g., using normal arrangements of tissue of a general population).

In some embodiments, anatomical images are combined with the functional images acquired using a radioactive tracer, for examples, images of a distribution of radioactive tracer such as described herein, and the combined image is used as a basis for identifying and/or locating synaptic centers. The method comprises generating image masks corresponding to regions of the anatomical image containing a synaptic center (e.g., GPs and/or the innervations of an organ). The synaptic centers are typically not visible on the anatomical image, for example, cardiac GPs on a CT scan that includes the heart. The selected image masks are applied to corresponding locations on the functional image, for example, by a registration process. Synaptic center characteristics within the functional image are reconstructed, instructed by the applied mask. Synaptic centers within the selected image mask applied to the functional image are identified, based on predefined rules (e.g., as described herein), for example, size of regions characterized by a relatively high concentration of radioactive tracer and/or an intensity of radioactive emission from a region relative to surrounding intensity. In this manner, anatomical information (e.g., anatomical data described herein) is used for reconstructing the location and/or activity of synaptic centers within the functional image. The anatomical information, in the form of the mask, is used for guiding the processing to certain regions of the functional image, to help in locating the synaptic centers of interest. The identified synaptic centers may be displayed on the anatomical image, and/or may be registered with a navigation system for patient treatment such as an electrophysiological catheter navigation system for treating cardiac disorders such as arrhythmias. In this manner, the anatomical image may serve as a guide for where to look within the radioactive tracer data to identify the relevant nerve structures, where the rough location of the nerve structure within the body is known beforehand, for example, based on a predefined anatomical atlas.

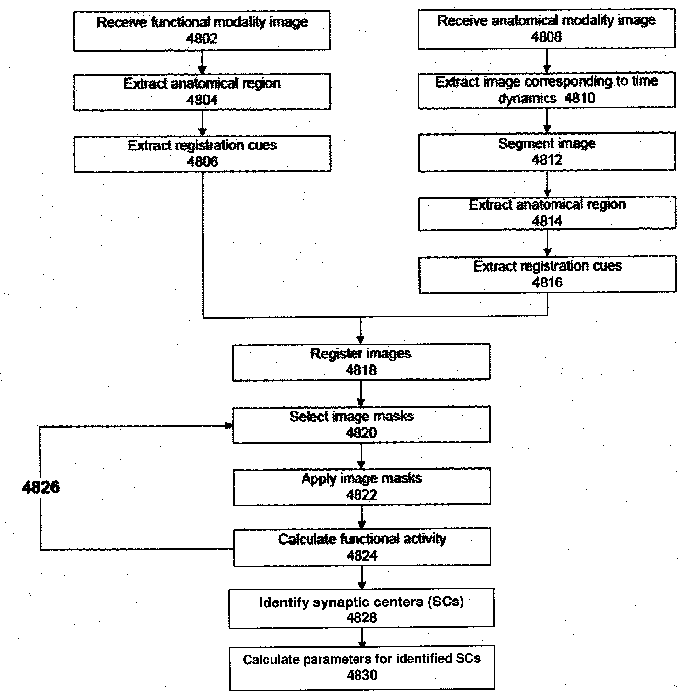

Reference is now made to FIG. 4 which is a flowchart of a method for processing functional images acquired using a radioactive tracer, for examples, images of a distribution of radioactive tracer such as described herein, to identify and/or locate synaptic centers (may correspond to method 300, and particularly to blocks 308, 310, 312 and/or 314 in FIG. 3), in accordance with some embodiments of the present invention. Optionally, the method combines the functional and anatomical images (may correspond to block 104 in FIG. 1). Alternatively, the anatomical image provides a basis for reconstructing selected parts of the functional image that contain the synaptic centers. The method may be performed, for example, by an image data processing unit. The method may use images from an anatomical imaging modality (which show organ structure, but not synaptic centers in sufficient detail or at all), e.g., as described herein, to reconstruct images from a functional imaging modality (which show a distribution of radioactive tracer, but not the organ structure in sufficient detail or at all), e.g., a nuclear imaging modality described herein. Reconstructed functional images may show the synaptic centers overlaid on the organ structure (e.g., as described herein).

Optionally, the method according to FIG. 4 provides (as an output) the general region where synaptic centers (e.g., GPs) are located. Alternatively or additionally, the method provides regions where the synaptic centers (e.g., GPs) are not located. The precise location of synaptic centers (e.g., GPs) may vary anatomically between different patients. The specific location of a synaptic center (e.g., GP) may be identified during an ablation procedure, for example, using high frequency stimulation (HFS). Alternatively or additionally, the method provides the precise location of the synaptic center (e.g., GP), for example, using a coordinate system.

Optionally, a neuronal presence is identified (e.g., according to radioactive tracer distribution) in preselected tissue regions. The image masks are defined based on the preselected tissue regions within the anatomical image that correspond to the functional activity (e.g., radioactive tracer emission) that is being detected. For example, in the heart, synaptic centers (e.g., cardiac GPs) are located within the heart wall or nearby. Image masks are defined for the anatomical image to look for the synaptic centers within the heart wall or nearby. The generated image masks are then applied to the functional data, to identify the synaptic centers based on radioactive tracer distribution within the heart wall or nearby.

Optionally the reconstruction is directed to anatomical regions where functional activity (e.g., from synaptic centers) is expected, for example, based on a predefined anatomical atlas.

Optionally, the image masks are defined to identify some or all synaptic centers and/or their activities, for example, different types of synapses and/or synapse activities.

In this manner, the image masks may serve a guide for directing the identification of the synaptic centers to certain regions within the functional image and/or data. There may be many regions of relatively high intensity within the radioactive tracer data, only a small subset of which may be relevant for diagnosis, monitoring and/or treating a medical condition, disease or disorder (e.g., as described herein). As the rough location of a synaptic center relative to body organs and/or tissues may be known (e.g., by an atlas) but not visualized on the anatomical image, the search for a synaptic center may be directed to the corresponding regions on the functional image. The search may be focused to regions having a large percentage of intensity readings that denote relevant nervous tissue.

The method according to FIG. 4 may be used to detect different types of synaptic centers, at different locations of the body (tissues, organs), for example, as described herein.

The method according to FIG. 4 may improve system performance, by performing calculations within the region of interest to identify the nervous tissue. Calculations may not need to be performed in regions without nervous tissue.

The method according to FIG. 4 may reduce radiation exposure to the patient. Additional radiation may be applied to regions containing the nervous tissue for imaging to provide higher resolution at the regions. Less radiation may be applied to regions not containing the nervous tissue.

The method according to FIG. 4 may improve analysis results and/or images. Nervous tissue within selected regions may be analyzed and/or imaged. Nervous tissue outside the selected regions may not be analyzed and/or images. Interference and/or image complexity from the nervous tissue outside the selected regions may be reduced or prevented. In this manner, nervous tissue that is not contributing to the medical condition of the patient and/or nervous tissue that is not a target (e.g., for ablation therapy) may be excluded from further analysis. Alternatively, the non-targeted nervous tissue may be identified separately from targeted nervous tissue (e.g., targeted for ablation).

Optionally, at 4802, functional imaging data (e.g., radioactive tracer data) and/or images are received, for example, a D-SPECT image or other images. The images may be of a body part, for example, a torso, an abdomen, or other body parts (e.g., based on scanning protocols). The body part includes the nervous tissue to be imaged and/or the innervated organ, for example, GPs of the heart, intestines or other organs. Optionally, the functional images includes regions of relatively high concentration of radioactive tracer (e.g., as described herein), that denote synaptic centers.

Optionally, functional data is collected from a body part that has regions where nervous system (e.g., ANS) activity is expected, and regions where nervous system (e.g., ANS) activity is not expected. For example, during imaging of the heart, data denoting nervous system activity is expected from the heart wall and/or surrounding tissues, and no nervous system activity is expected from inside the hollow chambers (containing blood). Noise may be received from areas corresponding to the inside of the heart chamber, even though no nervous system activity is expected. Optionally, the noise is removed from the functional data based on the corresponding anatomical image (e.g., after image registration). Optionally, emission intensity readings associated with noise within blood (or other fluid) filled chambers and/or vessels is removed. For example, emission intensity readings of the radioactive tracer data corresponding to heart chambers and/or surrounding blood vessels are removed.

Optionally, at 4804, an anatomical region is extracted from the image. Optionally, the tissue (which may contain nervous tissue) is separated from hollow spaces (which do not contain nervous tissue, but may contain fluid). For example, to image the heart, the wall of the left ventricle (LV) may be extracted. Alternatively or additionally, the hollow space within the LV may be extracted. It is noted that the extracted region may be a layer of tissue, such as the tissue layers forming the LV wall, instead of, for example, the LV including the hollow chamber inside the LV. For example, to image the kidney, the walls of the renal artery may be extracted and/or the inside of the artery may be extracted. When imaging other organs, dominant portions of the organ may be selected.

Optionally, at 4806, one or more registration cues are extracted from the image. The registration cues may be from the organ of interest, and/or surrounding anatomical structures, for example, LV axis, liver, heart septum, RV, torso.

Optionally, at 4808, anatomical data and/or images are received, for example, from a CT, MRI, 3D ultrasound, 2D ultrasound, or other modalities. The anatomical image denotes the structure of the tissue and/or organ innervated by the nervous tissue (e.g., a synaptic center described herein). The anatomical image denotes the tissue and/or organ structure corresponding to the location of the nervous tissue (e.g., synaptic center). The images contain the same nervous tissue to be imaged and/or the same innervated organ.

Optionally, anatomical data from an anatomical imaging modality (e.g., acquired as described herein) is received to reconstruct an anatomical image of a region of a body of a subject. Optionally, the region comprises a portion of at least one internal body part which borders on a target nervous tissue.

The anatomical images and the functional images denote corresponding regions of the body containing the synaptic centers (e.g., GPs) for identification and/or localization. For example, both anatomical imaging and functional imaging modalities may take pictures of the heart, kidney, or other organs.

For example, to image GPs of the heart, anatomical and/or functional images of the heart are obtained. For example, to image GPs of the kidney, anatomical and/or functional images of the kidney, renal artery and/or aorta are obtained.

Optionally, at 4810, images corresponding to different times during a dynamic cycle are extracted. For example, for the heart, images are extracted along the cardiac cycle, for example, the end diastolic volume (EDV) and/or the end systolic volume (ESV). In another example, for the bladder, images may be extracted for a full bladder and an empty and/or emptying bladder.

The average image may be computed, for example, (EDV+ESV)/2.

Optionally, at 4812, one or more images are segmented. Segmentation may be fully automatic and/or may require manual user intervention. An example of a system for segmentation is Carto ® from Biosense Webster ®.

Optionally, at 4814, an anatomical region is extracted. Optionally, the anatomical region corresponds to the anatomical region extracted at 4804. Optionally, the anatomical region is extracted from the segmented image of block 4812.

Optionally, at 4816, one or more registration cues are extracted from the image. The registration cues may be from the organ of interest, and/or surrounding anatomical structures, for example, LV axis, liver, heart septum, RV, torso.

Optionally, at 4818, the functional images and the anatomical images are registered. Optionally, the images are registered based on alignment of the extracted anatomical regions of blocks 4804 and 4814.

Optionally, the registration is performed to take into account the dynamics of an organ, for example, movement of the heart.

At 4820, one or more image masks are generated based on the anatomical image and/or data. Optionally, the image masks direct processing and/or visual display of the nerve tissue to specific locations of the image located within the image masks. For example, synaptic centers are displayed and/or processed within the volume of an applied image mask. Synaptic centers outside the volume of the image mask may optionally not be processed and/or displayed. Synaptic centers outside the volume of the image mask may optionally be processed and/or displayed differently than those synaptic centers inside the image mask.

Optionally, the anatomical images are processed to generate the image mask corresponding to dimensions of at least one internal body part, for example, the walls of the chambers of the heart.

Optionally, the image masks are selected and/or defined for tissue surrounding a hollow chamber, for example, the image masks are defined based on the shape of the heart chamber walls and do not include the hollow region within the chambers, the image masks are based on the shape of the arterial wall and do not include the hollow region within the artery, the image masks are based on the shape of the bladder wall and do not include the hollow region within the bladder. It is noted that synaptic centers may exist within the tissues defined by the image masks, but may not exist within the hollow spaces (which may be filled with fluid such as blood, urine or other fluids). The image masks may include tissue surrounding the organ of interest.

The image masks are defined, for example, based on image segmentation (e.g., according to the ability of the system to segment the image), based on tissue types (e.g., muscle vs. connective tissue), based on organ size, based on sub-structures within the organ (e.g., heart chambers, liver lobes, kidney parts), or other methods.

Different image masks may be generated for different tissue types, and/or for synaptic centers at different locations within the organ. For example, for synaptic centers (e.g., GPs) within the epicardium one set of image masks is generated. For synaptic centers (e.g., GPs) within the myocardium another set of image masks may be generated.

The image mask may be a 2D and/or 3D volume with a shape and/or size selected based on tissues and/or organ parts within the image. The image mask may correspond to anatomical parts believed to contain the synaptic center(s) for imaging (e.g., GPs), for example, corresponding to the walls of the four heart chambers, corresponding to the intestinal wall, bladder wall, renal artery, aortic branch region of the renal artery, kidney, or other structures. In one example, the image mask may be generated to contain synaptic centers (e.g., GPs) within the epicardial and/or myocardial tissue of the heart. In another example, the image masks may be generated to contain kidney innervating synaptic centers at the aorta-renal artery junction. It is noted that the image masks are generated based on an estimated location of the synaptic centers (e.g., based on normal patient anatomy), as the synaptic centers are often not visible on the anatomical image.

Optionally, the generated image masks correspond to the segments of the anatomical image. For example, the heart is segmented into the chamber walls, and the generated image masks correspond to the chamber walls.

For example, a first image mask is generated for the walls of each chamber of the heart. It is noted that the thickness of smaller chambers may be difficult to measure in certain images (e.g., CT). In such cases, the thickness of the first image masks for each chamber may be based on a measurable anatomical region such as the LV. Alternatively, the thickness of the chamber is measured using another imaging modality (e.g., ultrasound, MRI) and/or estimated. The measurement may be performed using the anatomical image, for example, the thickness for the image mask may be based on the thickness of the LV as measured on the CT image. Exemplary image mask thicknesses for the chambers may then be estimated based on the LV measurement, for example: 0.5 X LV thickness for the image masks of the LV, right ventricle (RV), right atrium (RA) and left atrium (LA).

Optionally, the image masks are generated and/or applied based on templates. The templates may define: the location of the innervated organ (or tissue) and/or the location of the synaptic centers within and/or in proximity to the innervated organ, outside of the organ. The templates may be generated, for example based on a predefined anatomical atlas that maps nerve structures to tissues and/or organs of the body.

Optionally, the generated image masks are adjacent to one another. Alternatively or additionally, the generated image masks overlap with each other. Alternatively or additionally the generated image masks are spaced apart with respect to one another.

At 4822, the image masks are applied to the functional image and/or data. Alternatively or additionally, the image masks are applied to the anatomical image and/or data. Alternatively or additionally, the image masks are applied to combined functional and anatomical images and/or data, for example, overlaid images.

Optionally, the image masks are applied based on the registration process (block 4818). The anatomical information serves as a guide, using the selected image masks, for selective reconstruction of synaptic center-related data within the functional image. The image masks may be correlated with the image to contain anatomical structures having the synaptic center(s). The application may be based on the image registration, for example, applied based on a common coordinate system. The image masks may be applied to a certain type of tissue containing nervous tissue. For example, the image masks may be applied to the epicardium of the heart. The image mask may be applied to have its inner surface aligned with the epicardial surface of the chamber wall, such that the image mask contains the epicardial space encompassing the chamber.

Optionally, the generated image mask is correlated with the functional data for guiding the reconstruction of a functional image depicting the target nervous tissue.

At 4824, functional activity (e.g., radioactive tracer distribution) is calculated within the applied image mask space. Optionally, the average functional activity (e.g., average radioactive tracer emission and/or concentration) is calculated. Optionally, the standard deviation of the functional activity (e.g., radioactive tracer emission and/or concentration) is calculated. For the heart, the functional activity is optionally calculated around each chamber separately, and around the entire heart. Average functional activity (e.g., average radioactive tracer emission and/or concentration) for the chambers may be denoted by *A1LV, A1RV, A1LA, A1RA.* Average functional activity (e.g., average radioactive tracer emission and/or concentration) for the heart may be denoted by *A1H.* Standard deviation of the activity may be denoted by *SD1LV, SD1RV, SD1LA, SD1RA, SD1H.*

Optionally, at 4826, one or more of 4820, 4822 and/or 4824 are repeated. Alternatively, one or more of 4820, 4822, 4824, 4828, 4830, 4832, 4834, 4836 and/or 4838 are repeated. Alternatively, one or more of all blocks in FIG. 4 are repeated. Optionally, additional image masks are generated for different anatomical parts (e.g., for different heart chambers, for different tissue layers), optionally for different tissues types containing nervous tissue. Optionally, additional image masks are generated for anatomical tissues and/or anatomical parts that are nearby and/or adjacent to the earlier analyzed anatomical parts. Different image masks may be generated, and then applied together to identify the synaptic center(s) in the vicinity of an organ. For example, different types of synaptic centers may innervate different tissues. Alternatively, different image masks are processed separately, for example, to differentiate between different synaptic centers (e.g., located within different tissues of an organ).

Alternatively or additionally, image masks are generated for different time frames, optionally on each image of the dynamic cycle (e.g., cardiac cycle). The mask may be dynamic. The mask may change over time after temporal registration. Optionally, the mask is a spatiotemporal mask. The dynamic image masks may correlate with the anatomical regions of interest during the cycle. For example, the image masks may move with the heart during the cardiac cycle, but maintaining the same relative position. For example, image masks applied to the LV wall move back and forth (and/or become smaller and larger) as the heart contracts and relaxes, but maintain the relative position against the LV wall.

Alternatively or additionally, image masks are generated for both the anatomical and the functional images. For example, image masks are generated based on the combined and/or registered images, which may form a single image, or two separate (optionally linked images).

Optionally, the anatomical images are obtained during a cyclic physiological process (e.g., cardiac cycle, bladder emptying, intestinal peristalsis). Optionally, different spatiotemporal image masks are selected for different images obtained during the physiological process. Optionally, the different spatiotemporal image masks are synchronized with the physiological process to correspond to the same location of the tissues. In this manner, the location of the tissues may be maintained as the tissues move during the physiological process.

For example, at 4820 (repeated) additional image masks are generated to detect nervous tissue (e.g., synaptic centers) within the myocardium. The size and/or shape of the myocardial masks may be different than the size and/or shape of the epicardial masks. Exemplary image mask thicknesses include: 1.2 X LV thickness for the image masks of the LV, 0.7 X LV thickness for the RV, 0.4 X LV thickness for the RA, 0.4 X LV thickness for the LA.

For example, at 4822 (repeated) the image masks are applied to the image to correlate and/or contain myocardium.

For example, at 4824 (repeated) the average and/or standard deviation of the functional activity may be calculated for the myocardium image masks. Average activity for the chambers may be denoted by *A2LV, A2RV, A2LA, A2RA.* Average activity for the heart may be denoted by A2H. Standard deviation of the activity may be denoted by *SD2LV, SD2RV, SD2LA, SD2RA, SD2H.*

Optionally, the calculated activity levels are normalized, for example, to a point or volume in the body, to a point or volume within the image mask space, or other methods. The normalization may allow for identification of the GPs.

At 4828, synaptic centers (e.g., GPs) are identified within the applied image mask space. Alternatively or additionally, synaptic centers (e.g., GPs) are identified within the organ volume and/or nearby tissues. Optionally, synaptic centers (e.g., GPs) identified within multiple different image masks are combined into a single image of all the identified synaptic centers (e.g., GPs), for example, the identified synaptic centers within the organ. Alternatively or additionally, synaptic centers (e.g., GPs) identified within corresponding image masks of multiple frames (e.g., all image masks of the LV myocardium during the cardiac cycle) over time are combined.

Optionally, synaptic centers are identified by adjusting the position and/or size and/or shape of the image mask. Optionally, the image mask is adjusted based on the corresponding anatomical image. Optionally, the image mask is adjusted to exclude regions that may not physically contain synaptic centers. Optionally, the functional data is adjusted instead of, and/or in addition to, and/or based on the adjusted image mask. For example, functional intensity data obtained from anatomical regions which may not include synaptic centers, for example, inside a hollow (e.g., fluid filled) space, such as heart chambers and/or blood vessels. The chamber itself may not contain nervous tissue. When intensity readings are detected in the chamber (e.g., next to the heart wall), the image data and/or image mask may be adjusted to reflect the estimated position of the intensity readings. Mask adjustment may be required, for example, when registration between anatomical image data and functional image data is imprecise and/or incomplete, for example, if the anatomical image data and functional image data were obtained at different angles.

Optionally, the synaptic centers within the image mask and/or organ volume are located. The relative position of one synaptic center to another may be calculated, for example, in 2D and/or 3D.

Optionally, the synaptic centers are combined together into an ANS map (e.g., an ANS model generated as described herein). Optionally, connectivity between synaptic centers is determined. Connected synaptic centers may be within the same image mask, within different images masks at different spatial locations, and/or within different image masks at different points in time (but at the same corresponding location). Optionally, the spatial relation between synaptic centers is determined. For example, the relative location between a first synaptic center with respect to the location of a second synaptic center.

Optionally, areas of extreme activity (e.g., high concentration of radioactive tracer) are identified. For example, epicardial GPs (EGP) and/or myocardial GPs (MGP) are optionally identified based on extreme activity of a radioactive tracer with affinity to adrenergic synapses (e.g., as described herein), for example, mIBG.

Optionally, synaptic centers are identified based on one or more predefined thresholds and/or rules, e.g., as described herein.

Optionally, the synaptic center (e.g., GP) may be identified by comparing calculated activity (e.g., image intensity) of a certain region to surrounding activity in the same image mask. Alternatively or additionally, the synaptic center (e.g., GP) may be identified by comparing calculated activity (e.g., image intensity) within the image mask to activity in another image mask. For example, the EGP may be identified as satisfying the rule that the total activity of the EGP is a predefined factor times the standard deviation (*SD1* and/or *SD2*), above average activity (*A1* and/or A2), and/or the adjacent activity surrounding it is lower than half of the EGP activity (e.g., correlated for volume). Optionally, the user may select and/or modify the predefined factor. For example, the MGP may be identified as satisfying the rule that the total activity of the MGP is another predefined factor times the standard deviation (*SD1* and/or *SD2*), above average activity (*A1* and/or A2), and/or the adjacent activity surrounding it is lower than half of the MGP activity (e.g., correlated for volume). Optionally, the user may select and/or modify the predefined factor.

Optionally, identification of synaptic centers is performed per frame, optionally per frame of the dynamic cycle (e.g., cardiac cycle).

Optionally, the identified synaptic center(s) is automatically related to a tissue type. Optionally, the identified synaptic center(s) is related to the tissue type based on the applied image mask. Alternatively or additionally, the identified synaptic center(s) is related to the tissue type based on the characteristics of the intensity readings, for example, large sizes (denoting large synaptic centers) may only be found in certain tissues. Optionally, different types of synaptic centers are related to different tissues.
For example, myocardial GPs are related to the myocardium and/or epicardial GPs are related to the epicardium.

Optionally, at 4830, one or more parameters are calculated for the identified synaptic centers. Examples of parameters include: average size, specific radioactive tracer level (e.g., counts per voxel of synaptic center divided by average counts in the corresponding image mask volume), power spectra (e.g., power below 1 Hz, power between 1-5 Hz, ratio of high to low frequencies), normalized power spectra, synaptic center connectivity map (e.g., connectivity and interaction between different synaptic centers), number of synaptic centers per predefined area (e.g., synaptic center density number/square centimeter).

For example, for an identified EGP, one or more of following parameters may be calculated: EGP size, EGP specific radioactive tracer level, EPG power spectra graph, EGP normalized power spectra (i.e., the difference between the EGP power at different frequencies minus the power of the total counts from the epicardial image mask space), EGP connectivity map.

For example, for identified MGP, one or more of the following parameters may be calculated: MGP number in an area and average size for each predefined area (e.g., Marshal ligament, left inferior LA wall, right inferior LA wall, other areas), MGP specific radioactive tracer level, MGP power spectra, MGP normalized power spectra (i.e., the difference between the MGP power at different frequencies minus the power of the total counts from the myocardial image mask space).

Optionally, calculation of synaptic center parameters is performed per frame, optionally per frame of the dynamic cycle (e.g., cardiac cycle).

Optionally, at 4832, the calculated and/or other parameters may be normalized. Normalization may take place at one or more blocks of the method, for example, during and/or after acquiring the functional and/or anatomical images, upon calculation of functional activity, upon identification of synaptic centers, upon calculating parameters for a synaptic center, upon comparison of data over time, or at other blocks.

Examples of one or more normalization techniques include: raw data, raw data divided by the raw data value in a known fixed anatomical location acquired at roughly the same time (for example, the activity of the tracer in the patient's mediastinum), normalization to a normal patient data set, normalization to a value of the activity at the first or the last image acquisition from a sequence of acquisitions, normalization to value acquired at different physiological state (e.g., rest, stress), a combination of some or all of the above, and/or other methods.

Alternatively, the normalization of 4832 is performed instead of and/or in addition to, before a different block in the process, for example, before synaptic centers are identified in block 4828. The normalization may help in identifying the synaptic center(s). For example, level of a radioactive tracer (e.g., as described herein, optionally mIBG) at a local region is compared to an average value and/or standard deviation across the organ volume, within the image mask space and/or relative to a predefined threshold.

Alternatively or additionally, the calculated data and/or measured functional intensity are corrected for sensitivity. Optionally, sensitivity correction is performed within each image mask and/or in related image masks. For example, some areas may have relatively higher sensitivity to uptake of the radioactive tracer, and some may have relatively lower sensitivity to the uptake of the radioactive tracer. Optionally, the anatomical data is correlated to the sensitivity. Optionally, the image masks are generated (block 4820) based on different sensitivity levels, for example, one set of image masks for higher sensitivity nervous tissue, and another set of image masks for lower sensitivity nervous tissue. Optionally, the different sensitivities are normalized to a common baseline.

Alternatively or additionally, measurements of the functional data are normalized, for example, measurements of uptake of the radioactive tracer are normalized to the level of corresponding chemical (e.g., a neurotransmitter for which the radioactive tracer is a radiolabeled derivative and/or analog of) in the subject. Optionally, intensity measurements are normalized according to the level of radioactivity of a synaptic center being identified. Optionally, measurements denoting radioactivity of the synaptic centers are taken. For example, in the case of a norepinephrine analog radioactive tracer (e.g., mIBG), measurements are normalized to the level of norepinephrine (and/or epinephrine) in the subject. For example, the level of norepinephrine is measured in the blood (e.g., by blood sample), urine, or other body fluids. The intensity of norepinephrine analog (e.g., mIBG) uptaken is normalized based on the measured norepinephrine. In another example, the level of radioactivity is measured by non-chemical methods. For example, normalization of a radioactive tracer (e.g., mIBG) is performed based on a measurements taken during a cardiac stress test (e.g., based on ECG measurements, heart rate, cardiac output, or other measurements). The measurements may be correlated with levels of activity of the synaptic tracers being identified (e.g., by a table, mathematical equation, or other methods).

Optionally, at 4834, changes in synaptic center parameters over time are identified. Optionally, dynamic changes of the calculated parameters between different acquisition times are determined. For example, the changes in synaptic center (e.g., EGP) activity over time may be calculated, from injection till 6 hours post injection, by repeating the image acquisition several times during this time window. The functional images may be acquired at more than one time after the tracer injection.

Optionally, at 4836, a functional image is reconstructed based on the mask applied to the functional data and/or image. Alternatively or additionally, an image is reconstructed based on the mask applied to the combined functional and anatomical data and/or images. The reconstructed image may contain the identified synaptic center(s), for example, as regions of increased intensity. The reconstructed image may be overlaid on the anatomical image, illustrating the physical location of the synaptic center(s).

Alternatively or additionally, the characteristics of the synaptic center(s) within the functional image are reconstructed. The reconstruction is instructed by the image mask.

Optionally, at 4838, the calculated results and/or reconstructed images are provided for presentation. For example, presented on a monitor to a physician. The calculated results may help in diagnosing the patient (e.g., as described herein) and/or in guiding treatment (e.g., as described herein).

Optionally, the results are provided for presentation on a certain frame, for example, the end systolic frame. Alternatively, results are provided for presentation on multiple frames, for example, a video of the cardiac cycle.

Optionally, the reconstructed functional image is provided for registration during a treatment procedure. The reconstructed image may be overlaid on and/or registered with anatomical images obtained during the treatment procedure. The overlaid and/or registered images may be used by the operator to physically determine locations of the synaptic center(s) during the treatment.

Optionally or alternatively, an analysis of a model and/or disease may be used to guide data acquisition, for example, a database may store the association of certain malfunctions with certain ANS parameters. Identifying a malfunction, such as prostatic hypertrophy, may suggest acquisition of data regarding certain parts of the ANS. It is known that transecting the sympathetic input to the prostate will cause a reduction of its volume by around 30 %. In patients with benign prostatic hypertrophy one may use the generic model of the ANS enervation of the prostate gland.

In some embodiments, the model is primarily functional. For example, a known input, such as breathing, is correlated to activity of different synaptic centers, for which information is then collected.

In some embodiments, a simple model (e.g., comprising a single hyperactive synaptic center) may be used to collect data. If this model predicts a response to a stimulus, the model may be used for treatment (e.g., ablation for treatment of atrial fibrillation); otherwise, a more complex model (e.g., several synaptic centers forming a feedback loop) is optionally used. In some embodiments, such changes in modeling are applied during an ablation procedure. For example, a catheter is inserted into the atrium and a synaptic center (e.g., GP) is ablated or ablated in part, then a determination is made to see if the resulting effect is as expected. If not, the model is optionally modified to match the new data. Optionally, an automated search over possible parameter spaces for the model and/or for several alternative models is made and a best matching model/parameter set is used.

A model may also be used to predict non-immediate effects of therapy, for example, by the model also modeling (e.g., having stored expected effects) healing and/or adaptive/post-therapy changes. In some embodiments, when an expected and desired effect is achieved and the model indicates that this effect predicts a desired outcome, ablation is stopped, even if the immediate effect is not the desired final effect.

### Location of nervous tissue:

In some embodiments, the identified nervous tissue (e.g., synaptic center) is localized by combining the information provided by the radioactive tracer (used to identify a synaptic center) with additional information regarding the location of identified nervous tissue (e.g., relative to the body of the subject). Such additional information may comprise, for example, anatomical data from the subject and/or a model of an anatomical region (e.g., based on anatomical data for a typical person) which may be used (e.g., using a mapping function) for associating identified nervous tissue with a location in the body.

As used herein, the phrase "anatomical data" encompasses data relating to the location of at least a portion of the body (e.g., a 2-D or 3-D image of at least a portion of the body), data relating to the location of an intrabody probe (e.g., treatment probe and/or imaging probe) and/or locational data gathered using an intrabody probe (e.g., treatment probe and/or imaging probe).

In some embodiments, an image of at least a portion of the body (e.g., presenting an image of an organ) includes an image of an intrabody probe (e.g., treatment probe and/or imaging probe).

In some embodiments, anatomical data relating to the location of an intrabody probe and/or locational data gathered using an intrabody probe (e.g., treatment probe and/or imaging probe) is used to guide a treatment in real time.

In some embodiments, the anatomical data is obtained via an imaging technique. Examples of suitable imaging techniques include, without limitation, x-ray CT, MRI, fluoroscopy, ultrasound, and nuclear imaging techniques such as SPECT and PET. The skilled person will be capable of using such imaging techniques to acquire anatomical data. X-ray CT, MRI, ultrasound, and PET are examples of techniques particularly suitable for use in combination with a radioactive tracer suitable for SPECT (e.g., 1-123-mIBG).

When anatomical data is acquired via a nuclear imaging technique such as SPECT and PET, the technique may be the same or different from the technique used to acquire radioactive tracer data.

In some embodiments, radioactive tracer data is acquired by SPECT (e.g., as described herein), and anatomical data is acquired by PET using any suitable PET technique known in the art.

In some embodiments, radioactive tracer data is acquired by PET (e.g., as described herein), and anatomical data is acquired by SPECT using any suitable SPECT technique known in the art.

In some embodiments in which SPECT is used to obtain anatomical data, the imaging agent is a Tc-99m labeled tracer. In some embodiments, a dose of the Tc-99m radiolabeled tracer is from about 2 mCi to 15 mCi, optionally from about 6 mCi to 12mCi, optionally from about 3mCi to 10 mCi, for example, about 10 mCi, about 8 mCi or about 5 mCi.

In some embodiments in which both PET and SPECT techniques are used on a subject, a signal of an imaging agent used for PET signal is distinguished from a signal of an imaging agent used for SPECT by the co-emission of two photons by PET imaging agents (and not by SPECT imaging agents).

In some embodiments, a nuclear imaging technique (e.g., as described herein) is used to simultaneously acquire radioactive tracer data and anatomical data. In some embodiments, simultaneous dual-isotope imaging is performed (e.g., using procedures known in the art).

In some embodiments, anatomical data is acquired by detection of an administered radioactive imaging agent (e.g., suitable for PET or SPECT) selected so as to exhibit a distribution in the body which overlaps relatively little with synaptic centers. Thus, a signal of the imaging agent will not overly mask the signal of the radioactive tracer in a synaptic center.

In some embodiments, an imaging agent is localized primarily to the bloodstream (e.g., following injection of the imaging agent into the bloodstream), thereby providing data regarding the location of at least one blood vessel and/or a blood-rich organ (e.g., heart).

In some embodiments, an imaging agent is localized in myocardium (e.g., following injection of the imaging agent into the bloodstream), thereby providing data regarding the location of the heart. Sestamibi, tetrofosmin and teboroxime (Tc-99m-radiolabeled imaging agents) and thallium (e.g., thallium chloride) are examples of such imaging agents.

In some embodiments, an imaging agent is localized primarily to the gastrointestinal tract (e.g., following oral administration of the imaging agent), thereby providing data regarding the location of organs of the gastrointestinal tract (e.g., stomach, intestines).

In some embodiments, an imaging agent is localized primarily to bone, thereby providing data regarding the location of bone tissue. Phosphonate and biphosphonate imaging agents are known in the art which are suitable for imaging bone tissue.

In some embodiments, an imaging agent is localized primarily to the respiratory tract (e.g., following inhalation of the imaging agent), thereby providing data regarding the location of organs of the respiratory tract (e.g., lungs, trachea).

Localization may be performed with and/or without reconstruction of an image based on data provided by the radioactive tracer (e.g., SPECT or PET data).

Reference is now made to FIG. 1, which is a flowchart of a method 100 of localizing nervous tissue (e.g., synaptic center) in an intrabody volume by combining SPECT data (e.g., acquired as described herein) and anatomical data, according to some embodiments of the present invention. It is to be appreciated that SPECT data is merely one example of a suitable nuclear imaging data, and that other nuclear imaging data (e.g., PET data) may be used instead of, or in addition to, SPECT data, in a method essentially as described in FIG. 1.

As shown at 101, anatomical data according to any one of the embodiments described herein, which optionally includes anatomical data (e.g., a fluoroscopy image) of an intrabody volume of the subject on a patient surface, is captured using an anatomical imaging modality (e.g., a fluoroscopy modality).

As shown at 102, a patient is injected with an imaging agent, that is, any one of the radioactive tracers described herein or any combination thereof. In some embodiments, an additional imaging agent (e.g., sestamibi) for providing anatomical data is also injected (e.g., as a cocktail comprising the radioactive tracer and the additional imaging agent).

Then, as shown at 103, a SPECT modality having one or more radiation detectors is used to acquire SPECT data, for example a SPECT image, describing a distribution of radioactive tracer according to any one of the embodiments described herein relating to SPECT data acquisition.

As shown at 104, the anatomical data (e.g., an image of at least a portion of a subject's body) and the SPECT radioactive tracer data are combined to allow locating the nervous tissue, for instance as shown at 105, and optionally according one or more mapping functions, as described herein. For example, the combination is optionally performed using a correlation matrix which may be calculated in advance to the specific intrabody volume imaged in the radioactive tracer data and anatomical data. The radioactive tracer data may optionally be in a form of a map showing a distribution of synaptic centers (e.g., synapses, ganglia), as described herein. The combination may optionally be performed by presenting the SPECT data side-by-side with, registered with and/or overlaid on the anatomical data. In some embodiments, a layered image is formed where SPECT data (e.g., a map) is added as an additional layer on top of an anatomical image (e.g., a fluoroscopy image), for example, an image of an organ.

In some embodiments, the radioactive tracer data and the anatomical data using a hybrid imaging device that combines a fluoroscopy modality for acquiring anatomical data (e.g., an electrophysiology fluoroscopy modality), which may optionally include an x-ray projection imaging module (e.g., x-ray image intensifier) that converts x-rays into a visible image, as well as a nuclear imaging modality, optionally a SPECT modality, for acquiring radioactive tracer data, and optionally having a plurality of radiation detectors. The hybrid imaging device is optionally configured to image a patient on a patient surface, which may optionally be adjusted to support a standing patient, a laying patient, a seating patient, and/or a leaning patient. For example, the surface may be a horizontal alignment surface, such as a patient bed, a vertical alignment surface, such as a wall or a back of a chair and the like. The patient surface is optionally made of a material that allows X-ray to pass from an actual X-ray source to an image intensifier via the patient on the patient surface.

It is to be appreciated that combining the anatomical data and the radioactive tracer data increases the signal-to-noise ratio (SNR) of the radioactive tracer data. Registering the radioactive tracer data and the anatomical data indicates an anatomical region in which different tracer distributions and/or changes in distribution (e.g., uptake and/or washout rates) are observed. This facilitates filtering the radioactive tracer data according to an estimated volume in the anatomical data in which a target nervous tissue is located, for example as described herein.

Optionally, the localization starts before or simultaneously with the injection of the imaging agent. In some embodiments, imaging of anatomical data begins immediately after the injection of the imaging agent. In some embodiments, the imaging begins after a delay of up to about 5 minutes after the injection. In some embodiments, the imaging begins after a delay of up to about 10 minutes after the injection. In some embodiments, the imaging begins after a delay of up to about 30 minutes after the injection. In some embodiments, the imaging begins after a delay of up to about 1 hour after the injection. In some embodiments, the imaging begins after a delay of from about 1 to 10 minutes after the injection. In some embodiments, the imaging begins after a delay of from about 5 to 20 minutes after the injection. In some embodiments, the imaging begins after a delay of from about 10 to 30 minutes after the injection. In some embodiments, the imaging begins after a delay of from about 15 to 60 minutes after the injection. In some embodiments, the imaging begins after a delay of from about 30 to 120 minutes after the injection. In some embodiments, the imaging begins after a delay of from about 1 to 6 hours after the injection. In some embodiments, the imaging begins after a delay of from about 5 to 48 hours after the injection.

Optionally, more than 2 imaging steps are provided for acquiring anatomical data. Optionally, the acquisition of anatomical data includes acquisition of dynamic physiological processes, such as perfusion, uptake, washout, and the like.

In some embodiments, the reconstructed image is of a certain tissue region, or a volume or a selected region of interest.

In some embodiments, the reconstruction is based on voxels or segments, model based, or a combination thereof.

In some embodiments combination of radioactive tracer data with anatomical data is made by aligning an image containing radioactive tracer data such that identified synaptic centers fall in an appropriate anatomical region (e.g., cardiac ganglia being aligned with an atrial wall).

In some embodiments, localization is used for monitoring the nervous tissue, for example, comparing data from a plurality of sessions held over the course of a treatment period. The treatment period may be, for example, a day, a week, a month, a year or any intermediate or shorter period.

According to some embodiments of the present invention, the method comprises associating regions (e.g., synaptic centers) identified in the radioactive tracer data (e.g., a SPECT image, a PET image) with corresponding organs and/or tissues.

In some embodiments, the aforementioned association does not require any additional data (i.e., in addition to the radioactive tracer data) relating specifically to the body of the subject. For example, general anatomical knowledge (e.g., relating to a typical distribution of synaptic centers in a body) may be used.

In some embodiments, the aforementioned association is effected by mapping nervous tissue and/or tissue surrounding the nervous tissue according to one or more mapping functions.

For example, FIG. 2 is a flowchart of a method of localizing a nervous tissue based on an association of different regions identified in the radioactive tracer data with corresponding organs and/or tissues, according to some embodiments of the present invention. 102, 103 may be as described above. It is to be appreciated that SPECT data is merely one example of a suitable nuclear imaging data, and that other nuclear imaging data (e.g., PET data) may be used instead of, or in addition to, SPECT data, in a method essentially as described in FIG. 2. In 201, a mapping function is provided, for example from a memory of a computing device. As shown at 202, the mapping function is used for associating one or more regions in the SPECT image to one or more organs and/or tissues. As shown at 203, the mapping allows localizing a target tissue in relation to surrounding tissues, for separating a nervous tissue from a surrounding area.

In some embodiments, the mapping function utilizes anatomical data as described herein (e.g., angiographic data).

In some embodiments, the mapping function is defined in advance. The mapping function may utilize, for example, multivariate analysis of radioactive tracer data (e.g., time-dependent data). In some embodiments, the mapping function differentiates between regions characterized by different kinetic behavior, uptake rate and/or washout rate.

In some embodiments, localizing comprises identifying at least one region of interest (ROI) in an intrabody area or volume according to a match with a reference value representing a reference uptake and/or washout rate of the radioactive tracer for a particular region in the body, thereby identifying the ROI with that particular region in the body. In some embodiments, the reference value is associated with a response (e.g., nervous response) to a stimulus (e.g., a stimulus described herein), the method comprising stimulating a nervous tissue in the intrabody area or volume with such a stimulus in order to facilitate identification. In some embodiments, the method further comprises filtering at least part of a representation of the intrabody area or volume in the radioactive tracer data based on a match with a reference value (e.g., by removing noise from regions other than a ROI. In some embodiments, the radioactive tracer is radioactive mIBG (e.g., I-123-mIBG), and the reference value is for mIBG uptake.

In some embodiments, the localization is used for guiding a medical treatment (e.g., as described herein). Examples of suitable medical treatments include, without limitation, a denervation procedure, such as renal denervation or denervation of ganglia in the atria; ablation of a synaptic center (e.g., imaged as described herein), for example, a cardiac ganglionated plexus, a pelvic plexus and/or a celiac ganglion; a muscle ablation procedure (e.g., of the atrial and/or ventricular walls); an innervation modulation procedure; blood treatment and/or a stent placement procedure (e.g., in a blood vessel) guidance.

In some embodiments, the localization is used for guiding an ablation of ganglia (e.g., ganglia associated with the atria), for example, for treating atrial fibrillation (AF). In some embodiments, ablation is performed during a catheterization procedure, optionally based on a combination of radioactive tracer data and anatomical data. Optionally, the catheterization uses an intracardiac echocardiography (ICE) catheter. In such embodiments, imaging data from the ICE catheter includes anatomical data that may optionally be combined with the radioactive tracer data for facilitating localization.

In some embodiments, the localization is a real time localization which is based on radioactive tracer data (e.g., SPECT data) and anatomical data which are captured simultaneously (e.g., via a simultaneous dual-isotope technique) and/or during a treatment period, for example during a medical treatment.

In some embodiments, radioactive tracer data (e.g., SPECT data) is acquired before a medical procedure (e.g., heart treatment) is initiated, for example few hours and/or a day before a procedure. In some embodiments, the radioactive tracer data is acquired shortly prior to the beginning of an invasive medical procedure, for example, before the subject enters a catheterization laboratory (CathLab) room. For example, the radioactive tracer data is optionally introduced to a workstation before the catheterization process.

In some embodiments, the radioactive tracer data is registered with (e.g., layered with) anatomical data, optionally an electro-anatomical map and/or a CT map, for example, by using a CartoMerge™ module. This allows providing an operator with an accurate guidance during a treatment, such as an ablation procedure, which may optionally be used to navigate a treatment probe, such as an ablation device (e.g., a radiofrequency probe) and/or a cryosurgery probe. When radioactive tracer data is forwarded to a workstation, the workstation optionally merges a real time electro-anatomical map with the radioactive tracer data acquired as described herein, and optionally also with a CT map. The results may optionally be presented to the operator as colored targets on the electro-anatomical map.

In exemplary embodiments in which SPECT is used, the subject is injected with an 1-123 radiolabeled tracer, for example, I-123-mIBG, e.g., at a dose described herein. The patient is optionally also injected with a supporting radiolabeled imaging agent, such as a Tc-99m labeled tracer (which is suitable for detection by SPECT) for perfusion mapping (e.g., cardiac perfusion mapping and/or perfusion gated SPECT), for example, an imaging agent such as sestamibi, tetrofosmin, and/or teboroxime, optionally at a dose described herein. The radioactive tracer detection and/or perfusion imaging (imaging agent detection) optionally occur at rest and/or at stress (e.g., physical exertion).

In some embodiments, the radioactive tracer data and anatomical data are obtained by measuring each radiolabeled agent (e.g., an 1-123 labeled radioactive tracer and a Tc-99m labeled imaging agent) one after the other, for example, by first injecting the radioactive (e.g., 1-123 labeled) tracer (e.g., I-123-mIBG), image the radioactive tracer, then inject Tc-99m imaging agent and image the imaging agent.

In some embodiments, the radioactive tracer data and anatomical data are obtained by measuring the radiolabeled agents (e.g., an 1-123 labeled radioactive tracer and a Tc-99m labeled imaging agent), thus obtained fully registered images of the tracers, and in a shorter time frame. In another example both tracers are injected with the dose ratio of about 2:1 between the Tc-99m labeled tracer and the 1-123 labeled tracer. For example, about 10 mCi of a Tc-99m labeled imaging agent (such as sestamibi) is optionally injected simultaneously with about 5 mCi of an 1-123 labeled tracer (such as I-123-mIBG). In some embodiments, a ratio of between about 1:1 to 3:1 is used, optionally from about 1.5:1 to 2.5:1.

In some embodiments, radioactive tracer data and/or imaging agent data (for cardiac perfusion imaging), for example, simultaneous acquisition of radioactive tracer data and imaging agent data as described herein, includes photon acquisition over a period of time of up to about 20 minutes (e.g., a period of time described herein for acquiring radioactive tracer data). In some embodiments, photon acquisition is performed at multiple time points (e.g., separated by a wait period of any duration described herein), for example, for providing an early image and a late image.

### Diagnosis and monitoring:

The imaging method disclosed herein relates to diagnosis and/or therapy control based on synaptic center distribution, as determined according to any one of the examples described herein for imaging. In some examples, the imaging method is used for diagnosing and/or monitoring a medical condition or disease or disorder. In some examples, the medical condition and/or disease or disorder is associated with an autonomic nervous system. In some examples, the medical condition and/or disease is associated with abnormal autonomic nervous system activity, for example, a disturbance in an input or involvement of the autonomic nervous system, which is diagnosed. In some exemples, the diagnosis and/or therapy control relates to the heart.

Thus, also described herein is a radioactive tracer (e.g., any one of the radioactive tracers described herein, or any combination thereof) for use in a method of diagnosing and/or monitoring a medical condition and/or disease. The medical condition and/or disease may be associated with an autonomic nervous system. The method of diagnosing and/or monitoring may use an imaging method according to any one of the embodiments described herein. In some examples, including the present invention, the radioactive tracer is radioactive mIBG (e.g., 1-123 -mIBG).

Also described herein is a use of a radioactive tracer (e.g., any one of the radioactive tracers described herein, or any combination thereof) in the manufacture of a diagnostic agent for diagnosing and/or monitoring a medical condition and/or disease. In some examples, the medical condition and/or disease is associated with an autonomic nervous system. In some examples, the diagnostic agent is for use in an imaging method according to any one of the embodiments described herein. In some examples, the radioactive tracer is radioactive mIBG (e.g., 1-123 -mIBG).

As used herein, the terms "diagnose", "diagnosing" and "diagnosis" and variations thereof refer to characterization of an activity, structure, behavior and/or any other feature of at least a portion of the body. For example, diagnosis may optionally consist essentially of characterization of connections between different parts of the body (e.g., between an organ and an autonomic nervous system).

Diagnosis and/or monitoring may be performed with or without localization of nervous tissue as described herein. For example, a pattern of synaptic centers distribution and/or activity provides sufficient information for diagnosis and/or monitoring, without requiring knowledge of the precise location of the synaptic centers (e.g., by localization as described herein). Alternatively, localization is utilized in order to facilitate diagnosis and/or monitoring, for example, to distinguish between closely positioned synaptic centers and/or to facilitate a treatment which is performed in association with the diagnosis and/or monitoring (e.g., a treatment guided by the diagnosis).

In some examples and embodiments of any of the methods and systems described herein, normal or abnormal synapse distribution and/or activity is determined by comparing the distribution and/or activity imaged in a subject with one or more sets of reference synapse distributions. An expected effect of synaptic activity (e.g., autonomic nervous system activity) may optionally be determined based on the comparison with reference data. A reference synapse distribution may be indicative of a normal synapse distribution and/or activity. A reference synapse distribution may be indicative of a medical condition and/or disease or disorder characterized by abnormal synapse distribution and/or neuronal activity. Optionally, different distributions are provided for different situations, such as according to gender, age, nationality, disease state, medication, stress, exercise, and stimuli.

Optionally, a medical condition and/or disease is identified based on a match between the existing distribution and one or more sets of reference distributions characterizing diseases and/or medical conditions. Optionally, such sets of reference distributions are acquired for a range of patients and/or healthy people and/or conditions and characterized, for example, by relative intensities, type of synaptic activity (e.g., adrenergic vs. cholinergic), location and/or sizes of "hot spots" (regions characterized by high concentration of radioactive tracer) and/or location and/or sizes of "cold spots" (regions characterized by low concentration of radioactive tracer).

In some cases, synapse density can teach about the disease process and/or about any remodeling that the nervous system may be undergoing or has undergone. It is important to note that in many patients the nervous system is highly dynamic in nature and the density and activity (e.g., amount and/or type of activity) of synaptic centers may respond to a disease and/or a therapy.

Synaptic distribution and/or neuronal activity may be characterized (e.g., as described herein) at time points (e.g., different imaging sessions) before and after a treatment, and the method may further comprise evaluating an effect of the treatment on autonomic nervous system activity.

In some examples, a change in the nervous system (e.g., caused by a disease or therapy) is identified by performing at least two imaging sessions (e.g., as described herein) and identifying a change between two imaging sessions. In some examples, treatment of a medical condition and/or disease (e.g., as described herein) is effected between the two imaging sessions.

In some examples, the diagnosis comprises estimating an effect of an autonomic nervous system on an organ (e.g., an organ affected by a medical condition and/or disease described herein), for example, by characterizing a functional effect of an autonomic nervous system activity and/or distribution on the organ, or vice versa.

In some examples, an imaging method described herein comprises distinguishing between afferent activity and efferent activity of an autonomic nervous system, by characterizing an effect of stimulation (e.g., at locations which are more peripheral and/or more central than a given synaptic center) on the nervous system, as determining by the imaging method.

In some examples, diagnosis takes into account both distribution and activity. For example, distribution may optionally indicate potential reactivity of innervated tissue whereas activity may optionally indicate the degree of utilization of that potential. The combination may also show the evenness of innervation and/or stimulation/control of the tissue (e.g., the heart).

Diagnosis may be used to estimate damage and/or prognosis of healing of a cardiac infarct. For example, it is sometimes the case that damage to nervous tissue is different from damage to cardiac muscle and/or that regeneration is different. Imaging of the heart can indicate, for example, portions of the heart which are not suitably innervated and thus may be the cause of cardiac chamber remodeling, mitral regurgitation, heart failure and/or cardiac dissynchrony.

An imaging method described herein may be used for monitoring an effect of a therapy of the medical condition and/or disease. In some embodiments, an effect of the autonomic nervous system and/or an organ is evaluated, before, during and/or after a therapeutic treatment. The evaluated effect may be an activity, a change in activity, and/or a response to a stimulus (e.g., a therapeutic treatment).

The effect of a treatment intended to affect nervous tissue, such as treatment with beta blockers and/or denervation (e.g., renal denervation, cardiac ganglion or ganglionated plexus ablation, pelvic plexus ablation, celiac ganglion ablation) may be monitored. In some examples, monitoring comprises comparing nerve activity to mechanical response of an organ (e.g., the heart), for example, an amplitude and/or velocity of movement (e.g., cardiac wall movement). Optionally, the treatment is modified, e.g., stopped, continued, increased and/or changed, based on the measurements.

In some examples, the effect of a chronic condition such as, for example, hypertension, cardiac arrhythmia, prostatic hyperplasia, autoimmune disease or disorder, diabetes, erectile dysfunction, irritable bowel syndrome and/or stress are monitored by monitoring synaptic center (e.g., ganglion) distribution and/or activity, optionally in conjunction with cardiac response.

In some examples, the measurements described herein are used to guide a therapy of a medical condition or disease (e.g., as described herein).

In some examples, the measurements described herein are used to select placement for a pacemaker or other cardiac electrical controller electrodes. For example, electrodes used for arrhythmia treatment are optionally placed where they can subdue, dampen and/or capture more highly activated tissue. In another example, pacemaker electrodes are placed according to an expected effect of the electrical stimulation on nervous activity and/or conduction. In another example, electrodes are placed so as to block conduction of stimuli from one region to another region and/or to reduce reactivity of cardiac tissue, further to over-activity of nervous tissue. In some embodiments, a method and/or apparatus as described in U.S. Patent No. 8,306,616 is used.

Thus, also described herein is a radioactive tracer (e.g., any one of the radioactive tracers described herein or any combination thereof) for use in guiding a therapy of a medical condition and/or disease. In some embodiments, the medical condition and/or disease is associated with an autonomic nervous system. In some examples, the radioactive tracer is radioactive mIBG (e.g., I-123-mIBG).

Also described herein is a use of a radioactive tracer (e.g., any one of the radioactive tracers described herein or any combination thereof) in the manufacture of a diagnostic agent for guiding a therapy of a medical condition and/or disease. The medical condition and/or disease may be associated with an autonomic nervous system. In some examples, the radioactive tracer is radioactive mIBG (e.g., I-123-mIBG).

In some examples and embodiments of any of the methods and systems described herein, the measurements described herein are used to guide ablation of nerves, a synaptic center such as a ganglionated plexus or other ganglia (e.g., cardiac ganglionated plexus, pelvic plexus, celiac ganglion), and/or the outer surface of the heart. In some embodiments, measurement is applied after (or during) ablation in order to monitor an effect of ablation and optionally repeat or modify ablation as needed.

The medical condition and/or disease may be one which is caused, exacerbated or sustained by an input of involvement of an autonomic nervous system.

Examples of medical conditions and/or diseases which may be treated, diagnosed and/or monitored according to some embodiments of the invention include (according to any of the aspects of the invention described herein), without limitation, hypertension, cardiac arrhythmias, prostatic hyperplasia (e.g., benign prostatic hyperplasia), autoimmune diseases and disorders, diabetes, stress, erectile dysfunction, irritable bowel syndrome, thyrotoxicosis, hypertension, hypertrophic cardiomyopathy, chronic obstructive pulmonary disease, syncope, hypothyroidism, idiopathic heart failure, asthma, deposition diseases (e.g., amyloidosis, deposition disease of lung), pathological weight gain, tortocolis (contraction of ipsilateral sternocledomastoid muscle in neck), idiopathic dilated cardiomyopathy, right ventricular outflow tachycardia, Brugada syndrome, tetralogy of Fallot (after repair), hypertrophic obstructive cardiomyopathy, sleep apnea, asthma, metabolic derangement of liver, hyperhydrosis, excessive salivation and excessive lacrimation. Rheumatoid arthritis is a non-limiting example of an autoimmune disease. Atrial fibrillation is an exemplary cardiac arrhythmia. Examples of diseases or disorders associated with ANS hyperactivity include, without limitation, tortocolis (associated with excessive muscle contraction), hypertrophic cardiomyopathy, idiopathic dilated cardiomyopathy, right ventricular outflow tachycardia, Brugada syndrome, tetralogy of Fallot (after repair), hypertrophic obstructive cardiomyopathy, deposition disease or lung, sleep apnea, asthma (associated with hyperactivity in airways), metabolic derangement of liver (e.g., associated with ANS), hyperhydrosis (associated with hyperactivity in skin region), excessive salivation and excessive lacrimation.

The measurements described herein may be used to select locations for drug eluting patches which elute, for example, stimulatory or inhibitory compounds (e.g., drugs) to a nervous tissue and/or associated tissue (e.g., heart tissue innervated by the nerve tissue) and/or compounds which encourage growth of nervous tis sue.

In some examples, diagnosis includes identifying parts of the heart which do not react as desired when an increase in demand is placed on the heart, for example, based on reduced activity and/or reduced mechanical reaction. In some embodiments, a map showing delay to activation time and/or conduction velocity is correlated with nerve activation. This may identify, for example, locations which are over-activated, in an attempt by the heart to compensate for delayed activation and/or conduction problems. A therapy may include balancing the activity (or changing the balance of activity in a desired manner) of certain regions, for example, by modulating the neural input and/or or affecting the underlying or associated heart condition.

In some examples, such measurements are used to assess causes of hypertrophy and/or hypotrophy in some or all of the heart. For example, patients with right ventricular heart failure may present with compensatory neural activation of the weaker tissue, which in some cases will have a spillover effect on the normal tissue. The neural effect beyond a certain level will cause a reduction on activity that can be treated by local blockade of the increase neural stimulation. Such spillover may also be implicated, for example, in arrhythmia.

It is expected that during the life of a patent maturing from this application many relevant diagnoses and treatments will be developed which can benefit from imaging of synaptic centers, and the scope of the terms "diagnosis" and "treatment" is intended to include all such new technologies *a priori.*

### Modulation of synaptic center activity:

As exemplified herein, the imaging method described herein may be particularly useful when used to modulate an activity of an imaged synaptic center.

Thus, the treatment may comprise modulating neuronal activity in at least one synaptic center imaged by the method.

The modulation may optionally be used in the context of treating a disease or disorder associated with an abnormal autonomic nervous system activity.

Thus, also described herein is a method of treating a disease or disorder associated with an abnormal autonomic nervous system activity, the method comprising imaging, according to a method described herein, at least one synaptic center of the autonomic nervous system associated with the disease or disorder in a subject in need thereof, and treating the disease or disorder based on the results of the imaging. In some embodiments, treatment comprises modulating neuronal activity in at least one synaptic center imaged by the imaging, thereby treating the disease or disorder.

According to some examples and embodiments of any of the methods and systems described herein, the imaging method described herein further comprises guiding a treatment based on the imaging of at least one synaptic center.

The treatment may comprise local treatment of a synaptic center (e.g., a ganglion, a ganglionated plexus), wherein imaging of a synaptic center as described herein is used to guide the local treatment. Examples of local treatment include, without limitation, ablation (e.g., laser ablation, radiofrequency ablation, rotoablation) of nervous tissue (e.g., a synaptic center), stimulation of a synaptic center, and local administration of a therapeutic agent.

The modulating may comprise ablation of the synaptic center.

The synaptic center to be modulated may be selected from the group consisting of a cardiac ganglionated plexus, a pelvic plexus and a celiac ganglion.

The method may comprises a catheterization procedure. In some examples, the catheterization procedure is guided by an imaging method described herein.

According to some examples of the invention, the disease or disorder is selected from the group consisting of cardiac arrhythmia, prostatic hyperplasia, an autoimmune disease or disorder, diabetes, stress, erectile dysfunction, and irritable bowel syndrome.

Treatment of such conditions is described in more detail in the Examples section herein.

In some embodiments and examples, radioactive tracer data (e.g., SPECT data) is combined with anatomical data, as described herein, so as to localize ganglionic plexuses (GPs) in the atria. This allows guiding a medical procedure, e.g., a catheterization procedure, based on combination between the anatomical data and the radioactive tracer data (e.g., SPECT data).

The treatment may comprise ablation (e.g., radiofrequency ablation) of nervous tissue in the vicinity of a cardiac atrium, for example, one or more cardiac ganglionated plexuses (GP). In some such examples, the radioactive tracer data is obtained via SPECT, and combined with anatomical data (e.g., as described herein). Anatomical data may also be obtained via SPECT (e.g., using a Tc-99m radiolabeled imaging agent), as described herein.

Localization of GPs (e.g., as described herein) may indicate to an operator where the GPs are located, allowing the operator to ablate some or all of them by operating an ablation unit located at the tip of a catheter, for example an ablation unit as described in International Patent Application PCT/IL2013/051045. In some embodiments, localization is performed using a combination of SPECT radioactive tracer data and anatomical data. The ablation unit is optionally used for high-frequency stimulation when guided to proximity with some or all of the above GPs and/or another synaptic center. Pre-acquired segmented images may be used, for example anatomical imaging data from a model indicating the location of some or all of the above GPs, for example imported via an image integration module, such as a CartoMergeTM module. Optionally, data pertaining to the treated GP is acquired and used for the imaging and/or the guiding of the treatment process, for example, spatial distribution and/or thickness of an epicardial fat in the surrounding area, for example, of the fat pad wherein the GP is located. Ablation of GP is an optional treatment for patients with paroxysmal or persistent atrial fibrillation.

Optionally, the radioactive tracer data (SPECT data) is segmented before combination with anatomical data. For example, pulmonary vein (PV) sections, left atrium (LA) sections, and/or GPs are segmented, optionally based on a match with a model.

Reference is now made to FIG. 5, which is a flowchart of a method for performing an ablation treatment by mapping complex fractionated atrial electrograms (CFAE) sites, contractile force (CF) sites, and/or dominant frequency (DF) sites in the atria as target areas, according to some examples of the disclosure. First, CFAE, CF, and DF sites are mapped. Then, intersections between the CFAE, CF and/or DF, and/or CFAE, CF, DF and/or GPs are calculated. The intersections may be with anticipated sites of anatomically known GPs, for example the above described GPs, with pre-acquired localized GPs, for example GPs which are identified in the radioactive tracer data (e.g., SPECT data), and/or intersections with GPs located in real time by high-frequency stimulation. Then, the intersections are selected as target areas for ablation. In some embodiments, an intersection between a CFAE site and at least one GP is targeted for ablation. In some embodiments, an intersection between a CFAE site, a DF site and at least one GP is targeted for ablation. An intersection between a CFAE site, a CF site and at least one GP may be targeted for ablation. Optionally, a full intersection is preferred over a partial intersection.

The method may comprise identifying at least one GP from the following GPs : superior left GP (SLGP), inferior left GP (ILGP), anterior right GP (ARGP), inferior right GP (IRGP), and Marshall GP, and optionally guiding a treatment given to the at least one GP based on the identification.

It is expected that during the life of a patent maturing from this application many relevant techniques for modulating activity of synaptic activity will be developed, and the scope of the term "modulating" is intended to include all such new technologies *a priori.*

### General definitions:

As used herein the term "about" refers to ± 10 %.

The terms "comprises", "comprising", "includes", "including", "having" and their conjugates mean "including but not limited to". This term encompasses the terms "consisting of' and "consisting essentially of".

The phrase "consisting essentially of' means that the composition or method may include additional ingredients and/or steps, but only if the additional ingredients and/or steps do not materially alter the basic and novel characteristics of the claimed composition or method.

As used herein, the singular form "a", "an" and "the" include plural references unless the context clearly dictates otherwise. For example, the term "a compound" or "at least one compound" may include a plurality of compounds, including mixtures thereof.

The word "exemplary" is used herein to mean "serving as an example, instance or illustration". Any embodiment described as "exemplary" is not necessarily to be construed as preferred or advantageous over other embodiments and/or to exclude the incorporation of features from other embodiments.

The word "optionally" is used herein to mean "is provided in some embodiments and not provided in other embodiments". Any particular embodiment of the invention may include a plurality of "optional" features unless such features conflict.

Throughout this application, various embodiments of this invention may be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals therebetween.

Various embodiments and aspects of the present invention as delineated hereinabove and as claimed in the claims section below find experimental support in the following examples.

### EXAMPLES

Reference is now made to the following examples, which together with the above descriptions illustrate some examples of the disclosure, in a non-limiting fashion.

### EXAMPLE 1

### Imaging of adrenergic synaptic activity in heart

A human patient was administered I-123-radiolabeled mIBG, a radioactive tracer for adrenergic synapses of the sympathetic nervous system. An image based on mIBG distribution was obtained by SPECT using a D-SPECT® Cardiac Imaging System (Spectrum Dynamics, Israel). The anatomy of the heart was determined by performing x-ray CT imaging and segmenting the obtained x-ray CT images. The x-ray CT data was then combined with the SPECT data so as to construct an image showing mIBG distribution in the heart. Images showing levels of adrenergic synapse activity in the left atrium and right ventricle are presented in FIGs. 6A-8B.

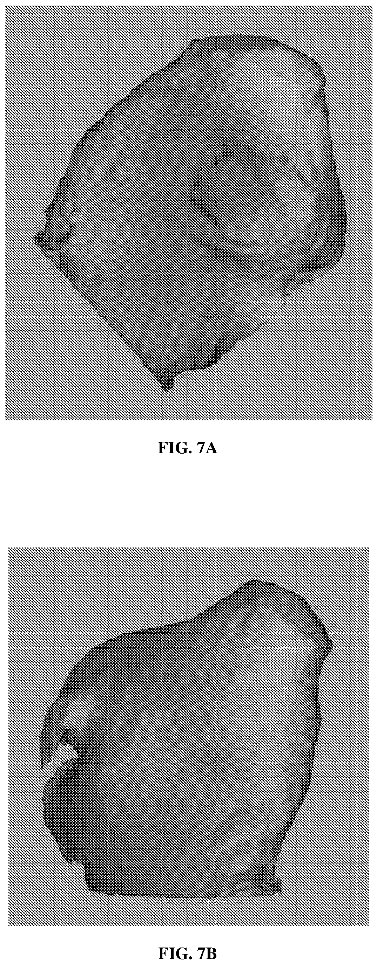

As shown in FIGs. 6A-6D, as well as in FIGs. 7A and 7B, regions with a high level of adrenergic synapses (red) were observed in certain regions of the left atrium, whereas most of the left atrium was characterized by a low level of adrenergic synapses (green). FIG. 6A shows with a high level of adrenergic synapses in the left inferior pulmonary vein. FIG. 6C shows with a high level of adrenergic synapses in the right superior pulmonary vein.

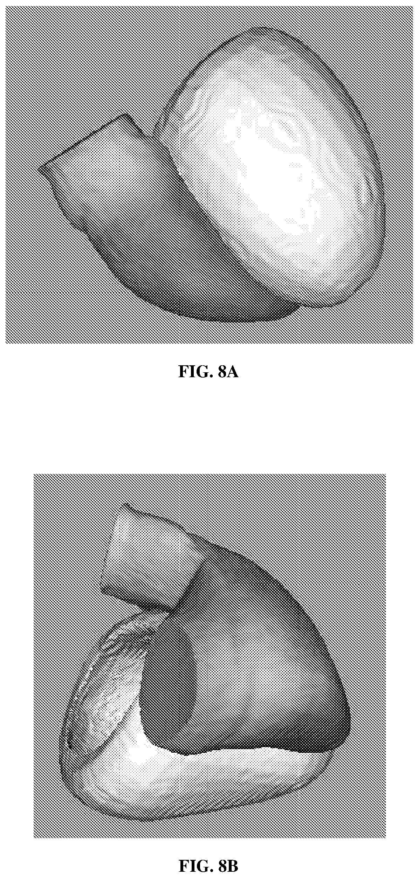

As shown in FIGs. 8A and 8B, a region with a high level of adrenergic synapses (red) was observed in a region of the interventricular septum, whereas the rest of the right ventricle was characterized by a low level of adrenergic synapses (green).

The "hot spot" of activity associated with the interventricular septum is of particular interest, as it has apparently not been known in the art, and not been heretofore used for planning treatment and/or diagnosis.

### EXAMPLE 2

### Imaging of cardiac ganglionated plexuses

In order to image cardiac ganglionated plexuses, a 60 year old male subject, having a body mass index (BMI) of 22 (height 176 cm; weight 69 kg) and a medical history of ventricular arrhythmias, chest discomfort, low ejection fraction (LVEF (left ventricular ejection fraction) = 35 %), and non-ischemic cardiomyopathy, was administered I-123-radiolabeled mIBG, and images based on mIBG distribution were obtained by SPECT using a D-SPECT® Cardiac Imaging System (Spectrum Dynamics, Israel). The anatomy of the heart and its surroundings was determined by performing x-ray CT imaging and segmenting the obtained x-ray CT images. The x-ray CT data was then combined with the SPECT data so as to construct an image showing mIBG distribution in the region of the heart, as described in FIG. 1.

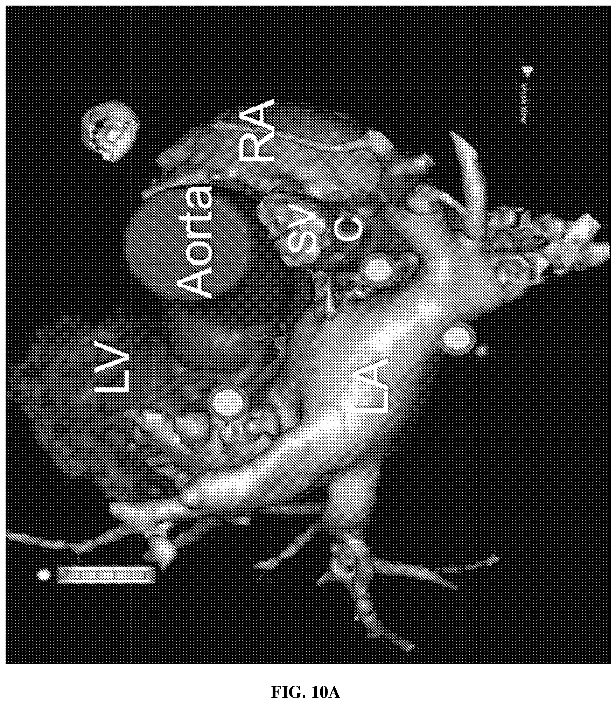

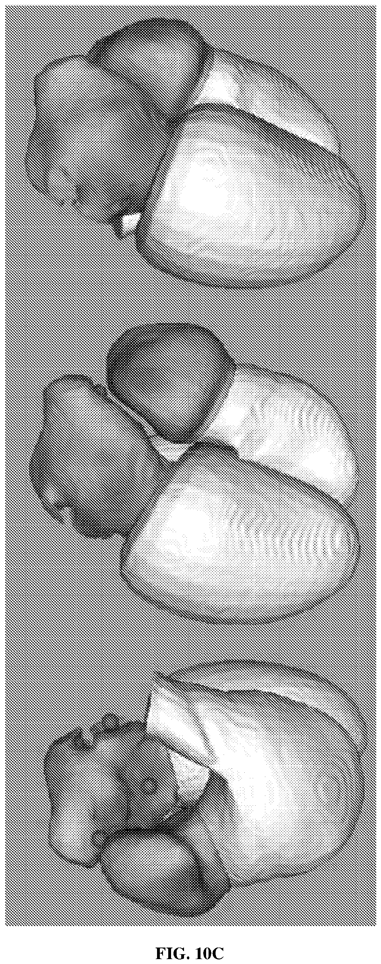

FIGs. 9A-9C and 10A-10C show the location of three identified (relatively large) synaptic centers adjacent to the heart of the subject. FIG. 10C further shows minor (i.e., relatively small) centers of adrenergic synapses (red shading of atrial surface) in the atria in addition to the three identified synaptic centers.

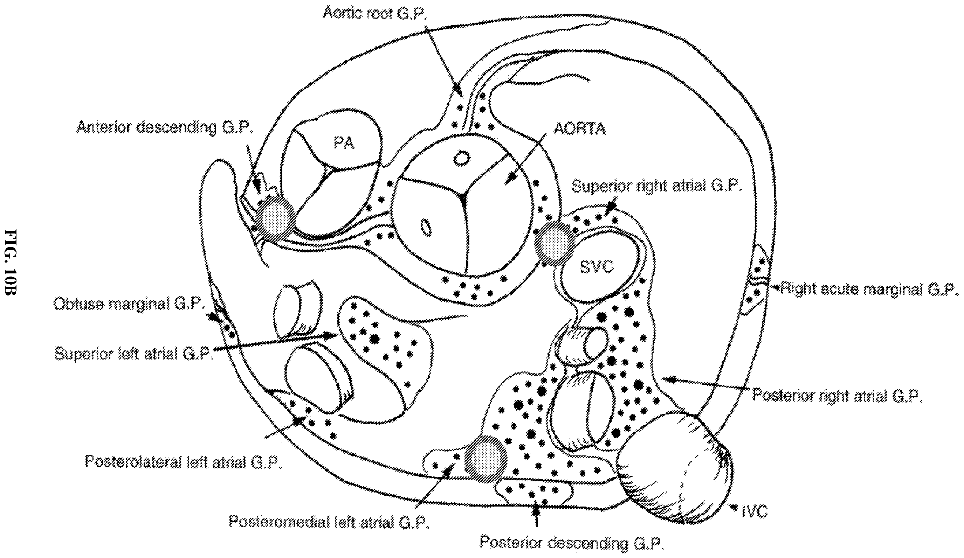

As shown in FIG. 10B, the locations of the identified synaptic centers correlate with the typical locations of the anterior descending ganglionated plexus, superior right atrial ganglionated plexus and posteromedial left atrial ganglionated plexus, as reported by Armour et al. [Anatomical Record 1997, 247:289-298].

These results indicate that the technique described herein detects and depicts cardiac ganglionated plexuses.

### EXAMPLE 3

### Imaging, verification and ablation of cardiac ganglionated plexuses

An electrophysiological study was performed in order to verify the identification of cardiac GP (ganglionated plexus) locations by imaging as described in Example 2, and to evaluate the utility of such imaging in ablation therapy.

Using the procedures described in Example 2, four synaptic centers representing ganglionic plexuses (GPs) were identified adjacent to the heart of a 47 year old male subject having a medical history of paroxysmal atrial fibrillation, catheter ablation for PVI (pulmonary vein isolation) and CVI (cavo-tricuspid isthmus), and normal LV (left ventricle) contraction ((LVEF (left ventricular ejection fraction) = 60 %, LVDd (left ventricular end-diastolic dimension) = 47 mm, and LAD (left atrial dimension) = 36 mm).

After imaging and identification of the four GPs, verification was performed by measuring the reaction of individual target sites to high frequency stimulation (HFS), as determined by electrocardiography.

At first, a site not associated with an imaged GP was subjected to HFS. This site is depicted in FIG. 11.

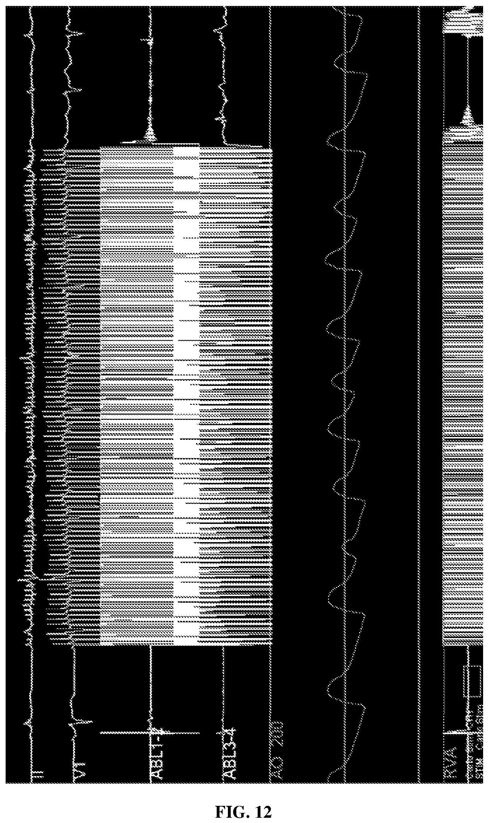

As shown in FIG. 12, the site not associated with an imaged GP did not respond to the HFS.

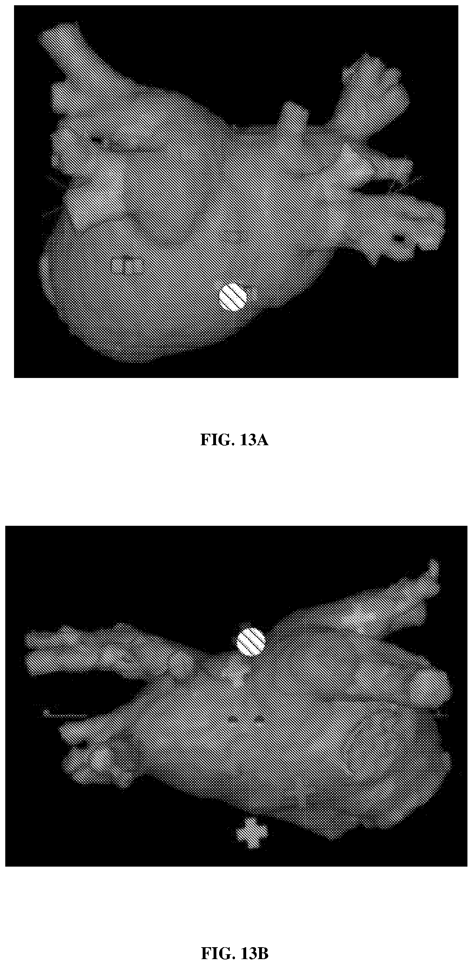

HFS was then performed at sites identified as the location of a GP in the abovementioned images. One site was a RIPV (right inferior pulmonary vein) GP location depicted in FIGs. 13A and 13B.

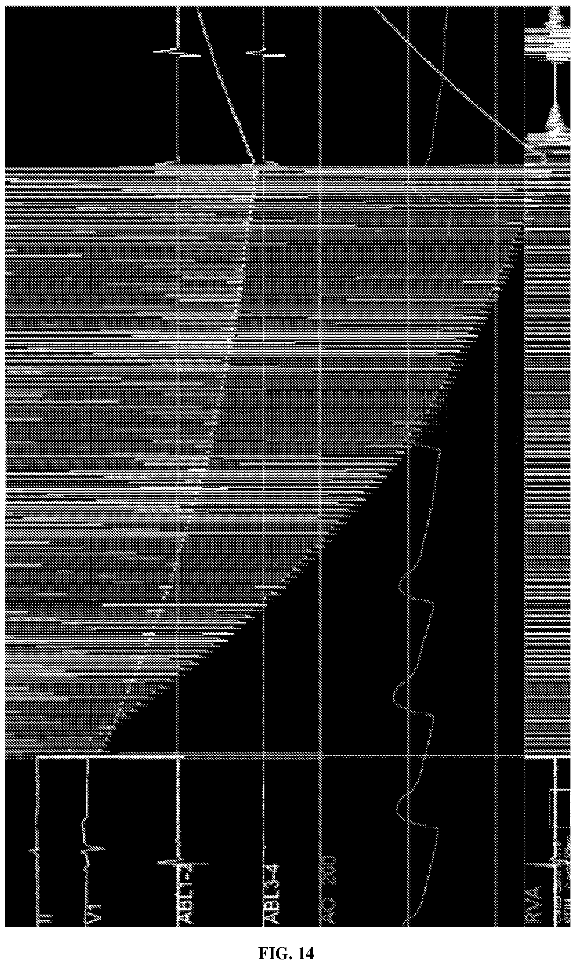

As shown in FIG. 14, the site associated with an imaged RIPV GP exhibited a positive response to HFS. Repetition of the application of HFS to the site associated with an imaged RIPV GP exhibited an additional positive response (data not shown).

Similarly, the site associated with an imaged LIPV GP exhibited a positive response to HFS (data not shown).

In such experiments, sites associated with imaged ganglionated plexuses exhibited a 100 % positive response rate to a limited number of HFS sessions, whereas sites not associated with imaged ganglionated plexuses exhibited a negative response to the same HFS treatment, even when applied more than 50 times (data not shown).

The above results confirm that the technique described herein accurately detects and depicts cardiac ganglionated plexuses.

The images of GPs were then used to ablate GPs. 5 ablation sessions at low power (up to 20 W) were applied by catheter at the LIPV GP location, as depicted in FIGs. 15A and 15B.

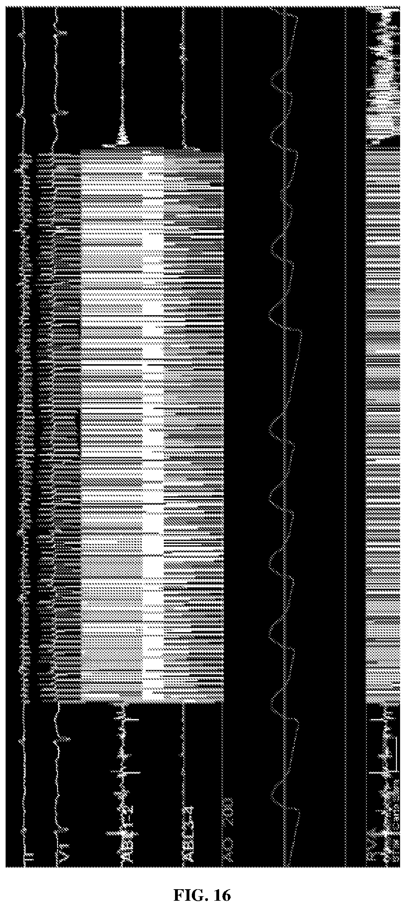

As shown in FIG. 16, the LIPV GP exhibited a negative response to local application of HFS after being subjected to ablation. Repetition of the HFS similarly failed to elicit a positive response from the LIPV GP (data not shown).

Similarly, the RIPV GP exhibited a negative response to each of two local applications of HFS after being subjected to 3 ablation sessions at low power (up to 20 W) (data not shown).

These results indicate that use of the technique described herein to image ganglionated plexuses in combination with ablation allows for effective ablation of ganglionated plexuses even when using limited power and a limited number of ablation sessions.

### EXAMPLE 4

### Imaging using combination of radioactive agents

Patients who had previously undergone cardiac ablation treatment were subjected to a stress test, and Tc-99m-radiolabeled sestamibi (300 MBq) (reference example) was injected at peak stress. Gated-SPECT data including 1 million left ventricular (LV) counts was acquired using a D-SPECT™ apparatus.

I-123-radiolabeled mIBG (150 MBq) was injected while subject was on the couch. Delayed redistribution (DR) SPECT images were acquired for 20 minutes at 10 minutes after mIBG injection (early mIBG), while patient was still on the couch.

Tc-99m-radiolabeled sestamibi (800 MBq) was then injected at rest, approximately 3 hours after the first sestamibi injection. A second set of DR SPECT images was acquired for 20 minutes at 4 hours after mIBG injection (late mIBG and sestamibi rest, 1 million LV counts).

The mIBG data was analyzed according to a method depicted in FIG. 4 (as described hereinabove) in order to characterize levels of innervation of different regions of the heart tissue, whereas the sestamibi data was analyzed in order to characterize vitality (as indicated by sestamibi perfusion) of different regions of the heart tissue. The distributions of innervation and vitality were compared.

FIG. 17 is a set 700 of three SPECT images of a left atrium 702, in accordance with an exemplary embodiment of the invention. The left image was reconstructed using SPECT data describing sestamibi distribution, which indicates viability. Red areas 704 indicate low vitality (believed to be at least partially associated with an ablation treatment), yellow areas 706 (appearing as a narrow band) indicate intermediate vitality and the surrounding green areas indicate high vitality. The middle image was reconstructed using SPECT data describing mIBG distribution, which indicates innervation by synapses. Red area 710 indicates areas characterized by a low level of innervation, as compared to the surrounding green areas characterized by a relatively high level of innervation. The right image shows a blue area 712 characterized by a relatively high level of innervation in combination with low viability, and an orange area 714 characterized by a low level of innervation in combination with high vitality. The surrounding green areas are characterized by a match between levels of innervation and vitality, e.g., innervation and vitality are both relatively low or both relatively high.

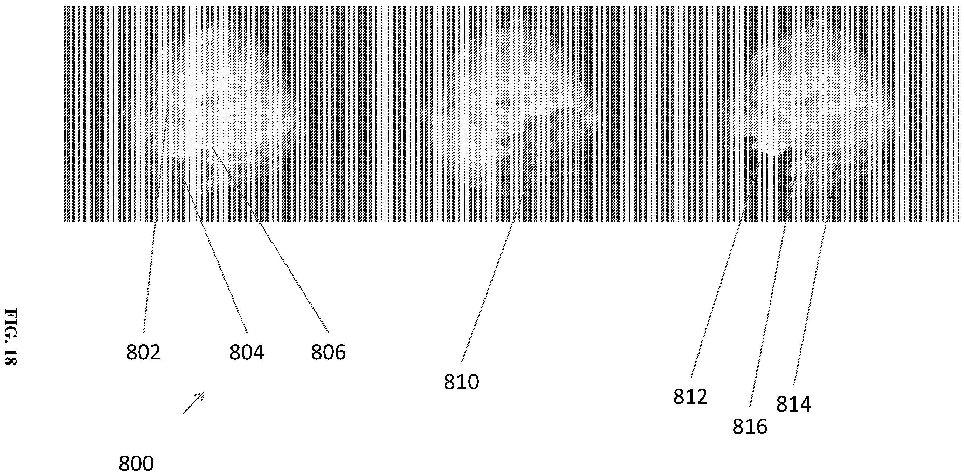

FIG. 18 is a set 800 of three SPECT images of a left ventricle 802, in accordance with an exemplary embodiment of the invention. The left image was reconstructed using SPECT data describing sestamibi distribution, which indicates viability. Red areas 804 indicate low vitality, yellow areas 806 (appearing as a narrow band) indicate intermediate vitality and the surrounding green areas indicate high vitality. The middle image was reconstructed using SPECT data describing mIBG distribution, which indicates innervation by synapses. Red area 810 indicates areas characterized by a low level of innervation, as compared to the surrounding green areas characterized by a relatively high level of innervation. The right image shows a blue area 812 characterized by a relatively high level of innervation in combination with low viability, and an orange area 814 characterized by a low level of innervation in combination with high vitality. The surrounding green areas are characterized by a match between levels of innervation and vitality, e.g., innervation and vitality are both relatively low or both relatively high.

### Example 5 (reference example)

### Diagnosis and treatment for benign prostatic hyperplasia (BPH)

Benign prostatic hyperplasia (BPH) is the most prevalent benign disorder affecting the prostate.

There is considerable evidence that modulation of sympathetic and/or parasympathetic tone will affect prostatic hyperplasia. Norepinephrine has a direct mitogenic effect on prostate stromal cells *in vitro.* Preganglionic sympathectomy in rat decreased the weight of the ventral prostatic lobe by 22.7 % via changes in cell size and cell number, and preganglionic parasympathectomy decreased the weight of the denervated side by 8.3 %, while the weight of the intact side increased by 24.8 %. The effects of combined preganglionic parasympathectomy and sympathectomy equaled the sum of the effects of each type of denervation performed separately.

In addition, smooth muscle contraction induced by noradrenergic sympathetic nerves results in constriction of the urethra, and contributes to clinical symptoms of BPH. Adrenergic receptor blockers are commonly used to relieve BPH symptoms.

FIG. 19 describes an exemplary procedure for diagnosing and/or treating BPH associated with abnormal ANS activity. An image of the pelvis (the whole pelvis or a portion of the pelvis in the vicinity of the prostate) is obtained using a radioactive tracer suitable for use as a marker of an ANS activity (e.g., as described herein). This information is co-registered with an acquired anatomical image of the pelvis (the whole pelvis or a portion of the pelvis in the vicinity of the prostate). The co-registered information is used to identify at least one anatomical region exhibiting an ANS hyperactivity (excess sympathetic and/or parasympathetic activity), thereby diagnosing abnormal ANS activity associated with BPH.

Treatment is optionally effected by applying a therapy to the identified region of ANS hyperactivity, for example, for inhibiting the ANS activity in that region (e.g., by ablation). The physiological effect of the therapy is optionally evaluated by any suitable means, for example, by performing high frequency stimulation of neurons at the identified region and measuring one or more markers of neuronal activity, such as blood pressure, hemodynamic response, and the like.

If treatment does not reduce or eliminate the ANS hyperactivity in the identified region, the treatment procedure may be repeated.

### Example 6 (reference example)

### Diagnosis and treatment for erectile disorder

Penile erection is a vascular event controlled by the ANS. Sympathetic pathways are anti-erectile and sacral parasympathetic pathways are pro-erectile. Penile erection is regulated by neurons in the inferior hypogastric plexus (also known as the "pelvic plexus"), which includes both sympathetic and parasympathetic neurons and ganglia, as well as by the pudendal nerve, which includes sympathetic neurons.

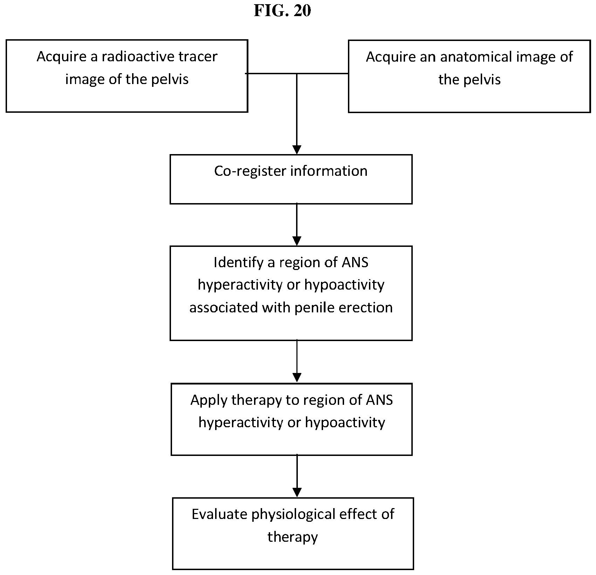

FIG. 20 describes an exemplary procedure for diagnosing and/or treating erectile disorder. An image of the pelvis (the whole pelvis or a portion of the pelvis in the vicinity of the penis) is obtained using a radioactive tracer suitable for use as a marker of an ANS activity (e.g., as described herein). This information is co-registered with an acquired anatomical image of the pelvis (the whole pelvis or a portion of the pelvis in the vicinity of the penis). The co-registered information is used to identify at least one anatomical region exhibiting an ANS hyperactivity or hypoactivity (e.g., excess sympathetic activity and/or deficient parasympathetic activity), thereby diagnosing abnormal ANS activity associated with erectile disorder.

Treatment is optionally effected by applying a therapy to the identified region of ANS hyperactivity or hypoactivity, for example, for inhibiting sympathetic activity in that region (e.g., by ablation). The physiological effect of the therapy is optionally evaluated by any suitable means, for example, by performing high frequency stimulation of neurons at the identified region and measuring one or more markers of neuronal activity, such as blood pressure, hemodynamic response, and the like.

If treatment does not reduce or eliminate the ANS hyperactivity or hypoactivity in the identified region, the treatment procedure may be repeated.

### Example 7 (reference example)

### Diagnosis and treatment for diabetes

Liver metabolism is affected by ANS innervation, which includes sympathetic innervation associated with the celiac ganglion, and parasympathetic innervation by the hepatic vagal branch and right posterior subdiaphragmatic vagal nerve.

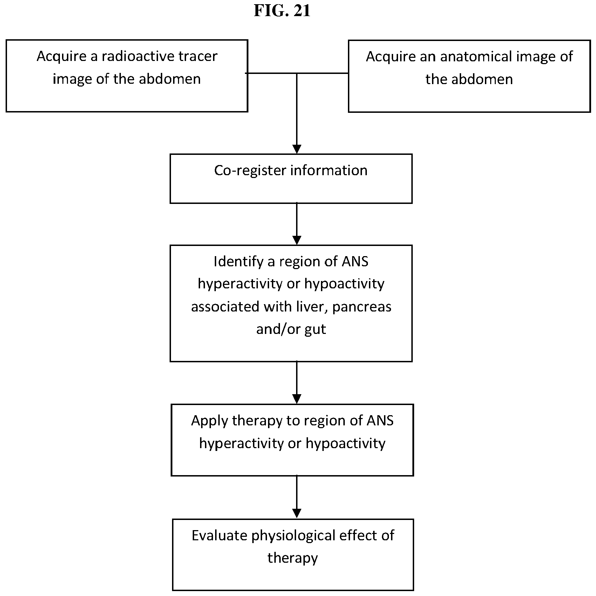

FIG. 21 describes an exemplary procedure for diagnosing and/or treating diabetes associated with abnormal ANS activity in the liver, pancreas and/or gut. An image of the abdomen (the whole abdomen or a portion of the abdomen in the vicinity of the liver, pancreas and/or gut) is obtained using a radioactive tracer suitable for use as a marker of an ANS activity (e.g., as described herein). This information is co-registered with an acquired anatomical image of the abdomen (the whole abdomen or a portion of the abdomen in the vicinity of the liver, pancreas and/or gut). The co-registered information is used to identify at least one anatomical region exhibiting an ANS hyperactivity or hypoactivity (e.g., of sympathetic and/or parasympathetic activity), thereby diagnosing abnormal ANS activity associated with diabetes.

Treatment is optionally effected by applying a therapy to the identified region of ANS hyperactivity or hypoactivity, for example, for inhibiting sympathetic and/or parasympathetic activity in that region (e.g., by ablation). The physiological effect of the therapy is optionally evaluated by any suitable means, for example, by performing high frequency stimulation of neurons at the identified region and measuring one or more markers of neuronal activity, such as blood pressure, hemodynamic response, and the like.

If treatment does not reduce or eliminate the ANS hyperactivity or hypoactivity in the identified region, the treatment procedure may be repeated.

### Example 8 (reference example)

### Diagnosis and treatment for arthritis

The nervous system is in communication with the immune system. Arthritis has been found to be associated with increased sympathetic nerve density in the spleen in the hilar region and in the red pulp, and with decreased sympathetic nerve density in the spleen in regions distal to the hilus and in the white pulp. This redistribution of nerves may be critical in modulating immune functions that contribute to chronic inflammatory stages of arthritis.

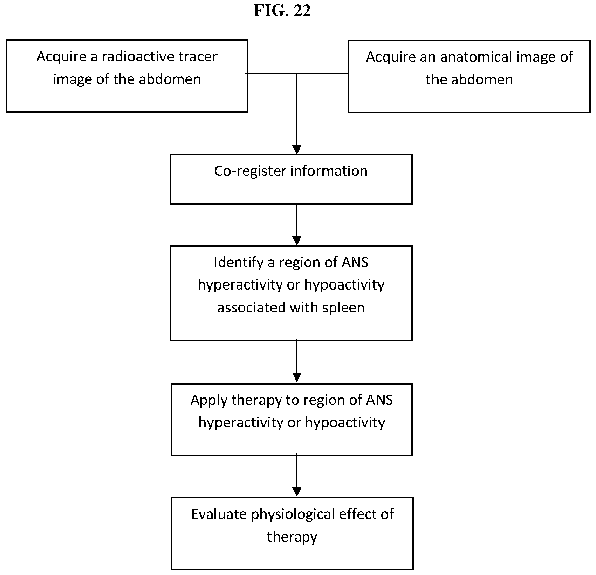

FIG. 22 describes an exemplary procedure for diagnosing and/or treating arthritis (e.g., rheumatoid arthritis) associated with abnormal ANS activity in the spleen. An image of the abdomen (the whole abdomen or a portion of the abdomen in the vicinity of the spleen) is obtained using a radioactive tracer suitable for use as a marker of an ANS activity (e.g., as described herein). This information is co-registered with an acquired anatomical image of the abdomen (the whole abdomen or a portion of the abdomen in the vicinity of the spleen). The co-registered information is used to identify at least one anatomical region exhibiting an ANS hyperactivity or hypoactivity (e.g., sympathetic hyperactivity in red pulp and/or sympathetic hypoactivity in white pulp), thereby diagnosing abnormal ANS activity associated with arthritis.

Treatment is optionally effected by applying a therapy to the identified region of ANS hyperactivity or hypoactivity, for example, for inhibiting sympathetic activity in that region (e.g., by ablation). The physiological effect of the therapy is optionally evaluated by any suitable means, for example, by performing high frequency stimulation of neurons at the identified region and measuring one or more markers of neuronal activity, such as blood pressure, hemodynamic response, and the like.

If treatment does not reduce or eliminate the ANS hyperactivity or hypoactivity in the identified region, the treatment procedure may be repeated.

### Example 9 (reference example)

### Diagnosis and treatment for irritable bowel syndrome

Irritable bowel syndrome may be associated with changes in nervous system activity, for example, by compression of the meninges. As the nervous system controls the rate at which food passes through the digestive system, changes in the nervous system can result in an increased rate which may cause diarrhea, or in a decreased rate which may cause constipation. Meningeal compression may be caused by accidents, trauma and even stress. Pulling and irritation of nerves may result in irregular impulses to the brain, which may be interpreted by the brain as pain, itching, burning, coldness, numbness or other feelings. In addition, excessive sympathetic activity may result in negative feelings such as gloom associated with adrenaline production, while inhibiting parasympathetic activity associated with normal digestive activity.

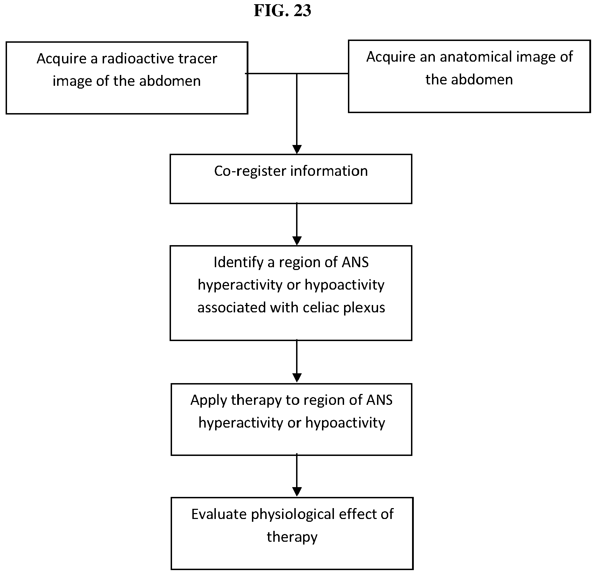

FIG. 23 describes an exemplary procedure for diagnosing and/or treating irritable bowel syndrome associated with abnormal ANS activity. An image of the abdomen (the whole abdomen or a portion of the abdomen in the vicinity of the celiac plexus) is obtained using a radioactive tracer suitable for use as a marker of an ANS activity (e.g., as described herein). This information is co-registered with an acquired anatomical image of the abdomen (the whole abdomen or a portion of the abdomen in the vicinity of the celiac plexus). The co-registered information is used to identify at least one anatomical region exhibiting an ANS hyperactivity or hypoactivity (e.g., sympathetic hyperactivity and/or parasympathetic hypoactivity), thereby diagnosing abnormal ANS activity associated with irritable bowel syndrome.

Treatment is optionally effected by applying a therapy to the identified region of ANS hyperactivity or hypoactivity, for example, for inhibiting sympathetic activity in that region (e.g., by ablation). The physiological effect of the therapy is optionally evaluated by any suitable means, for example, by performing high frequency stimulation of neurons at the identified region and measuring one or more markers of neuronal activity, such as blood pressure, hemodynamic response, and the like.

If treatment does not reduce or eliminate the ANS hyperactivity or hypoactivity in the identified region, the treatment procedure may be repeated.

Citation or identification of any reference in this application shall not be construed as an admission that such reference is available as prior art to the present invention. To the extent that section headings are used, they should not be construed as necessarily limiting.

## Claims

1. Radiolabeled mIBG (metaiodobenzylguanidine) for use in an *in vivo* method of diagnosis of cardiac arrhythmia, wherein said method of diagnosis comprises the use of radiolabeled mIBG in an imaging method of identifying autonomic nervous system ganglia of between 1 and 20 mm in maximal diameter, wherein said imaging method comprises combining SPECT data with anatomical data.

2. Radiolabeled mIBG for use according to claim 1, wherein said imaging method is used for mapping a distribution and/or activity of autonomic nervous system synapses and/or ganglia.

3. Radiolabeled mIBG for use according to claim 1, wherein said imaging method is used for displaying a map of a distribution and/or activity of autonomic nervous system synapses and/or ganglions.

4. Radiolabeled mIBG for use according to claim 3, wherein said method further comprises overlaying said map on an image of an organ.

5. Radiolabeled mIBG for use according to any one of claims 2 to 4, wherein said imaging method further comprises determining abnormal synapse distribution and/or activity by comparing said distribution with a set of distributions which is indicative of a normal synapse distribution and/or with a set of distributions which is indicative of a medical condition or disease.

6. Radiolabeled mIBG for use according to claim 1, wherein said imaging method further comprises stimulating an autonomic nervous system in conjunction with said imaging.

7. Radiolabeled mIBG for use according to claim 1, wherein said imaging method is for use in guiding a therapy of cardiac arrhythmia.

8. Radiolabeled mIBG for use according to claim 1, wherein said cardiac arrhythmia is caused, exacerbated or sustained by an input or involvement of an autonomic nervous system.

9. Radiolabeled mIBG (metaiodobenzylguanidine) for use in an *in vivo* method of diagnosis of cardiac arrhythmia, wherein said method of diagnosis comprises the use of radiolabeled mIBG in an imaging method of locating at least one ganglionic plexus (GP) in a nervous tissue, wherein said imaging method comprises combining SPECT data with anatomical data.

10. Radiolabeled mIBG for use according to claim 9, wherein the imaging method is used for identifying an innervated tissue, the method comprising: collecting radiation emission data from tissue, assigning the data to spatial locations according to a model of a structure of an organ and identifying nervous tissue synapses and/or innervations based on data associated with said organ.

11. Radiolabeled mIBG for use according to claim 9, wherein locating said at least one ganglionic plexus comprises identifying at least one region of interest (ROI) in an intrabody area or volume that comprises said nervous tissue according to a match with a reference value representing a reference uptake rate of the radiolabeled mIBG by an organ.

12. Radiolabeled mIBG for use according to claim 11 wherein locating said at least one ganglionic plexus is used for targeting a sub-region in an intrabody area as a target for ablation.

## Patentansprüche

1. Radioaktiv markiertes mIBG (Metaiodobenzylguanidin) zur Verwendung bei einem *in* vivo-Verfahren zur Diagnose von Herzrhythmusstörungen, wobei das Diagnoseverfahren die Verwendung von radioaktiv markiertem mIBG in einem Bildgebungsverfahren zur Identifizierung von Ganglien des autonomen Nervensystems mit einem maximalen Durchmesser zwischen 1 und 20 mm umfasst, wobei das Bildgebungsverfahren die Kombination von SPECT-Daten mit anatomischen Daten umfasst.

2. Radioaktiv markiertes mIBG zur Verwendung nach Anspruch 1, wobei das Bildgebungsverfahren zur Kartierung der Verteilung und/oder Aktivität von Synapsen und/oder Ganglien des autonomen Nervensystems verwendet wird.

3. Radioaktiv markiertes mIBG zur Verwendung nach Anspruch 1, wobei das Bildgebungsverfahren zum Darstellen einer Karte der Verteilung und/oder Aktivität von Synapsen und/oder Ganglien des autonomen Nervensystems verwendet wird.

4. Radioaktiv markiertes mIBG zur Verwendung nach Anspruch 3, wobei das Verfahren ferner das Überlagern der Karte auf einer Abbildung eines Organs umfasst.

5. Radioaktiv markiertes mIBG zur Verwendung nach einem der Ansprüche 2 bis 4, wobei das Bildgebungsverfahren ferner das Bestimmen einer ungewöhnlichen Synapsenverteilung und/oder - aktivität durch Vergleichen der Verteilung mit einem Satz von Verteilungen, der für eine normale Synapsenverteilung kennzeichnend ist, und/oder mit einem Satz von Verteilungen, der für ein medizinisches Problem oder eine Krankheit kennzeichnend ist, umfasst.

6. Radioaktiv markiertes mIBG zur Verwendung nach Anspruch 1, wobei das Bildgebungsverfahren ferner die Stimulation eines autonomen Nervensystems in Verbindung mit der Bildgebung umfasst.

7. Radioaktiv markiertes mIBG zur Verwendung nach Anspruch 1, wobei das Bildgebungsverfahren zur Verwendung bei der Durchführung einer Therapie von Herzrhythmusstörungen bestimmt ist.

8. Radioaktiv markiertes mIBG zur Verwendung nach Anspruch 1, wobei die Herzrhythmusstörung durch einen Einsatz oder eine Beteiligung des autonomen Nervensystems verursacht, verschlimmert oder aufrechterhalten wird.

9. Radioaktiv markiertes mIBG (Metaiodobenzylguanidin) zur Verwendung bei einem *in* vivo-Verfahren zur Diagnose von Herzrhythmusstörungen, wobei das Diagnoseverfahren die Verwendung von radioaktiv markiertem mIBG in einem Bildgebungsverfahren zur Lokalisierung mindestens eines Ganglienplexus (GP) in einem Nervengewebe umfasst, wobei das Bildgebungsverfahren das Kombinieren von SPECT-Daten mit anatomischen Daten umfasst.

10. Radiomarkiertes mIBG zur Verwendung nach Anspruch 9, wobei das Bildgebungsverfahren zur Identifizierung eines innervierten Gewebes verwendet wird, wobei das Verfahren umfasst: Erfassen von Strahlungsemissionsdaten von Gewebe, Zuordnen der Daten zu räumlichen Orten gemäß dem Modell einer Struktur eines Organs und Identifizieren von Nervengewebesynapsen und/oder Innervationen basierend auf Daten, die mit dem Organ verbunden sind.

11. Radioaktiv markiertes mIBG zur Verwendung nach Anspruch 9, wobei das Lokalisieren des mindestens einen Ganglienplexus das Identifizieren mindestens einer Untersuchungsregion (Region of Interest, ROI) in einem Intrabody-Bereich oder -Volumen umfasst, die das Nervengewebe gemäß einer Übereinstimmung mit einem Referenzwert umfasst, der eine Referenz-Aufnahmerate des radioaktiv markierten mIBG durch ein Organ darstellt.

12. Radioaktiv markiertes mIBG zur Verwendung nach Anspruch 11, wobei das Lokalisieren des mindestens einen Ganglienplexus zum Ansteuern einer Subregion in einem Intrabody-Bereich als Ziel für die Ablation verwendet wird.

## Revendications

1. mIBG (métaiodobenzylguanidine) radiomarquée pour une utilisation dans un procédé *in vivo* de diagnostic de l'arythmie cardiaque, ledit procédé de diagnostic comprenant l'utilisation de mIBG radiomarquée dans un procédé d'imagerie d'identification de ganglions du système nerveux autonome d'un diamètre maximal compris entre 1 et 20 mm, ledit procédé d'imagerie comprenant la combinaison de données SPECT avec des données anatomiques.

2. mIBG radiomarquée pour une utilisation selon la revendication 1, ledit procédé d'imagerie étant utilisé pour cartographier une distribution et/ou une activité de synapses et/ou de ganglions du système nerveux autonome.

3. mIBG radiomarquée pour une utilisation selon la revendication 1, ledit procédé d'imagerie étant utilisé pour afficher une carte d'une distribution et/ou d'une activité de synapses et/ou de ganglions du système nerveux autonome.

4. mIBG radiomarquée pour une utilisation selon la revendication 3, ledit procédé comprenant en outre la superposition de ladite carte sur une image d'un organe.

5. mIBG radiomarquée pour une utilisation selon l'une quelconque des revendications 2 à 4, ledit procédé d'imagerie comprenant en outre la détermination d'une distribution et/ou d'une activité anormales de synapses par comparaison de ladite distribution avec un ensemble de distributions qui est indicatif d'une distribution normale de synapses et/ou avec un ensemble de distributions qui est indicatif d'un état médical ou d'une maladie.

6. mIBG radiomarquée pour une utilisation selon la revendication 1, ledit procédé d'imagerie comprenant en outre la stimulation d'un système nerveux autonome conjointement avec ladite imagerie.

7. mIBG radiomarquée pour une utilisation selon la revendication 1, ledit procédé d'imagerie étant destiné à être utilisé dans le guidage d'une thérapie de l'arythmie cardiaque.

8. mIBG radiomarquée pour une utilisation selon la revendication 1, ladite arythmie cardiaque étant provoquée, exacerbée ou nourrie par un apport ou une implication d'un système nerveux autonome.

9. mIBG (métaiodobenzylguanidine) radiomarquée pour une utilisation dans un procédé *in vivo* de diagnostic de l'arythmie cardiaque, ledit procédé de diagnostic comprenant l'utilisation de mIBG radiomarquée dans un procédé d'imagerie de localisation d'au moins un plexus ganglionnaire (GP) dans un tissu nerveux, ledit procédé d'imagerie comprenant la combinaison de données SPECT avec des données anatomiques.

10. mIBG radiomarquée pour une utilisation selon la revendication 9, le procédé d'imagerie étant utilisé pour identifier un tissu innervé, le procédé comprenant : la collecte de données d'émission de rayonnement provenant du tissu, l'affectation des données à des emplacements spatiaux en fonction d'un modèle d'une structure d'un organe et l'identification de synapses et/ou d'innervations du tissu nerveux sur la base de données associées audit organe.

11. mIBG radiomarquée pour une utilisation selon la revendication 9, la localisation dudit au moins un plexus ganglionnaire comprenant l'identification d'au moins une région d'intérêt (ROI) dans une zone ou un volume intracorporel qui comprend ledit tissu nerveux en fonction d'une correspondance avec une valeur de référence représentant un taux d'absorption de référence de la mIBG radiomarquée par un organe.

12. mIBG radiomarquée pour une utilisation selon la revendication 11, la localisation dudit au moins un plexus ganglionnaire étant utilisée pour cibler une sous-région dans une zone intracorporelle comme cible d'ablation.
